(19) Europäisches Patentamt
European Patent Office
Office européen des brevets

(11) **EP 2 545 480 B1**

(12) **EUROPEAN PATENT SPECIFICATION**

(45) Date of publication and mention
of the grant of the patent:
**22.04.2020 Bulletin 2020/17**

(51) Int Cl.:
***G16B 20/00*** (2019.01)    *G16B 40/00* (2019.01)

(21) Application number: **11710244.2**

(22) Date of filing: **10.03.2011**

(86) International application number:
**PCT/GB2011/050470**

(87) International publication number:
**WO 2011/110853 (15.09.2011 Gazette 2011/37)**

(54) **IMPROVEMENTS IN AND RELATING TO THE CONSIDERATION OF EVIDENCE**

VERBESSERUNG BEI UND IM ZUSAMMENHANG MIT DER BERÜCKSICHTIGUNG VON BEWEISEN

AMÉLIORATIONS CONCERNANT LA PRISE EN CONSIDÉRATION DE PREUVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.03.2010 GB 201004004**

(43) Date of publication of application:
**16.01.2013 Bulletin 2013/03**

(73) Proprietor: **Eurofins Forensic Services Limited Teddington, Middlesex TW11 0LY (GB)**

(72) Inventors:
• **PUCH-SOLIS, Roberto**
  **Birmingham West Midlands B37 7YN (GB)**
• **RODGERS, Lauren**
  **Birmingham West Midlands B37 7YN (GB)**

(74) Representative: **Potter Clarkson**
**The Belgrave Centre**
**Talbot Street**
**Nottingham NG1 5GG (GB)**

(56) References cited:
**WO-A1-2006/059132    WO-A1-2009/066067**
**WO-A1-2010/116158**

• **PUCH-SOLIS R ET AL: "Assigning weight of DNA evidence using a continuous model that takes into account stutter and dropout", SCIENCE AND JUSTICE, HARROGATE, GB, vol. 50, no. 1, 1 March 2010 (2010-03-01), pages 46-47, XP026934377, ISSN: 1355-0306, DOI: DOI:10.1016/J.SCIJUS.2009.11.074 [retrieved on 2010-03-01]**
• **BRIGHT J A ET AL: "Examination of the variability in mixed DNA profile parameters for the Identifiler multiplex", FORENSIC SCIENCE INTERNATIONAL: GENETICS, ELSEVIER BV, NETHERLANDS, vol. 4, no. 2, 1 February 2010 (2010-02-01), pages 111-114, XP026829810, ISSN: 1872-4973 [retrieved on 2009-08-04]**

**Description**

This invention concerns improvements in and relating to the consideration of evidence, particularly, but not exclusively the consideration of DNA evidence.

In many situations, particularly in forensic science, there is a need to consider one piece of evidence against one or more other pieces of evidence.

For instance, it may be desirable to compare a sample collected from a crime scene with a sample collected from a person, with a view to linking the two by comparing the characteristics of their DNA. This is an evidential consideration. The result may be used directly in criminal or civil legal proceedings. Such situations include instances where the sample from the crime scene is contributed to by more than one person.

In other instances, it may be desirable to establish the most likely matches between examples of characteristics of DNA samples stored on a database with a further sample. The most likely matches or links suggested may guide further investigations. This is an intelligence consideration.

In both of these instances, it is desirable to be able to express the strength or likelihood of the comparison made, a so called likelihood ratio.

The present invention has amongst its possible aims to establish likelihood ratios. The present invention has amongst its possible aims to provide a more accurate or robust method for establishing likelihood ratios. The present invention has amongst its possible aims to provide probability distribution functions for use in establishing likelihood ratios, where the probability distribution functions are derived from experimental data. The present invention has amongst its possible aims to provide for the above whilst taking into consideration stutter and/or dropout of alleles in DNA analysis. The present invention has amongst its possible aims to provide for the above whilst taking into consideration one or more peak imbalance effects, such as degradation, amplification efficiency, sampling effects and the like in DNA analysis. Document WO 2009/066067 discloses methods of comparing a first DNA profile with a second DNA profile using the likelihood ratio for one hypothesis relative to another as the sources of the DNA are conditioned on quantity of DNA in the test samples.

According to the invention we provide a method of comparing a test sample result set with another sample result set, as defined by claim 1.

The method of comparing may be used to considered evidence, for instance in civil or criminal legal proceedings. The comparison may be as to the relative likelihoods, for instance a likelihood ratio, of one hypothesis to another hypothesis. The comparison may be as to the relative likelihoods of the evidence relating to one hypothesis to another hypothesis. In particular, this may be a hypothesis advanced by the prosecution in the legal proceedings and another hypothesis advanced by the defence in the legal proceedings. The likelihood ratio may be of the form:

$$LR = \frac{p(c, gs|V_p)}{p(c, gs|V_d)} = \frac{f(c|gs, V_p)}{f\, c|gs, V_d)}$$

where

- c is the first or test result set from a test sample, more particularly, the first result set taken from a sample recovered from a person or location linked with a crime, potentially expressed in terms of peak positions and/or heights and/or areas;
- gs is the second or another result set, more particularly, the second result set taken from a sample collected from a person, particularly expressed as a suspect's genotype;
- $V_p$ is one hypothesis, more particularly the prosecution hypothesis in legal proceedings stating "The suspect left the sample at the scene of crime";
- $V_d$ is an alternative hypothesis, more particularly the defence hypothesis in legal proceedings stating "Someone else left the sample at the crime scene".

The method may include a likelihood which includes a factor accounting for stutter. The factor may be included in the numerator and/or the denominator of a likelihood ratio, LR. The method may include a likelihood which includes a factor accounting for allele dropout. The factor may be included in the numerator and/or denominator of an LR. The method may include a likelihood which includes a factor accounting for one of more effects which impact upon the amount of an allele, for instance a height and/or area observed for a sample compared with the amount of the allele in the sample. The effect may be one or more effects which gives a different ratio and/or balance and/or imbalance between observed and present amounts with respect to different alleles and/or different loci. The effect may be and/or include degradation effects. The effect may be and/or include variations in amplification efficiency. The effect may be and/or include variations in amount of allele in a sub-sample of a sample, for instance, when compared with other sub-samples and/or the sample. The effect may be one whose effect varies with alleles and/or loci and/or allele size and/or locus size. The effect may be an effect which causes a reduction in the observed amount compared with that which would have occurred without the effect. The effect may exclude any stutter effect.

The method may include an LR which includes a factor accounting for stutter in both numerator and denominator. The method may include an LR which includes a factor accounting for allele dropout in both numerator and denominator. The method may include an LR which includes a factor accounting for one of more effects which impact upon the amount of an allele, for instance a height and/or area observed for a sample compared with the amount of the allele in the sample in both numerator and denominator.

In an initial embodiment, the method may consider one or more samples which are from a single source.

Particularly in the context of the initial embodiment, the invention may provided that the method is used in an evidential use.

The method may include a step including an LR. The LR may summarise the value of the evidence in providing support to a pair of competing propositions: one of them representing the view of the prosecution ($V_p$) and the other the view of the defence ($V_d$). The propositions may be:

1) $V_p$: The suspect is the donor of the DNA in the crime stain;
2) $V_d$: Someone else is the donor of the DNA in the crime stain.

The LR may be:

$$LR = \frac{f(c|g_s, V_p)}{f(c|g_s, V_d)}$$

with the crime profile c in a case consists of a set of crime profiles, where each member of the set is the crime profile of a particular locus. The suspect genotype $g_s$ may be a set where each member is the genotype of the suspect for a particular locus. As a result, the notation may be used as:

$$c = \left\{ c_{L(i)} : i =, 2, ..., n_i \right\} \quad \text{and} \quad g_s = \left\{ g_{s,L(i)} := 1, 2, ..., n_i \right\}$$

where $n_i$ is the number of loci in the profile.

The method may include accounting for peak imbalance. The method may include conditioning on the sum per locus; $\chi_{l(i)}$, the sum of peak heights in a locus.

The definition of the numerator may be or include:

$$Num = f\left( c|g_s, H_p \right) = \prod_{i=T}^{n_l} f(C_{l(i)}|g_{s,l(i)}, H_P)\chi_{l(i)}, \delta)$$

where the peak heights are summed for loci i and $\delta$ is a parameter, such as an effect parameter or peak imbalance parameter.

The right-hand side factor of the above equation, $f(C_{l(i)}|g_{s,l(i)}, H_P, \chi_{l(i)}, \delta)$ can be written as: $f(C_{l(i)}|g_{l(i)}, \chi_{l(i)}, \delta)$ where it is assumed that $g_{l(i)}$ is the genotype of the donor of $C_{l(i)}$, potentially with the donor varying according to the prosecution hypothesis and the defence hypothesis.

The comparison may include use of : $f(C_{l(i)}|g_{l(i)}, \chi_{l(i)}, \delta)$.

The definition of the denominator may be or include:

$$Den = f\left( c|g_s, H_d \right) = \prod_{i=T}^{n_l} f(C_{l(i)}|g_{s,l(i)}, H_d, \chi_{l(i)}, \delta)$$

The right-hand side factor of the above equation, $f(C_{l(i)}|g_{s,l(i)}, H_d, \chi_{l(i)}, \delta)$ can be written as:

$$f(C_{l(i)}|g_{s,l(i)},\chi_{l(i)},H_d,\delta) = \sum_i f(C_{l(i)}|g_{u(i)},\chi_{l(i)},H_d,\delta,g_{u,l(i)})\Pr(g_{u,l(i)}|g_{s,l(i)})$$

where the function $f(C_{l(i)}|g_{u(i)},\chi_{l(i)},H_d,\delta,g_{u,l(i)})$ can be written as: $f(C_{l(i)}|g_{l(i)},\chi_{l(i)},\delta)$ where we assume that $g_{l(i)}$ is the genotype of the donor of $C_{l(i)}$.

The factors in the right-hand-side of the equation may be computed using the model of Balding and Nichols. This can be computed using existing formula for conditional genotype probabilities given putative related and unrelated contributors with population structure or not, for instance using the approach defined in J.D. Balding and R. Nichols. DNA profile match probability calculation: How to allow for population stratification, relatedness, database selection and single bands. Forensic Science International, 64:125-140, 1994.

Particularly in the context of the initial embodiment, the invention may provided that the method is used in an intelligence use.

The method of comparing may be used to gather information to assist further investigations or legal proceedings. The method of comparing may provide intelligence on a situation. The method of comparison may be of the likelihood of the information of the first or test sample result given the information of the second or another sample result. The method of comparison may provide a listing of possible another sample results, ideally ranked according to the likelihood. The method of comparison may seek to establish a link between a DNA profile from a crime scene sample and one or more DNA profiles stored in a database.

The method of comparing may provide a link between a DNA profile, for instance from a crime scene sample, and one or more profiles, for instance one or more profiles stored in a database.

The method of comparing may consider a crime profile with the crime profile consisting of a set of crime profiles, where each member of the set is the crime profile of a particular locus. The method may propose, for instance as its output, a list of profiles from the database. The method may propose a posterior probability for one or more or each of the profiles. The method may propose, for instance as its output, a list of profiles, for instance ranked such that the first profile in the list is the genotype of the most likely donor.

The method of comparing may compute posterior probabilities of the genotype given the crime profile for locus i. Given the crime stain, quantity of DNA and effect (such as peak imbalance/EQA parameter), the method may assign probabilities to the genotypes which could be behind the crime stain. The term $\chi_{l(i)}$ may denotes the sum of peak heights in locus i bigger than reporting threshold $T_r$. The term $\delta$ may denote the effect factor/parameter.

The posterior genotype probability for $g^*_{u,l(i)}$ given $C_{l(i)}$, $\chi_{l(i)}$ and $\delta$ may be calculated using Bayes theorem:

$$p(g^*_{u,l(i)}|C_{l(i)},\chi_{l(i)},\delta) = \frac{f\left(c_{l(i)}\middle|g^*_{u,l(i)},\chi_{l(i)},\delta\right)p\left(g^*_{u,l(i)}\right)}{\sum_{g_{U,l(i)}}f\left(c_i\middle|g_{U,l(i)},\chi_{l(i)},\delta\right)p\left(g_{U,l(i)}\right)}$$

where $p(g_{U,l(i)})$ is the probability of genotype $g_{U,l(i)}$ prior to observing the crime profile. The method may provide that its sets a uniform prior to all genotypes so that only the effect of the crime profile is considered. The formula above may be simplified to:

$$p(g^*_{u,l(i)}|C_{l(i)},\chi_{l(i)},\delta) = \frac{f\left(c_{l(i)}\middle|g^*_{u,l(i)},\chi_{l(i)},\delta\right)}{\sum_{g_{U,l(i)}}f\left(c_i\middle|g_{U,l(i)},\chi_{l(i)},\delta\right)}$$

As above in the evidential uses, both numerator and denominator can be presented in a form based around the core pdf:

$$f(C_{l(i)}|g_{l(i)},\chi_{l(i)},\delta)$$

where we assume that $g_{l(i)}$ is the genotype of the donor of $C_{l(i)}$.

The method may not compute all possible genotypes in a locus. The method may compute/generate genotypes that may lead to a non-zero posterior probability. Starting with the crime profile $C_{l(i)}$ in this locus, peaks may be designated either as a stutter or alleles. The set of designated alleles may be used for generating the possible genotypes. There may be three possibilities:

> 1. No peaks bigger than reporting threshold $T_r$ and/or no genotype is generated as there is no peak height information to inform them.
> 2. One peak bigger than $T_r$ as allele, denoted by $a$ and/or the possible genotypes are {a,a} and {a, Q} where $Q$ denotes any allele other than $a$.
> 3. Two peaks bigger than $T_r$ designated as alleles, denoted by $a$ and $b$ and/or the only possible genotype is {a,b}.

The method may consider the position where allele dropout is not involved given the suspect's genotype.

The method may include, for instance where all the expected peaks given the genotype, including any stutter peaks present, are above the detection threshold limit T, the construction of a pdf according to one or more of the following steps:

| | | |
|---|---|---|
| **Step 1** | The peak-height sum may be denoted by $\chi_{l(i)}$. The corresponding means for the peak heights of the alleles and the stutters of the putative donor $g_{l(i)}$ may be denoted by $\mu_{a,1,l(i)}$ and $\mu_{s,1,l(i)}$ respectively. They may be a function of $\chi_{l(i)}$. For each allele $a$ of the donor, we may assign the allele mean $\mu_{a,1,l(i)}$ to the position of allele $a$, and the stutter mean $\mu_{s,1,l(i)}$ to a position $a$ - 1. If the donor is homozygote, we may do the assignment twice. | |
| **Step 2** | If the donor is a heterozygote, the means may be modified using the factor $\delta$ to take into account factors, such as PCR efficiency and degradation, that affect the resulting peak heights. For example, if the donor is a heterozygote in locus $l(i)$ the mean for his/her alleles and stutters may be: $\delta_1 x \mu_{a,1,l(i)}$ and $\delta_1 x \mu_{s,1,l(i)}$ for the low-molecular-weight allele and $\delta_2 x \mu_{a,1,l(i)}$ and $\delta_2 x \mu_{s,1,l(i)}$ for the high-molecular-weight allele. | |
| **Step 4** | The variances for each allele and stutter may be obtained as a function of their corresponding means. In another step we may add random Gamma variables. A condition for a close form calculation of this addition may be that the $\beta$-parameters are the same. Also in a later step, we may divide each Gamma by the overall sum of peak height to account for using the sum of peak heights in this locus. A closed form calculation can be done if all $\beta$ parameters may be the same. The conditioned on the $\beta$-parameters may be obtained by estimating a line between the points form by the means, in the x-axis, and the variances, in the y-axis. A regression line with zero intercept may be fitted to obtain:<br><br>$$\hat{\sigma}^2 = \kappa_2 x \mu$$<br><br>So, if peak i has mean and variance ($\mu_i$, $\sigma_i^2$),<br><br>$$\beta = \mu_i / \sigma_i^2 = \mu_i / (\kappa_2 x \mu_i) = 1/\kappa_2$$<br><br>may be true, regardless of the value of $\mu_1$. | |
| **Step 5** | The shape ($\alpha$) and rate ($\beta$) parameters may be obtained from the mean and the variances. | |
| **Step 6** | The alpha parameters for alleles and stutters in the same allele position may be added to obtain an overall $\alpha$ for that allele position. This may provide the parameters of a Gamma distribution for each allele position. | |
| **Step 7** | To account for using the sum of peak height in the locus, the collection of Gamma pdf's whose peak heights are above the peak-height reporting limit may be converted to a Dirichlet pdf. This may be achieved in closed form because all $\beta$'s are the same. The resulting Dirichlet pdf may inherit the $\alpha$ parameters of the Gammas. | |

In a second consideration, below, we consider the position where allele dropout is involved given the suspect's genotype. The consideration may reflect one or more of the heights in the profile being below the threshold T. In such a case, the peak which is below the threshold may not form part of the value of $\chi_{l(i)}$ and the correction may only applied to those peaks above the threshold.

In the case of a non-adjacent heterozygous alleles case, when $h_{s,1,l(i)} < T$ then the PDF may be given by:

$$f\left(h_{s,1,l(i)} < T, h_{a,1,l(i)}, h_{s,2,l(i)}, h_{a,2,l(i)} \middle| g_{l(i)} = \left\{a_{1,l(i)}, a_{2,l(i)}\right\}, \chi_{l(i)}, \delta\right)$$

which can be expressed as:

$$F(T|\alpha_{s,1,l(i)},\beta)f_{Dir}(\pi_{a,1,l(i)},\pi_{s,2,l(i)},\pi_{a,2,l(i)},|\alpha_{a,1,l(i)},\alpha_{s,2,l(i)},\alpha_{a,2,l(i)},)$$

where F is the cdf of a gamma distribution with parameters $\alpha_{s,1,l(i)}$ and $\beta$.

If there is more than one peak below the threshold T, then there may be a corresponding number of f.

The method may include a consideration of whether the peaks in the crime profile are either bigger or smaller than the reporting threshold $T_r$, or not present at all. The method may treat missing peaks and peaks smaller than $T_r$ as peaks that have dropped out. We may partition the crime profile for a given pair of genotype as:

$$c_{l(i)} = \left\{h : h \in c_{l(i)}, h < T_r\right\} \cup \left\{h : h \in c_{l(i)}, h \geq T_r\right\}$$

The resulting pdf may be given by:

$$f(c_{l(i)}|g_{1,l(i)},g_{2,l(i)},\chi_{l(i)},\delta,\omega) = f(\pi|\alpha) x \prod_{\{h:h\in c_{l(i)},h<T_r\}} F(T|\alpha_h,\beta_h)$$

$f(\pi|\alpha)$ is a Dirichlet pdf with parameters:

$$\alpha = \cup\left\{\alpha_h : h \in c_{l(i)}, h \geq T_r\right\}$$

and

$$\pi = \cup\left\{h\big/\chi'_{l(i)} : h \in c_{l(i)}, h \geq T_r\right\}$$

where $a_h$ is the alpha parameter of the associated Gamma pdf in the corresponding position of height $h$.

$$* \chi'_{l(i)} = \sum_{h\in c_{l(i)},h\geq T_r} h$$

$h$ is the sum of peak heights bigger than reporting threshold $T_r$. $F(T_r|\alpha_h,\beta_h)$ is the CDF of a Gamma distribution with parameters $\alpha_h$ and $\beta_h$ for the peak in the position of $h$
calculated as described above.

The method may include the use of a peak imbalance parameter/ effective amplified quanitity (EQE) parameter, $\delta$, particularly in the form of a set of $\delta$'s, such that there is for instance one for each of the alleles. Each of the peak imbalance parameters in the set can be used to adjust the means for the alleles.

The approach preferably models the effect, such as degradation and other peak imbalance effects, prior to any knowledge of the suspect's genotype. For each locus, the molecular weight of the peaks in the profile may be associated with the sum of the heights. As the molecular weight of the locus increase, a reduction in the sum of the peak heights may be estimated.

The method may provide that for locus l(i), there are a set of peak heights: $h_{l(i)} = \{h_{j,l(i)} : j = 1,......n_{l(i)}\}$. Each height may have an associated base pair count: $b_{l(i)} = \{b_{j,l(i)} : j = 1,......n_{l(i)}\}$. An average base pair count may be used as a measure of molecular weight for the locus, weighted by peak heights. This may be defined as:

$$\bar{b}_{l(i)} = \frac{\sum_{j=1}^{n} b_{j,l(i)}h_{j,l(i)}}{\chi_{l(i)}}$$

where $\chi_{l(i)} = \sum_{j=1}^{n_{l(i)}} h_{j,l(i)}$ and so the degradation model may be defined as: $\chi_{l(i)} = d_1 + d_2 \bar{b}_{l(i)}$ where $d_1$ and $d_2$ are the same for all loci.

The parameters $d_1$ and $d_2$ may be calculated using the least squared estimation. As some loci may behave differently to degradation etc, the sum of the peak heights for these loci may be treated as outliers. To deal with these outliers, a Jacknife method may be used. If there are $n_L$ loci with peak height and base pair information, then the approach may include one or more or all of:

    1. fiting a regression model $n_L$ times, removing the $i^{\text{th}}$ value of the sets $\{\chi_{l(i)} : i = 1,......n_L\}$ and $\{\overline{b}_{l(i)} : i = 1,......n_L\}$.

    2. using the regression model to produce a prediction interval, $\hat{\chi}_{l(i)} = l - 2\sigma$ where $\sigma$ is the standard deviation of the residuals in the fitted regression line.

    3. when the sum of the peak height $\chi_i$, which is not used for the estimation of the regression line, does not lie within the prediction interval, then consider it as an outlier.

    4. removes any outliers from the data set and refits the model, after the $n_L$ models have been produced.

    5. the values of $d_1$ and $d_2$ being extracted from the model estimated without outliers.

If the effect on in the profile is negligible, peak height variability may cause the estimated value of $d_2$ to be greater than zero. In such cases, $d_2$ may be set as 0 and/or $d_1$ as 1.

In the deployment of the model of the parameter, at locus l(i) there may be a crime profile with peaks having allele designations $\alpha_{j,l(i)}$ and base pair counts $b_{l(i)} = \{b_{j,l(i)} : j = 1,......n_{l(i)}\}$. If degradation were not being accounted for, then given the sum of the peak heights $\chi_{l(i)}$ it is possible to obtain a mean and a variance from a Gamma distribution.

When considering the effect, the same Gamma distribution may be used, but the model may be used to adapt the Gamma pdf to account for the molecular weight of the allele.

As previously mentioned, peak heights increase with the sum of peak heights $\chi_{l(i)}$ and therefore the mean and variance may also increase accordingly. If an allele is of high molecular weight, a reduction of $\chi_{l(i)}$ may result in a reduction in the mean and variance. The model may reduce or increases the $\chi_{l(i)}$ associated with an allele according to the effect by using an appropriate $\delta$ for that allele.

The appropriate $\delta$'s may be calculated as follows using the degradation model $\chi_{l(i)} = d_1 + d_2 \overline{b}_{l(i)}$.

The degradation parameter associated with alleles $\alpha_{j,l(i)}$ may be defined as $\delta_{j,l(i)}$ so that the sum of peak heights associated with this allele are $\delta_{j,l(i)} \cdot \chi_{l(i)}$.

For each allele the model may be used to estimate the associated peak height sum:

$$\hat{\chi}_{j,l(i)} = d_1 + d_2\, b_{j,l(i)}$$

The calculations of $\delta$ may be made such that the ratio of the estimated peak height sums are preserved; that is:

$$\frac{\hat{\chi}_{j,l(i)}}{\hat{\chi}_{j+1,l(i)}} = \frac{\delta_{j,l(i)} \cdot \chi_{l(i)}}{\delta_{j+1,l(i)} \cdot \chi_{l(i)}}$$

To do this, a set of $n_{l(i)}-1$ equations with $n_{l(i)}$ unknowns, may be provided:

$$\frac{\hat{\chi}_{j,l(i)}}{\hat{\chi}_{j+1,l(i)}} = \frac{\delta_{j,l(i)}}{\delta_{j+1,l(i)}}$$

The ratios on the left-hand side may be obtained from the degradation model and the $\delta$'s may be the unknown variables. A restriction is set, such that the average peak height sum in the locus remains the same after the application of the $\delta$'s, may be:

$$\frac{\sum_{j=1}^{n_{l(i)}} \delta_{j,l(i)} \cdot \chi_{l(i)}}{n_{l(i)}} = \chi_{l(i)}$$

which gives a further equation with the $\delta$'s as unknown quantities. This may allow a solution to be found as there are

$n_{l(i)}$ equations in the system and $n_{l(i)}$ unknowns.

The ratio of the estimated peak height sum may be denoted:

$$r_{j,l(i)} = \frac{\hat{\chi}_{j,l(i)}}{\hat{\chi}_{j+1,l(i)}}, j = 1, 2 \ldots \ldots n_{l(i)} - 1$$

$$r_{j,l(i)} = 1, j = n_{l(i)}$$

The degradation parameters $\delta$'s, may be given by:

$$\delta_{j,l(i)} = \frac{n_{l(i)} \prod_{k=j}^{n_{l(i)}} r_{k,l(i)}}{\sum_{k=1}^{n_{l(i)}} \prod_{k}^{n_{l(i)}} r_{k,l(i)}}$$

The stutter associated with an allele, may have the same degradation parameter $\delta$ as the allele because the starting DNA molecule is the same in each case.

In another embodiment, the method may consider one or more samples which are from multiple sources. Two and/or three and/or more sources for the sample may be present.

Particularly in the context of an initial embodiment, the invention may provided that the method is used in an evidential use.

The method may provide that the comparison includes a numerator stated as:

$$Num = f\left(c \middle| g_{U1}, g_{U2s}, H_p\right) = \prod_{i=T}^{n_l} f\left(c_{l(i)} \middle| g_{U1,l(i)}, g_{U2,l(i)}, H_P, \chi_{l(i)}, \delta\right)$$

The method may provide that the comparison includes a denominator stated as:

$$Den = f\left(c \middle| g_{U1}, g_{U2}, H_d\right) = \prod_{i=T}^{n_l} f\left(C_{l(i)} \middle| g_{U1,l(i)}, g_{U2,l(i)}, H_d, \chi_{l(i)}, \delta\right)$$

The method may include the consideration of the pdf: $f(c_{l(i)} | g_{U,1,l(i)}, g_{U,2,l(i)}, \chi_{l(i)}, \delta)$

Particularly in the context of the initial embodiment, the invention may provided that the method is used in an intelligence use.

The method may compute the posterior probability $p(g_{U,1,l(i)}, g_{U,2,l(i)} | C_{l(i)}, \chi_{l(i)}, \delta)$ of pair of genotypes given the peak heights in the profile. This probability may be computed using Bayes theorem. The probability may be computed as:

$$p(g^*_{U,1,l(i)}, g^*_{U,2,l(i)} \middle| C_{l(i)}, \chi_{l(i)}, \delta) = \frac{f\left(c_{l(i)} \middle| g^*_{U,1,l(i)}, g^*_{U,2,l(i)}, \chi_{l(i)}, \delta\right) p\left(g^*_{U,1,l(i)}, g^*_{U,2,l(i)}\right)}{\sum_{g_{U,1,l(i)}, g_{U,2,l(i)}} f\left(c_{l(i)} \middle| g_{U,1,l(i)}, g_{U,2,l(i)}, \chi_{l(i)}, \delta\right) p\left(g_{U,1,l(i)}, g_{U,2,l(i)}\right)}$$

The method may assume that the prior probability for the pair of genotypes is the same for any genotype combination in the locus. The method may state the probability as:

$$p(g^*_{U,1,l(i)}, g^*_{U,2,l(i)} \middle| C_{l(i)}, \chi_{l(i)}, \delta) = \frac{f\left(c_{l(i)} \middle| g^*_{U,1,l(i)}, g^*_{U,2,l(i)}, \chi_{l(i)}, \delta\right)}{\sum_{g_{U,1,l(i)}, g_{U,2,l(i)}} f\left(c_{l(i)} \middle| g_{U,1,l(i)}, g_{U,2,l(i)}, \chi_{l(i)}, \delta\right)}$$

The pdf for the peak heights given a pair of putative genotypes may be calculated using the formula below:

$$f\left(c_{l(i)} \middle| g_{U,1,l(i)}, g_{U,2,l(i)}, \chi_{l(i)}, \delta\right) = \sum_{\omega} f\left(c_{l(i)} \middle| g_{U,1,l(i)}, g_{U,2,l(i)}, \chi_{l(i)}, \delta, \omega\right) p(\omega)$$

where $\omega$ is the mixing proportion.

The method may provide that not all pair of genotypes will have a non-zero probability and/or be calculated. The method may use the crime profile to guess pair of genotypes that may have zero probability. The method may designate peaks in the crime profile as alleles or stutters. The genotypes may be produced based on the peaks designated as alleles. One or more of the following cases may be considered in the method:

1. No peaks bigger than the reporting threshold $T_r$ and/or no genotypes are generated.

2. One peak bigger than $T_r$ is designated as allele, denoted by *a* and/or there are two possible genotypes {a, a} and a, Q} where Q denoted any allele other than *a* and/or any pairing of these genotypes is possible: ({a, a}, {a, a}), ({a, a}, {a, Q}) and ({a, Q}, {a, Q}).

3. Two peaks bigger than $T_r$ designated as alleles, denoted by *a* and *b* and/or the possible genotypes are {a, a}, {a, b}, {a, Q}, {b, b}, {b, Q} and {Q, Q} where Q is any allele other than *a* and *b* and/or any combination of pair of genotypes whose union contains *a, b* is a possible pair of genotypes: {a, a} with any genotype that contains *b;* {a, b} with any genotype in the list; {a, Q} with any genotype that contains *b;* {b, b} with any genotype that contains *a;* {b, Q} with any genotype that contains *a;* and {Q, Q} with {a, b}.

4. Three peaks bigger than $T_r$, denoted by *a, b* and *and/or t*his case follows exactly the same logic as in the case above and/or there are 10 genotypes that are possible from allele set: *a, b, c* and *Q,* where *Q* denotes any allele other than *a, b* and *and/or* all genotype pairs whose union contains *a, b, c.*

5. Four peaks bigger than $T_r$ are designated as alleles, denoted by *a, b, c* and *d* and/or in this case there are 10 possible pair of genotypes and/or any genotype pair whose union is *a, b, c, d* is considered as a possible genotype pair.

The interest may lie on genotype pairs such that the first and second genotype corresponds to the major and minor contributor respectively. The calculation of the posterior probabilities in this section may be done for all possible combinations of genotypes and mixing proportions. Moving from all combinations of genotypes to major minor may require folding the space of all combinations of genotypes and mixing proportions in two.

The method may consider:

$$\Pr\left(G_M = g_1, G_m = g_2 \middle| c_{l(i)}\right) = \sum_{\omega \in \Omega \geq 0.5} \Pr\left(G_M = g_1, G_m = g_2 \middle| c_{l(i)}, \omega\right) p(\omega)$$

where $\Omega \geq 0.5$ is a discreet set of mixing proportions greater or equal to 0.5. When $\omega > 0.5$ the first factor in the summation in the above equation may be:

$$\Pr\left(G_M = g_1, G_m = g_2 \middle| c_{l(i)}\omega\right) = \Pr\left(G_1 = g_1, G_2 = g_2 \middle| c_{l(i)}, \omega\right)$$

$$+ \Pr\left(G_1 = g_2, G_2 = g_1 \middle| c_{l(i)}, 1 - \omega\right)$$

$$= 2x\Pr\left(G_1 = g_1, G_{2m} = g_2 \middle| c_{l(i)}, \omega\right)$$

If $\omega = 0.5$:

$$\Pr\left(G_M = g_1, G_m = g_2 \middle| c_{l(i)}\omega\right) = \Pr\left(G_1 = g_1, G_2 = g_2 \middle| c_{l(i)}, \omega\right).$$

The method, particularly for mixed source samples, may consider the mixing proportion involved. The posterior probability of the mixing proportion given the peaks heights across all loci may be used, and may be expressed as:

$$f\left(c_{l(i)}\middle|g_{U,1,l(i)},g_{U,2,l(i)}\right)=\sum_{\omega}f\left(c_{l(i)}\middle|g_{U,1,l(i)},g_{U,2,l(i)},\omega\right)p\left(\omega\middle|c_{l(1)},c_{l(2)},......c_{l(nLoci)}\right)$$

The method may provide that for each locus $l(i)$ it generates a set of possible genotype pairs of potential contributors of the crime profile $c_{l(i)}$. The $j$-th instance of the genotype of the contributor 1 and 2 may be denoted by $g_{U1,j,l(i)}$ and $g_{U2,j,l(i)}$, respectively, where $n_g$ is the number of genotype pairs. The method may calculate the posterior probability of pair of genotypes given the peak heights in the crime profile $c_{l(i)}$. The calculation may use a probability distribution for mixing proportion. A sequential method for calculating the posterior distribution of mixing proportion given peak heights across loci may be used.

The mixing proportion may be a continuous quantity in the interval (0, 1). A discrete probability distribution may be used. Assume that we have mixing proportions $\omega = \{\omega_k : k = 1,...,n_\omega\}$, where $n_\omega$ is the number of mixing proportions considered, the method may set a prior distribution for mixing proportion as uniform over the discrete values. Using Bayes theorem, the posterior distribution for mixing proportion given the peak heights in locus i may be:

$$p(\omega_k|c_{l(1)},\chi_{l(i)},\delta)=\frac{f\left(c_{l(1)}\middle|\chi_{l(1)},\omega_k,\delta\right)p(\omega_k)}{\sum_{m=1}^{n_\omega}f\left(c_{l(1)}\middle|\chi_{l(1)},\delta,\omega_m\right)p\left(\omega_m\right)}$$

The posterior distribution of mixing proportion for locus i, i = 2, 3, ...,$n_L$ may be given by:

$$p(\omega_k|c_{l(1)},....,c_{l(i)},\chi_{l(1)},..........\chi_{l(i)},\delta)=\frac{f\left(c_{l(i)}\middle|\chi_{l(i)},\omega_k,\delta\right)p(\omega_k)\middle|c_{l(1)},....,c_{l(i-1)},\chi_{l(1)},..........\chi_{l(i-1)},\delta}{\sum_{m=1}^{n_\omega}f\left(c_{l(i)}\middle|\chi_{l(i)},\delta,\omega_m\right)p\left(\omega_m\middle|c_{l(1)},....,c_{l(i-1)},\chi_{l(1)},..........\chi_{l(i-1)},\delta\right)}$$

where $f(\omega_k|c_{l(i)},\chi_{l(i)})$, i = 1,2, ......$n_L$ is defined in the following paragraph. If there are any loci with no information they may be ignored in the calculation as having no information.

The probability density of the peak height in the crime profile $c_{l(i)}$ at locus $l(i)$ for a given mixing proportion $\omega_k$ may be given by:

$$f(c_{l(i)}|\chi_{l(i)},\omega_k)=\sum_{j=1}^{n_g}f\left(c_{l(i)}\middle|g_{U1,j,l(i)},g_{U2,j,l(i)},\chi_{l(i)},\omega_k\right)p\left(g_{U1,j,l(i)},g_{U2,j,l(i)}\right)$$

where $p\left(g_{U1,j,l(i)},g_{U2,j,l(i)}\right)$ is the probability of the two genotypes prior to observing the crime profile. This may be based

$$p\left(g_{U1,j,l(i)},g_{U2,j,l(i)}\right)=\frac{1}{n_g}$$

on the assumption of an equal probability of all genotype pairs:

This $\dfrac{1}{n_g}$ may cancel out in the following equations and it may thus be ignored. Then the probability density in the above equation may simplify to:

$$f(c_{l(i)}|\chi_{l(i)},\omega_k)=\sum_{j=1}^{n_g}f\left(c_{l(i)}\middle|g_{U1,j,l(i)},g_{U2,j,l(i)},\chi_{l(i)},\omega_k\right)$$

The consideration may include the use of the function $f(c_{l(i)}|g_{1,l(i)},g_{2,l(i)},\omega,\chi_{l(i)},\delta)$

The method may construct the pdf using one or more or all of the following steps:

**Step 1** The associated peak-height sum for donor 1 may be $\omega \times \chi_{l(i)}$. The corresponding means for the peak height of the alleles and the stutters of this donor may be denoted by $\mu_{a,1,l(i)}$ and $\mu_{s,1,l(i)}$, respectively. They may be obtained as a function of $\chi_{D2}$. For each allele $a$ of donor 1, we may assign the allele mean $\mu_{a,1,l(i)}$ to the position of allele $a$, and the stutter mean $\mu_{s,1,l(i)}$ to position $a - 1$. If the donor is homozygote, we may do the assignment twice.

(continued)

| | | |
|---|---|---|
| Step 2 | The associated peak-height sum for donor 2 may be $(1 - \omega) \times \chi_{l(l)}$ with associated mean for allele and stutters: $\mu_{a,2,l(l)}$ and $\mu_{s,2,l(l)}$. The assignment of means may be done as in step 1. | |
| Step 3 | If a donor is a heterozygote, the means may be modified to take into account factors, such as PCR efficiency and degradation, that affect the resulting peak heights. For example, if donor 1 is a heterozygote in locus $l$ (i), the mean for his/her alleles and stutters may be: $\delta_1 \times \mu_{a,1,l(l)}$ and $\delta_1 \times \mu_{s,1,l(l)}$ for the low-molecular-weight allele and $\delta_2 \times \mu_{a,1,l(l)}$ and $\delta_2 \mu_{s,1,l(l)}$ for the high-molecular-weight allele. | |
| Step 4 | The variances for each allele and stutter may be obtained as a function of means. In later step we may need to add random Gamma variables. A condition for a close form calculation may be that the β-parameters are the same. Also in a later step, we may divide each Gamma by the overall sum of peak height to account for using the sum of peak heights in this locus. A closed form calculation can be done if all β parameters are the same. The conditioned on the β-parameters can be obtained by estimating a line between the points formed by the means, in the x-axis, and the variances, in the y-axis. A regression line with zero intercept may be fitted to obtain: $$\hat{\sigma}^2 = \kappa_2 x \mu$$ So, if peak i has mean and variance $\left(\mu_i, \sigma_i^2\right),$ $$\beta = \mu_i \big/ \sigma_i^2 = \mu_i \big/ \left(\kappa_2 x \mu_i\right) = 1 \big/ \kappa_2$$ regardless of the value of $\mu_i$. | |
| Step 5 | The shape ($\alpha$) and rate (β) parameters may be obtained from the mean ($\mu$) and the variances $(\hat{\sigma}^2)$ using the known A formulae $\alpha = (\mu/\hat{\sigma}^2)^2$ and $\beta = \mu/\hat{\sigma}^2$. | |
| Step 6 | The alpha parameters for alleles and stutters in the same allele position may be added to obtain an overall $\alpha$ for that position. | |
| Step 7 | To account for using the sum of peak height in the locus, the collection of Gamma pdf's whose peak heights are above the peak-height reporting limit may be converted to a Dirichlet pdf. This may be achieved in closed form because all β's are the same. The resulting Dirichlet pdf may inherit the $\alpha$ parameters of the Gammas. | |

The method may include that the peaks in the crime profile are either bigger or smaller than the reporting threshold $T_r$, or not present at all. The method may treat missing peaks and peaks smaller than $T_r$ as peak that has dropped out. The method may consider the crime profile for a given pair of genotype as:

$$c_{l(i)} = \left\{ h : h \in c_{l(i)}, h < T_r \right\} \cup \left\{ h : h \in c_{l(i)}, h \geq T_r \right\}$$

The resulting pdf may be given by:

$$f(c_{l(i)} | g_{1,l(i)}, g_{2,l(i)}, \chi_{l(i)}, \delta, \omega) = f(\pi | \alpha) x \prod_{\{h : h \in c_{l(i)}, h < T_r\}} F(T | \alpha_h, \beta_h)$$

The terms are explained below:
$f(\pi|\alpha)$ may be a Dirichlet pdf with parameters

$$\alpha = \cup \left\{ \alpha_h : h \in c_{l(i)}, h \geq T_r \right\}$$

and

$$\pi = \cup \left\{ h \big/ \chi'_{l(i)} : h \in c_{l(i)}, h \geq T_r \right\}$$

where $\alpha_h$ may be the alpha parameter of the associated Gamma pdf in the corresponding position of height $h$.

$$\chi'_{l(i)} = \sum_{h \in c_{l(i)}, h \geq T_r}$$

$h$ may be the sum of peak heights bigger than reporting threshold $T_r$. $F(T_r|\alpha_h, \beta_h)$ may be the CDF of a Gamma distribution with parameters $\alpha_h$ and $\beta_h$ for the peak in the position of $h$

calculated as described above.

The method may provide that in locus $l(i)$ there are the set of peak heights $h_{l(i)} = \{h_{j,l(i)} : j = 1,...,n_{l(i)}\}$. Each height may have an associated base pair count $b_{l(i)} = \{b_{j,l(i)} : j = 1,...,n_{l(i)}\}$. An average base pair count, weighted by peak heights, may be used as a measure of molecular weight for the locus. More specifically, this may be defined as:

$$\bar{b}_{l(i)} = \frac{\sum_{j=1}^{n} b_{j,l(i)} h_{j,l(i)}}{\chi_{l(i)}}$$

where

$$\chi_{l(i)} = \sum_{j=1}^{n_{l(i)}} h_{j,l(i)}$$

We may define the EAQ model as: $\chi_{l(i)} = d_1 + d_2 \bar{b}_{l(i)}$ where $d_1$ and $d_2$ are the same for all loci.

The sum of peak heights $\chi_{l(i)}$ may be assumed to be a linear function of the weighted base-pair average,

$$\bar{b}_{l(i)} = \frac{\sum_{j=1}^{n} b_{j,l(i)} h_{j,l(i)}}{\chi_{l(i)}}.$$

The method may provide a calculation of the parameters $d_1$ and $d_2$, for instance by calculated using least squared estimation. However some loci may behave differently, and therefore the sum of peak heights of these loci can be treated as outlier. We may use a Jackknife method to deal with this problem. If there are $n_L$ loci with peak height and base pair information, then the method may use one or more or all of the following steps..

1. Fit the regression model $n_L$ times, removing the $i^{th}$ value of the sets $\{\chi_{l(i)} : i = 1,...,n_L\}$ and $\{b_{l(i)} : i = 1,...n_L\}$.
2. Use least-squares estimation to produce a prediction interval, $\chi_{l(i)} \pm 2\sigma$, where $\sigma$ is the standard deviation of the residuals in the fitted regression line.
3. If the sum of peak height $\chi_i$ which was not used for the estimation of the regression line, does not lie within the prediction interval then we consider it an outlier.
4. After the $n_L$ models have been produced we remove any outliers from the dataset and re-fit the model.
5. The values of $d_1$ and $d_2$ are extracted from the model estimated without outliers.
6. If the degradation in the profile is negligible, peak height variability may cause the estimated value of $d_2$ to be greater than zero. In this case we set $d_2 = 0$ and $d_1 = 1$.

The method may include use of the peak imbalance parameter or EAQ model for taking into account EAQ within a locus. EAQ between loci may be taken into account by conditioning on the sum of peak height per locus. The EAQ model may be used when the pdf of the peak heights for single and two-person profiles is deployed. More specifically, it may be deployed for each heterozygote donor.

Assume that at locus $l(i)$ we have a putative heterozygote donor with alleles $a_1,l(i)$ and $a_2,l(i)$ with corresponding molecular weights in base pairs $b_1, l(i)$ and $a_2, l(i)$, respectively, the method may include one or more of: if we were not considering EAQ, given the sum of peak heights $\chi_{l(i)}$ for this locus we can obtain a mean $\mu_{l(i)}$ and variance $\sigma^2_{l(i)}$ of a Gamma distribution that models the behaviour of a peak height; if $H_{j,l(i)}$ denotes the random variable for the height corresponding the allele $s_{j,l(i)}$, then:

$$H_{j,l(i)} \: \Gamma\left(\mu_{l(i)}, \sigma^2_{l(i)} \Big| \chi_{l(i)}\right), j = 1, 2.$$

The same Gamma pdf may be used for any allele in the locus. The EAQ model issued may adapt the Gamma pdf by taking into account the molecular weight of the allele. The EAQ model may be used to calculate a pair of factors $\delta_1$ and $\delta_2$ so that the mean values of the Gamma distribution are adjusted accordingly. The new mean may be given by:

$$\mu_{j,l(i)} = \delta \; x\mu_{l(i)}, j = 1, 2.$$

The method may include a method for calculating $\delta_1$ and $\delta_2$ using the slope $d_2$ of the EAQ regression line. The first condition that the $\delta$'s must fulfil may be that the slope of a line going through the coordinates $(b_{1,l(i)}, \mu_{1,l(i)})$ and $(b_{2,l(i)}, \mu_{2,l(i)})$ is the same as the slope $d_2$ of the EAQ regression line, i.e.:

$$\frac{\mu_{1,l(i)} - \mu_{2,l(i)}}{b_{1,l(i)} - b_{2,l(i)}} = d_2$$

The second condition that the $\delta$'s must fulfilled may be the preservation of the mean $\mu_{l(i)}$:

$$\frac{\mu_{1,l(i)} - \mu_{2,l(i)}}{2} = \mu_{l(i)}$$

Substituting $\mu_{j,l(i)} = \delta x\mu_{l(i)}$, $j = 1, 2.$ in $\dfrac{\mu_{1,l(i)} - \mu_{2,l(i)}}{b_{1,l(i)} - b_{2,l(i)}} = d_2$ and $\dfrac{\mu_{1,l(i)} - \mu_{2,l(i)}}{2} = \mu_{l(i)}$ we obtain two equations with two unknowns $\delta_1$ and $\delta_2$. The solution of the equations may be:

$$\delta_1 = \frac{d_2(b_{1,l(i)} - b_{2,l(i)} + 2\mu_{l(i)}}{2\mu_{l(i)}}$$

$$\delta_2 = 2 - \delta_1$$

The stutter associated with the allelic peak may be treated as having the same degradation factor because it is the starting DNA molecules of the allele that is affected by degradation.

According to a second aspect of the invention we provide a method of comparing a first, potentially test, sample result set with a second, potentially another, sample result set, the method including:

provide information for the first result set on the one or more identities detected for a variable characteristic of DNA; providing information for the second result set on the one or more identities detected for a variable characteristic of DNA; and

wherein the method uses in the definition of the likelihood ratio the factor: $f(C_{l(i)}|g_{l(i)}, \chi_{l(i)}, \delta)$.

The second aspect of the invention may include any of the features, options or possibilities set out elsewhere in this document, including in the other aspects of the invention.

According to a third aspect of the invention we provide a method of comparing a first, potentially test, sample result set with a second, potentially another, sample result set, the method including:

providing information for the first result set on the one or more identities detected for a variable characteristic of DNA;
providing information for the second result set on the one or more identities detected for a variable characteristic of DNA; and

wherein the method uses as the definition of the likelihood ratio the factor:

$$f\left(c_{l(i)} \middle| g_{U,1,l(i)}, g_{U,2,l(i)}, \chi_{l(i)}, \delta\right).$$

The third aspect of the invention may include any of the features, options or possibilities set out elsewhere in this document, including in the other aspects of the invention.

According to a fourth aspect of the invention we provide a method of comparing a first, potentially test, sample result set with a second, potentially another, sample result set, the method including:

providing information for the first result set on the one or more identities detected for a variable characteristic of DNA;
providing information for the second result set on the one or more identities detected for a variable characteristic of DNA; and

wherein the method uses in the definition of the likelihood ratio the factor:

$$f(c_{l(i)} \middle| g_{1,l(i)}, g_{2,l(i)}, \omega, \chi_{l(i)}, \delta).$$

The fourth aspect of the invention may include any of the features, options or possibilities set out elsewhere in this document, including in the other aspects of the invention.

According to a fifth aspect of the invention we provide a method for generating one or more probability distribution functions relating to the detected level for a variable characteristic of DNA, the method including:

a) providing a control sample of DNA;
b) analysing the control sample to establish the detected level for the at least one variable characteristic of DNA;
c) repeating steps a) and b) for a plurality of control samples to form a data set of detected levels;
d) defining a probability distribution function for at least a part of the data set of detected levels.

The method may particularly be used to generate one or more of the probability distribution functions provided elsewhere in this document.

The method may be used to generate one or more probability distributions related to the effect of one or more of: a factor accounting for one of more effects which impact upon the amount of an allele, for instance a height and/or area observed for a sample compared with the amount of the allele in the sample; the effect may be one or more effects which gives a different ratio and/or balance and/or imbalance between observed and present amounts with respect to different alleles and/or different loci; the effect may be and/or include degradation effects; the effect may be and/or include variations in amplification efficiency; the effect may be and/or include variations in amount of allele in a sub-sample of a sample, for instance, when compared with other sub-samples and/or the sample; the effect may be one whose effect varies with alleles and/or loci and/or allele size and/or locus size; the effect may be an effect which causes a reduction in the observed amount compared with that which would have occurred without the effect; the effect may exclude any stutter effect; and in particular their variation with DNA quantity. The DNA quantity may be with respect to an allele and/or allele and stutter and/or two or more alleles and/or two or more stutters and/or the alleles and/or stutters for one or more loci.

The one or more probability distributions may be generated from feed data.

The feed data may be obtained experimentally. The feed data may be obtained by computer modelling.

The experimental determination of the feed data may include one or more of: a sampling step; a dilution step, preferably to provide a range of different dilutions; a purification step; a pooling of samples step; a division of samples step; an amplification step, such as PCR; a detection step, for instance of one of more characteristic units introduced to the amplification products, such as dyes; an electrophoreis step; an interpretation step; a peak identification step; a peak height and/or area determination step.

The number of samples may be greater than 30, preferably greater than 50 and ideally greater than 100. The number of profiles obtained from samples may be greater than 500, preferably greater than 750 and ideally greater than 1000. The samples may be diluted to less than 1000 picograms per microlitre. The samples may be at least 25 picograms per microlitre. The dilution range may be between preferably 10 to 1000 picograms per microlitre, more preferably 50 to 500 picograms per microlitre. The dilutions may be provided in increments of between 10 and 100 pg/$\mu$l, for instance of 25 pg/$\mu$l. One or more process protocols may be used to process samples.

The experimental determination may include or further include combining, for instance through addition, one or more of the heights and/or areas for one or more of the loci. The combination may be used to provide a measure of DNA quantity. All of the heights and/or areas from one or more loci may be combined. All of the heights and/or areas from all of the loci, or all bar one of the loci, may be combined.

The experimental determination may include or may further include combining, for instance through addition, all of the heights and/or areas for a locus. The combination may provide a measure of DNA quantity for the locus. The combination may provide a mean height and/or area for the locus and/or one or more alleles of the locus.

The experimental determination may include or may further include obtaining a mean height and./or area for one or more alleles and/or one or more loci. Such a separate mean height and/or area may be obtained for each locus.

The experimental determination may include or may further include a consideration and/or plot of mean height and/or area against DNA quantity, preferably on a locus basis. Such a consideration and/or plot may be provided for two or more and preferably all loci. The DNA quantity may be subject to a scaling factor, such as a multiplier.

The experimental determination may include or may further include fitting a distribution to the feed data, particularly a consideration and/or plot of mean height and/or area against DNA quantity. The fitted distribution may be a linear Gamma distribution. The fitted distribution may pass through the origin. The distribution may be specified through two parameters, preferably the shape parameter $\alpha$ and the rate parameter $\beta$.

The experimental determination may include or may further include fitting one or more distributions to the feed data, particularly a consideration and/or plot of mean height and/or area against DNA quantity for one or more of the alleles and/or one or more of the stutters of alleles.

The experimental determination may include or may further include a consideration and/or plot of variance against mean height and/or area, preferably on a locus basis. Such a consideration and/or plot may be provided for two or more and preferably all loci.

The experimental determination may include or may further include fitting one or more distributions to the feed data, particularly a consideration and/or plot of variance against mean height. The fitted distribution may be one or more a Gamma distributions. The fitted distribution may pass through the origin. The distribution may be specified through two parameters, preferably the shape parameter $\alpha$ and the rate parameter $\beta$. The fitted distribution may be provided by two different distributions, for instance connected by a knot. The distributions may be two quadratic polynomials, preferably joined in a chosen knot. The knot may chosen through experimenting with several candidates and selecting candidates that give a best and/or good fit. The distribution may be of the form, if $\mu \leq knot$: $\sigma^2 = \kappa_{2,1,l(i)} x\mu + \kappa_{3,1l(i)} x\mu^2$ and/or if $\mu > knot$: $\sigma^2 = \kappa_{2,2,l(i)} x\mu + \kappa_{3,2l(i)} x\mu^2$.

The experimental determination may include or may further include fitting one or more distributions to the feed data, particularly a consideration and/or plot of variance against mean height and/or area for one or more of the alleles and/or one or more of the stutters of alleles.

The experimental determination may include or may further include providing that the $\beta$ values for one or more of the distributions be the same. In particular, the $\beta$ values for the distribution(s) of variance against mean height and/or area may be the same across two or more loci, and preferably all the loci or all bar one loci. The condition: $\sigma^2 = k_2 x\mu$

and/or $\beta = \dfrac{\mu}{\sigma^2} = \dfrac{\mu}{k_2 . \mu} = \dfrac{1}{k^2}$ may be met for one or more of the distributions and/or one or more loci.

The method may include or further include use of an algorithm to estimate the parameters of the mean and variance models. The values of the parameters in iteration $m$ of the algorithm may be denoted by:

$$\kappa_{1,a,l(i)}[m], \kappa_{2,1,l(i)}[m], \kappa_{3,1,l(i)}[m], \kappa_{2,2,l(i)}[m], \kappa_{3,2l(i)}[m],$$

Preferably in the first iteration of the algorithm zeros (for instance those heights and/or areas smaller than a threshold) may be ignored. Preferably in and/or from the second iteration of the algorithm onwards, the zeros are replaced by samples obtained from the tail of the Gamma pdfs estimated in the previous step. In particular, one or more of the following may be applied:

1. Parameter $k_{1,a,l(i)}[1]$ is estimated using standard linear regression methods where the response variable are non-zero allele heights and the covariate is the corresponding $\chi$.
2. Parameters $k_{2,1,l(i)}[1]$, $k_{3,1,l(i)}[1]$, $k_{2,2,l(i)}[1]$, $k_{3,2,l(i)}[1]$ are estimated by using the estimated mean $\mu_{a,l(i)}$ as the mean and computing the variance of the heights around these mean according to a window size.

3. In iteration *m,* zeros are replaced by samples taken from the family of Gamma distribution estimated in the previous iteration.

4. For a zero corresponding to $\chi$, we first obtain a $\mu_{a,l(i)}[m-1]$ from $\chi$ and $\sigma^2_{a,l(i)}[m-1]$ from $\mu_{a,l(i)}[m-1]$.

5. Parameter $\alpha[m-1]$ and $\beta[m-1]$ can be computed from $\mu_{a,l(i)}[m-1]$ and $\sigma^2_{a,l(i)}[m-1]$.

6. A sample is then taken in the interval (0, 30) from the tail of the distribution using the CDF inverse method using uniform samples in the interval (0, $F$(30, $\alpha[m-1]$, $\beta[m-1]$)) where $F$ is the CDF of a Gamma distribution.

7. Parameter $k_{1,a,l(i)}[m]$ is also estimated using standard linear regression methods where the response variable are allele heights and the covariate are the corresponding $\chi$'s.

8. Zeros are replaced using the method described above.

9. Parameters $k_{2,1,l(i)}[m]$, $k_{3,1,l(i)}[m]$, $k_{2,2,l(i)}[m]$, $k_{3,2,l(i)}[m]$ are estimated by using the estimated mean $\mu_{a,l(i)}[m]$ as the mean and computing the variance of the heights around these mean according to a window size.

10. The process is repeated until the parameters converge according to the rules:

(a) $|k_{1,a,l(i)}[m] - k_{1,a,l(i)}[m-1]| < 0.0001$;
(b) $|k_{2,a,l(i)}[m] - k_{2,a,l(i)}[m-1]| < 0.01$; and
(c) $|k_{3,a,l(i)}[m] - k_{3,a,l(i)}[m-1]| < 0.001$.

The method may include or further include use of an algorithm to estimate the parameters of the mean and variance models for both the alleles and stutters, with one or more of the same features being used for both and/or the same algorithm being used for both.

The fifth aspect of the invention may include any of the features, options or possibilities set out elsewhere in this document, including in the other aspects of the invention.

Any of the proceeding aspects of the invention may include the following features, options or possibilities or those set out elsewhere in this document.

The terms peak height and/or peak area and/or peak volume are all different measures of the same quantity and the terms may be substituted for each other or expanded to cover all three possibilities in any statement made in this document where one of the three are mentioned.

The method may be a computer implemented method.

The method may involve the display of information to a user, for instance in electronic form or hardcopy form.

The test sample, may be a sample from an unknown source. The test sample may be a sample from a known source, particularly a known person. The test sample may be analysed to establish the identities present in respect of one or more variable parts of the DNA of the test sample. The one or more variable parts may be the allele or alleles present at a locus. The analysis may establish the one or more variable parts present at one or more loci.

The test sample may be contributed to by a single source. The test sample may be contributed to by an unknown number of sources. The test sample may be contributed to by two or more sources. One or more of the two or more sources may be known, for instance the victim of the crime.

The test sample may be considered as evidence, for instance in civil or criminal legal proceedings. The evidence may be as to the relative likelihoods, a likelihood ratio, of one hypothesis to another hypothesis. In particular, this may be a hypothesis advanced by the prosecution in the legal proceedings and another hypothesis advanced by the defence in the legal proceedings.

The test sample may be considered in an intelligence gathering method, for instance to provide information to further investigative processes, such as evidence gathering. The test sample may be compared with one or more previous samples or the stored analysis results therefore. The test sample may be compared to establish a list of stored analysis results which are the most likely matches therewith.

The test sample and/or control samples may be analysed to determine the peak height or heights present for one or more peaks indicative of one or more identities. The test sample and/or control samples may be analysed to determine the peak area or areas present for one or more peaks indicative of one or more identities. The test sample and/or control samples may be analysed to determine the peak weight or weights present for one or more peaks indicative of one or more identities. The test sample and/or control samples may be analysed to determine a level indicator for one or more identities.

Various embodiments of the invention will now be described, by way of example only, and with reference to the accompanying drawings, in which:

Figure 1 shows a Bayesian network for calculating the numerator of the likelihood ratio; the network is conditional on the prosecution view $V_p$. The rectangles represent know quantities. The ovals represent probabilistic quantities.

Arrows represent probabilistic dependencies, e.g. the PDF of $C_{L(1)}$ is given for each value of $g_{s,L(1)}$ and $\chi$.

Figure 2a illustrates an example of a profile for a homozygous source;

Figure 2b is a Bayesian Network for the homozygous position;

Figure 2c is a further Bayesian Network for the homozygous position;

Figure 2d shows homozygote peak height as a function of DNA quantity; with the straight line specified by $\overline{h}$ = -12.94 + 1.27 $\times$ $\chi$.

Figure 2e shows the parameters of a Beta PDF that model stutter proportion $\pi_s$ conditional on parent allele height $h$.

Figure 3a illustrates an example of a profile for a heterozygous source whose alleles are in non-stutter positions relative to one another;

Figure 3b is a Bayesian Network for the heterozygous position with non-overlapping allele and stutter peaks;

Figure 3c is a further Bayesian Network for the heterozygous position with non-overlapping allele and stutter peaks;

Figure 3d

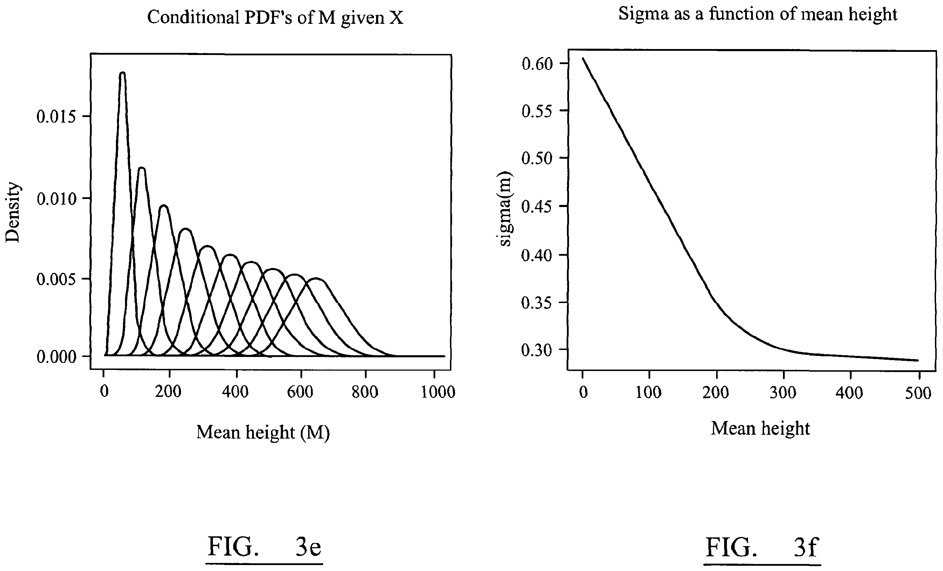

Figure 3e shows the variation in density with mean height for a series of Gamma distributions;

Figure 3f shows the variation of parameter $\sigma$ as a function of mean height $m$ ;

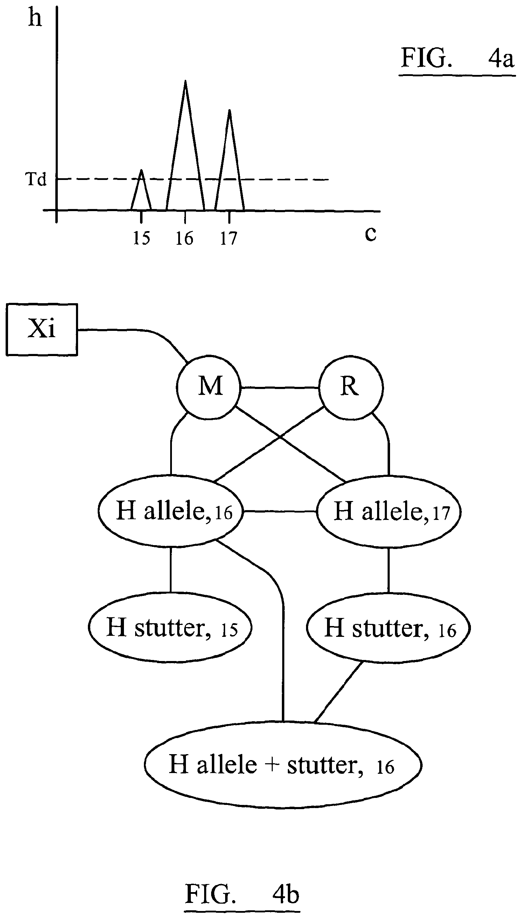

Figure 4a illustrates an example of a profile for a heterozygous source whose alleles include alleles in stutter positions relative to one another;

Figure 4b is a Bayesian Network for the heterozygous position with overlapping allele and stutter peaks;

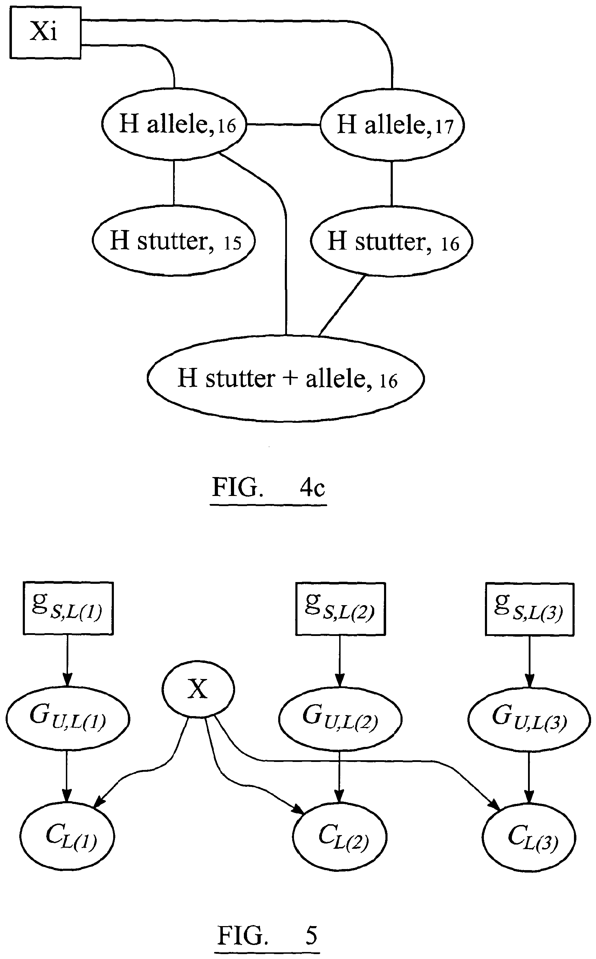

Figure 4c is a further Bayesian Network for the heterozygous position with overlapping allele and stutter peaks;

Figure 5 shows a Bayesian network for calculating the denominator of the likelihood ratio. The network is conditional on the defence hypothesis $V_d$. The oval represent probabilistic quantities whilst the rectangles represent known quantities. The arrows represent probabilistic dependencies;

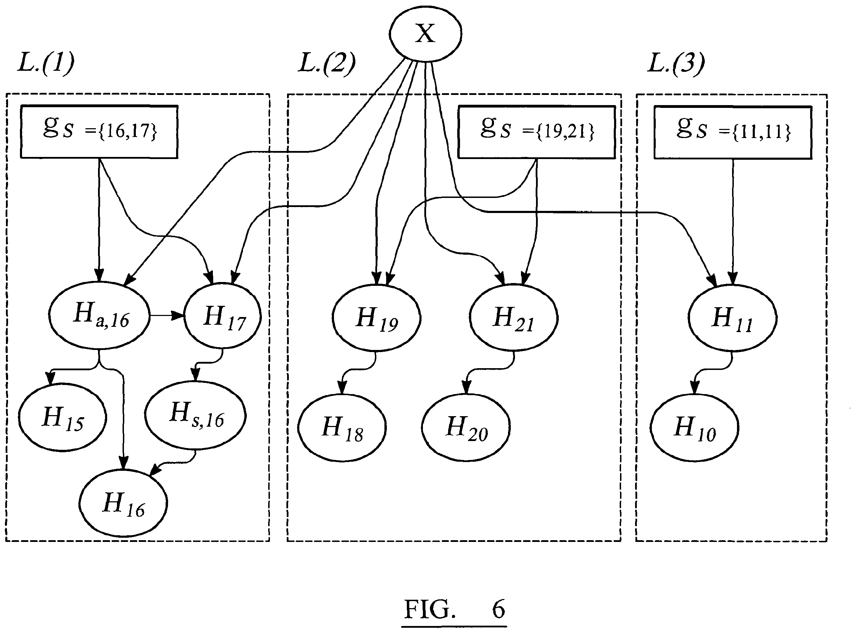

Figure 6 shows a Bayesian Network for calculating likelihood per locus in a generic example;

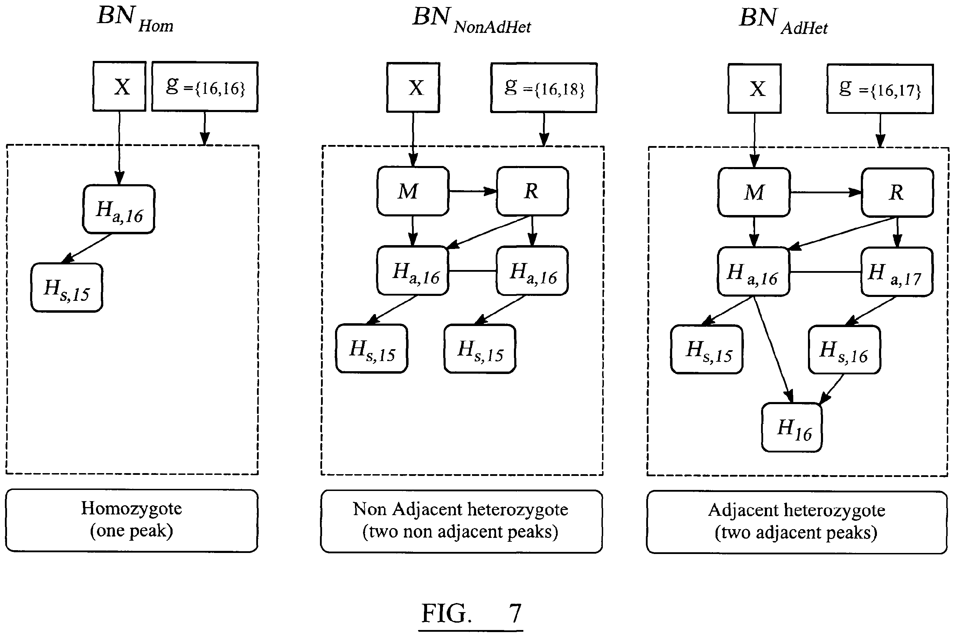

Figure 7 shows Bayesian Networks for three allele situations;

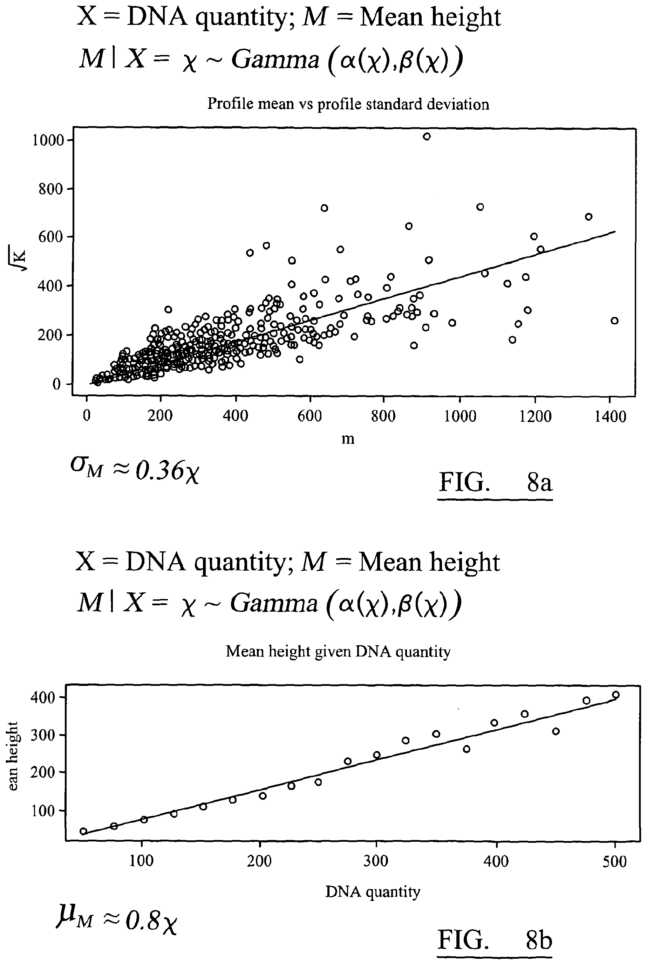

Figure 8a is a plot of profile mean against profile standard deviation;

Figure 8b is a plot of mean height against DNA quantity;

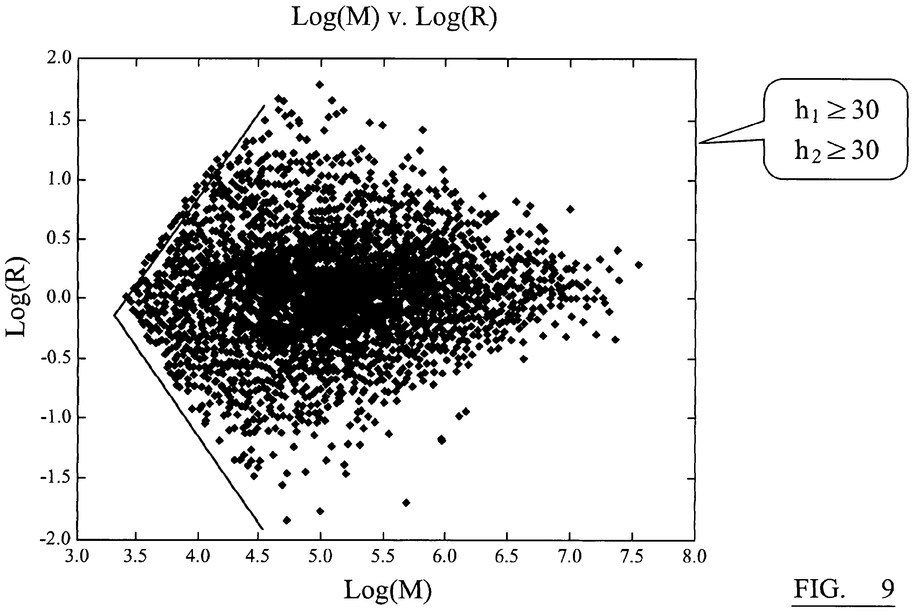

Figure 9 a pdf for R/M=m;

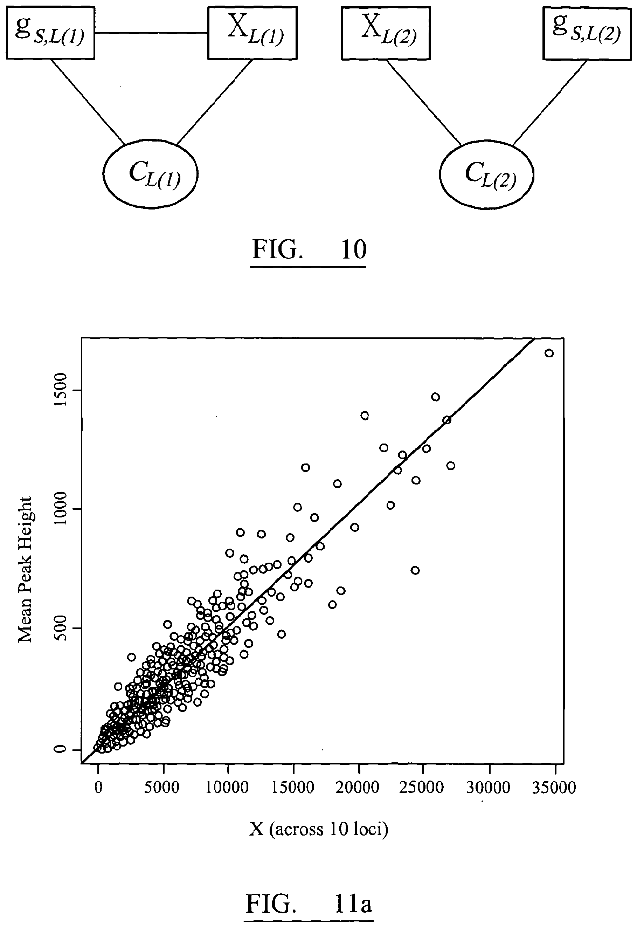

Figure 10 shows a Bayesian Network for part of the degradation consideration;

Figure 11a is a plot of mean peak height against DNA quantity x10;

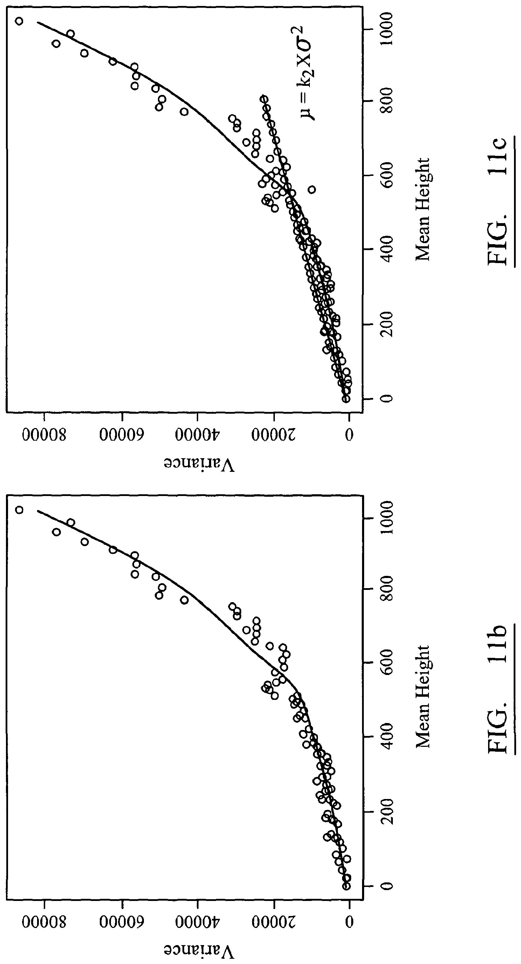

Figure 11b is a plot of variance against mean height;

Figure 11c is a plot of variance against mean height with a regression fitted;

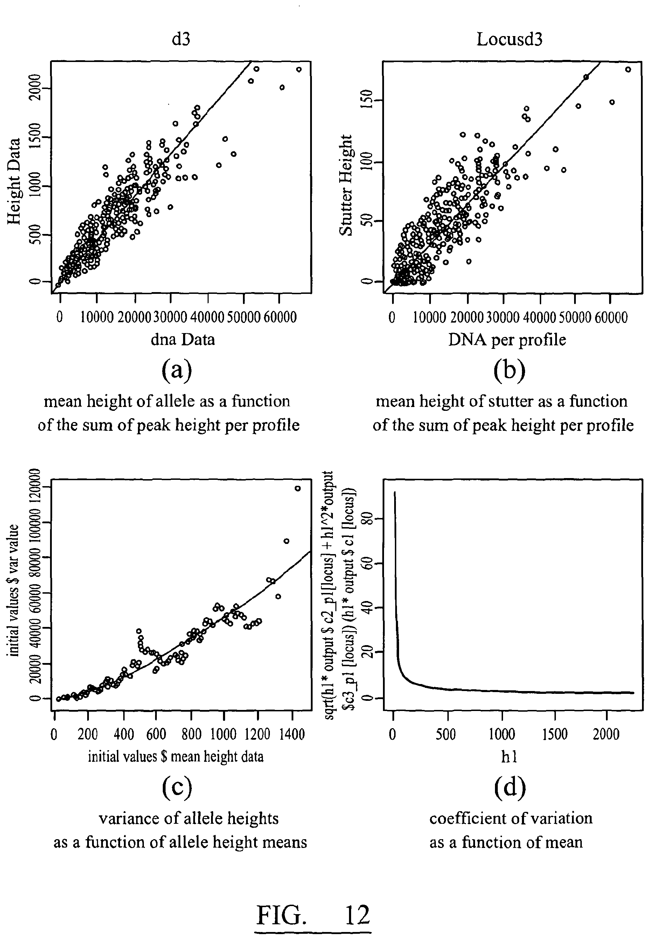

Figure 12 shows plots of allele mean peak height against DNA quantity x10, stutter mean peak height against DNA quantity x10, variance against mean height and coefficient of variation as a function of mean for locus D3;

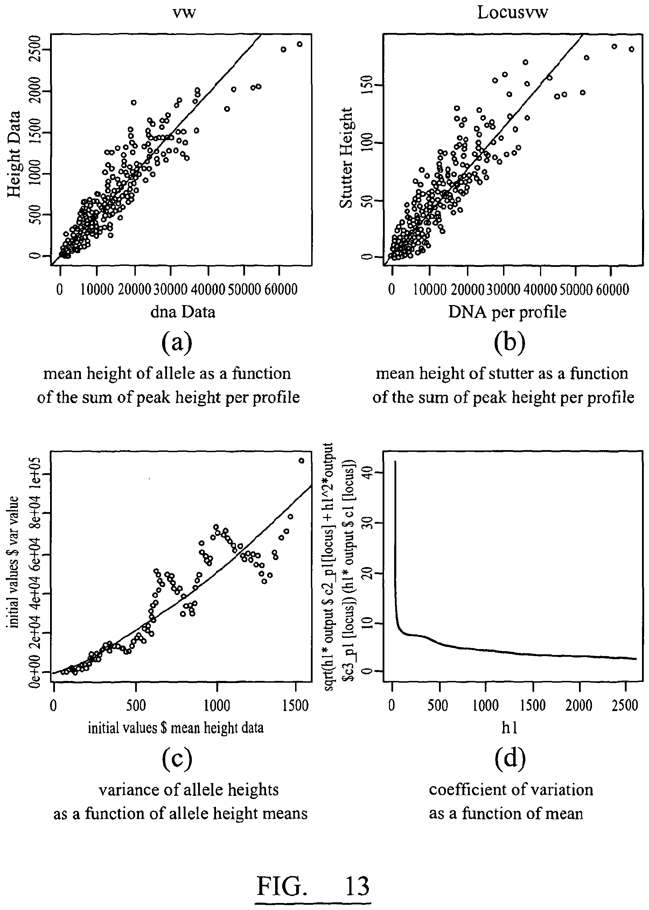

Figure 13 shows the plots of Figure 12 for locus vWA;

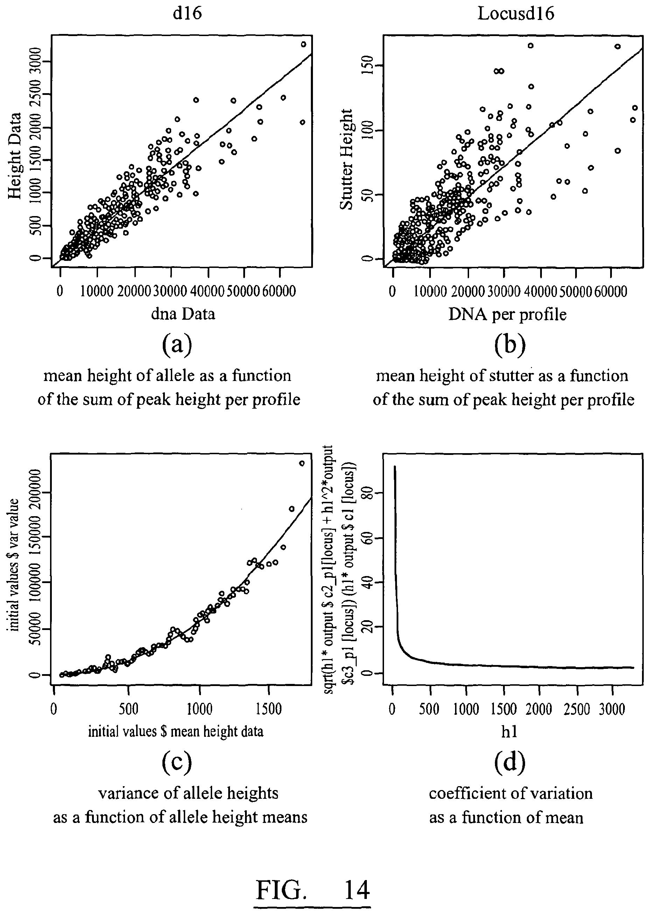

Figure 14 shows the plots of Figure 12 for locus D16;

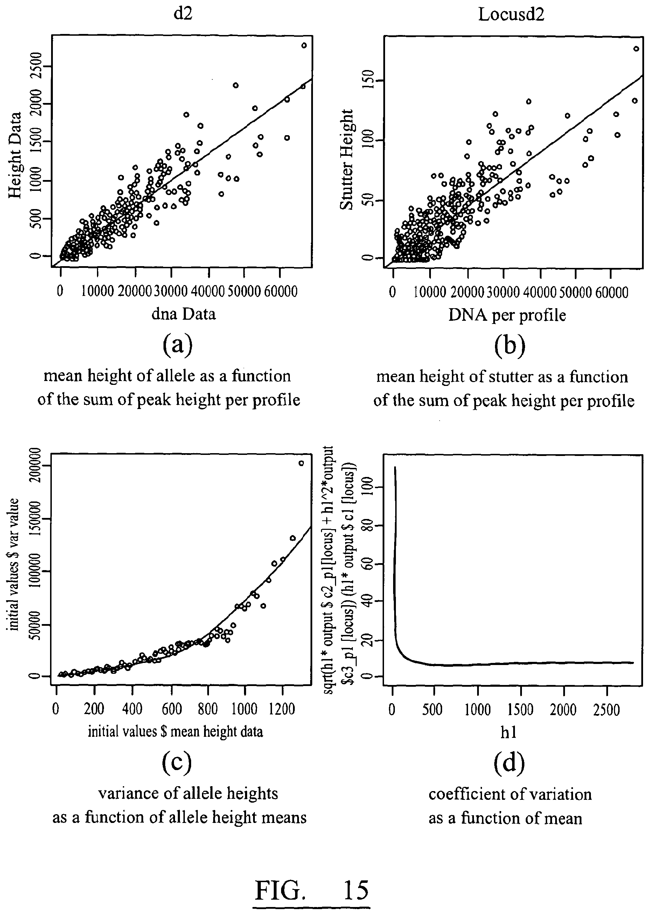

Figure 15 shows the plots of Figure 12 for locus D2;

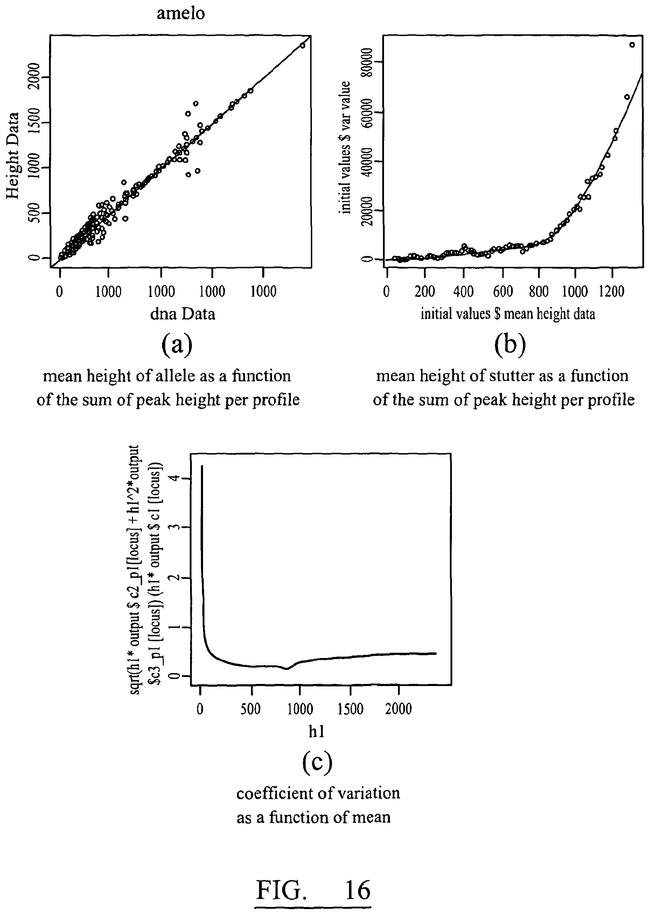

Figure 16 shows the plots of Figure 12 for Amelogenin;

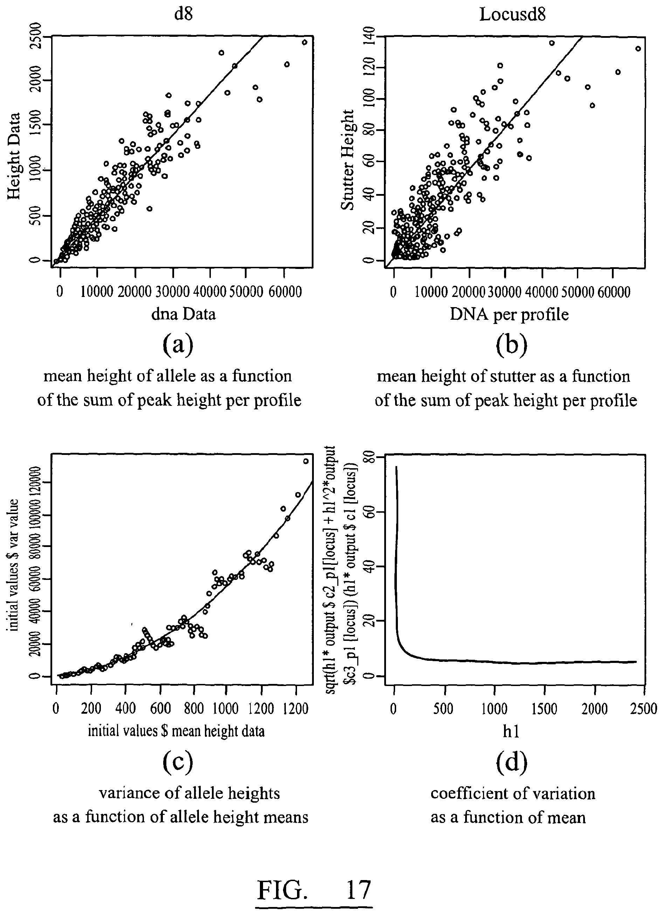

Figure 17 shows the plots of Figure 12 for locus D8;

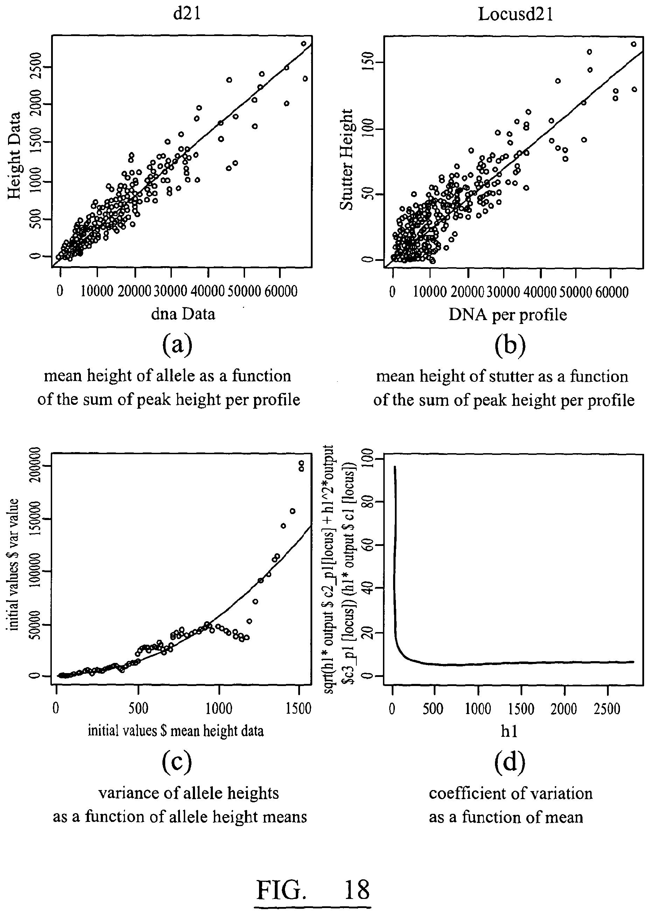

Figure 18 shows the plots of Figure 12 for locus D21;

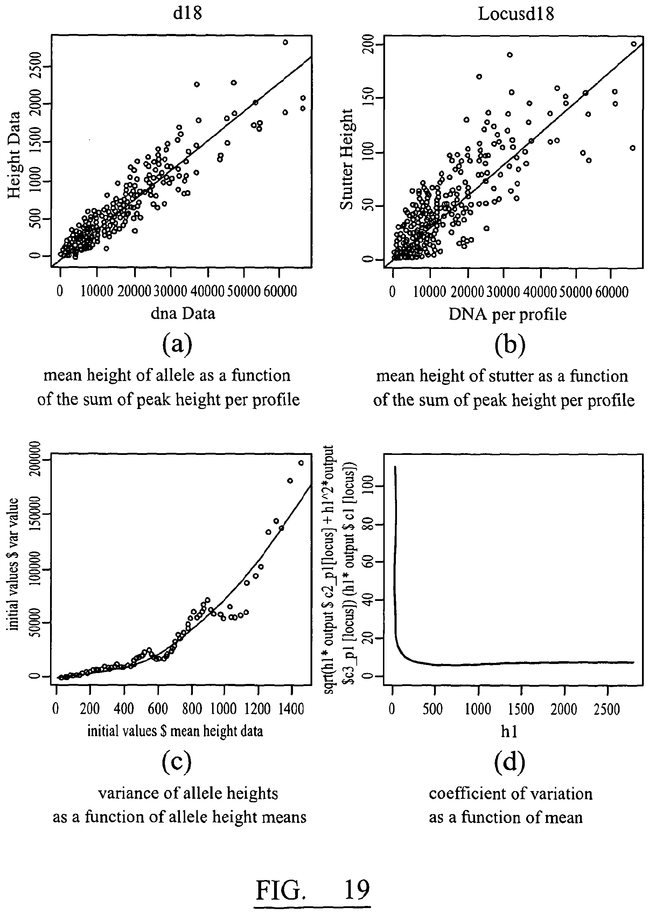

Figure 19 shows the plots of Figure 12 for locus D18;

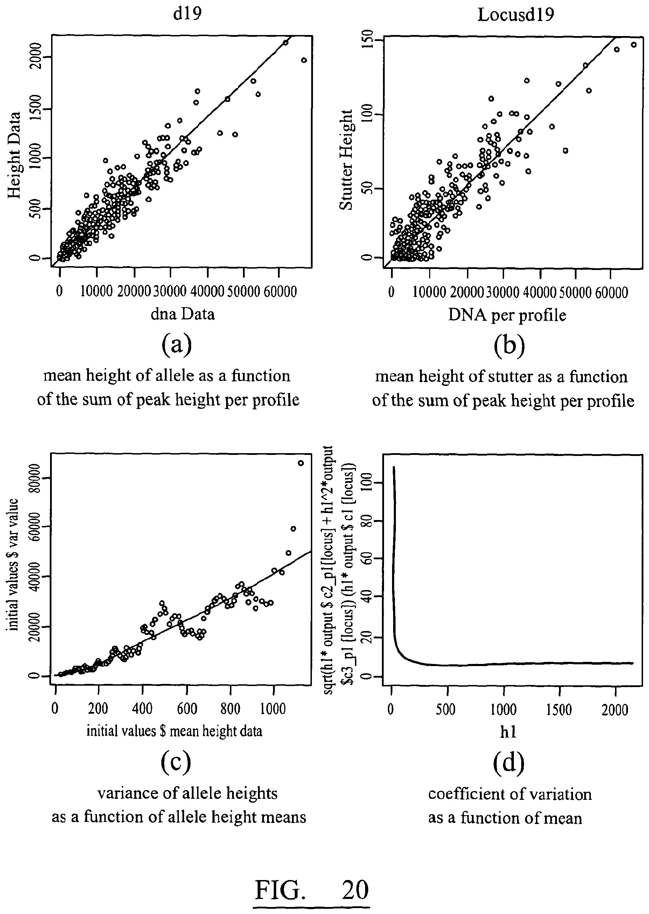

Figure 20 shows the plots of Figure 12 for locus D19;

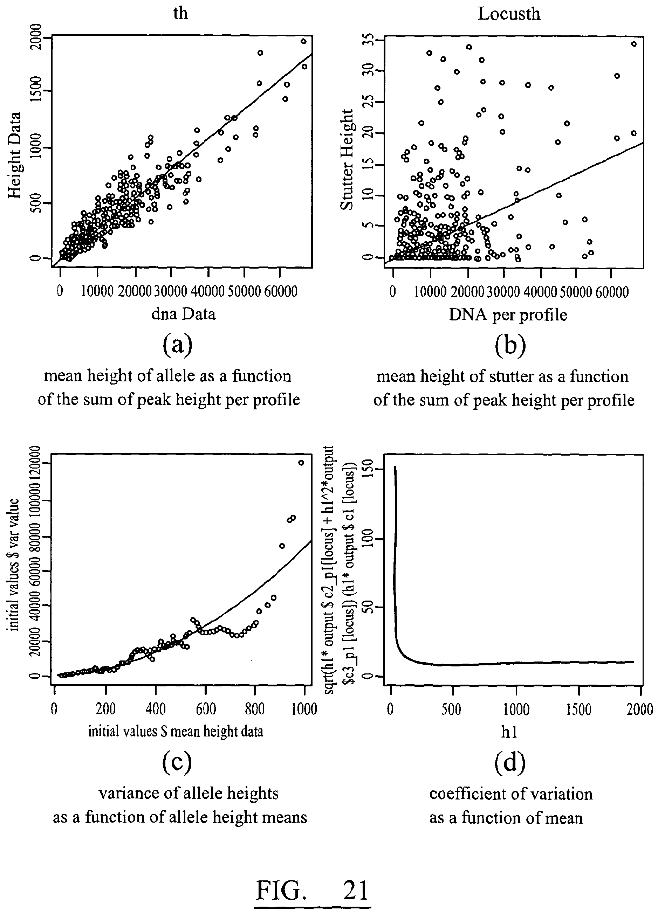

Figure 21 shows the plots of Figure 12 for locus THO;

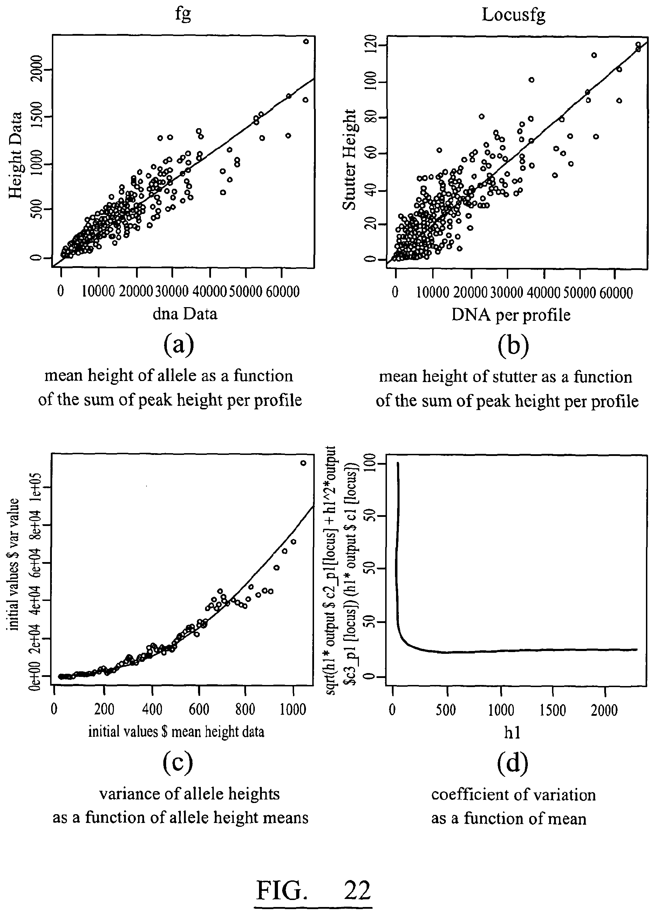

Figure 22 shows the plots of Figure 12 for locus FGA;

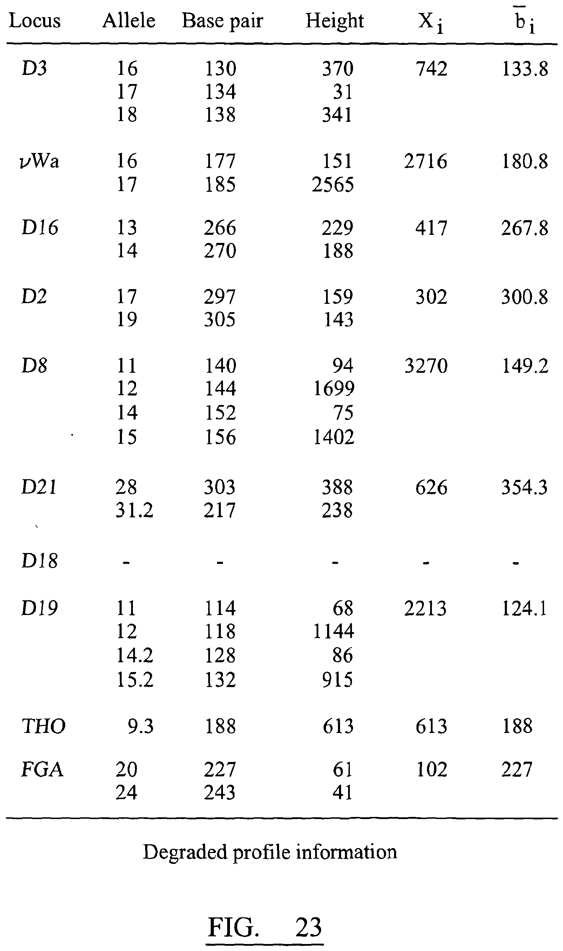

Figure 23 is a table showing degraded profile information;

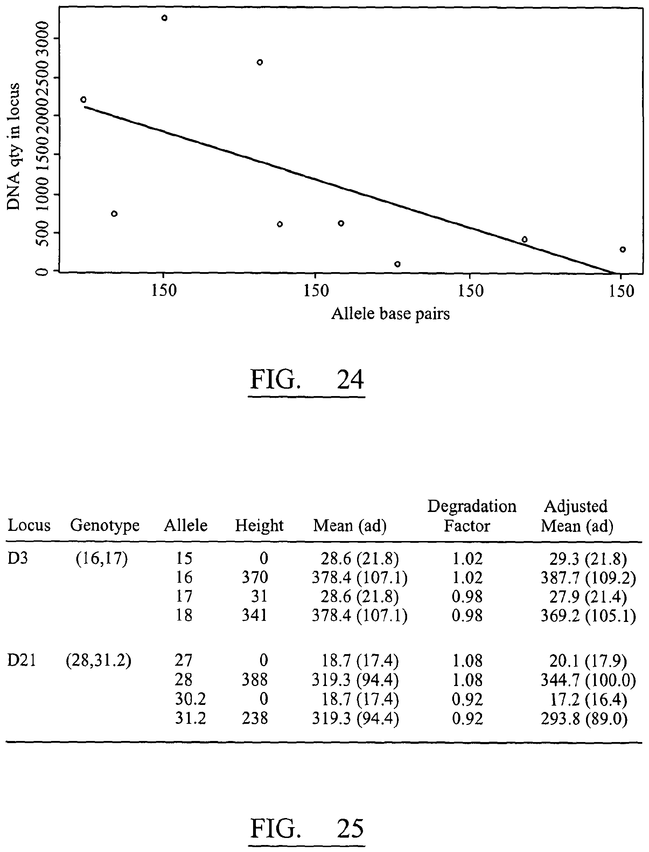

Figure 24 is a plot of DNA quantity in a locus against allele base pairs;

Figure 25 is a table showing two example sof the degradation model as deployed;

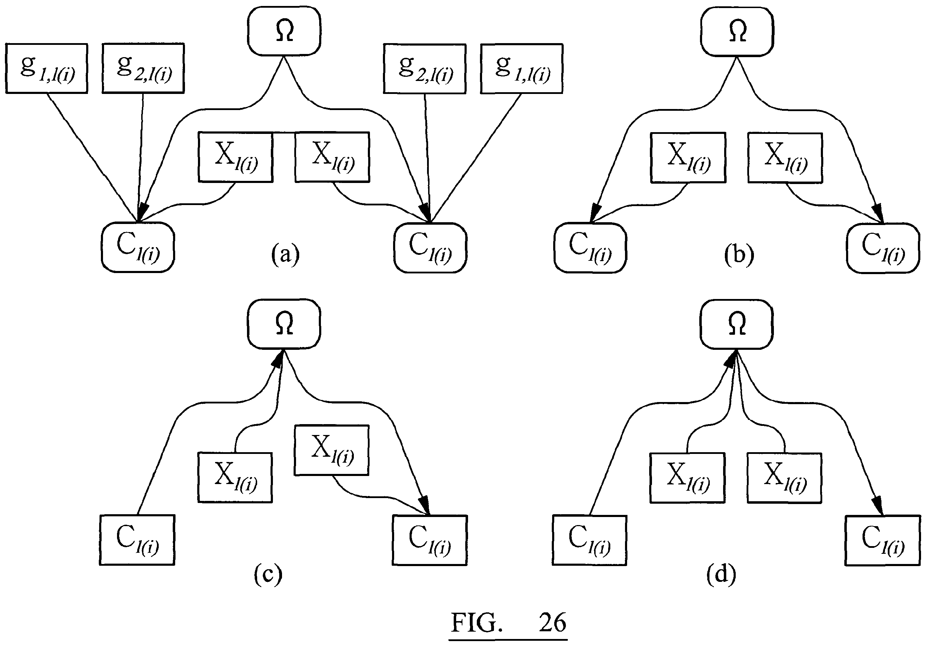

Figure 26a,b,c and d show developing Bayesian Networks

## 1. Background

The present invention is concerned with improving the interpretation of DNA analysis. Basically, such analysis involves taking a sample of DNA, preparing that sample, amplifying that sample and analysing that sample to reveal a set of results. The results are then interpreted with respect to the variations present at a number of loci. The identities of the variations give rise to a profile.

The extent of interpretation required can be extensive and/or can introduce uncertainties. This is particularly so where the DNA sample contains DNA from more than one person, a mixture.

The profile itself has a variety of uses; some immediate and some at a later date following storage.

There is often a need to consider various hypotheses for the identities of the persons responsible for the DNA and evaluate the likelihood of those hypotheses, evidential uses.

There is often a need to consider the analysis genotype against a database of genotypes, so as to establish a list of stored genotypes that are likely matches with the analysis genotype, intelligence uses.

Previously the generally accepted method for assigning evidential weight of single profiles is a binary model. After interpretation, a peak is either in the profile or is excluded from the profile.

When making the interpretation, quantitative information is considered via thresholds which determine decisions and via expert opinion. The thresholds seek to deal with allelic dropout, in particular; the expert opinion seeks to deal with heterozygote imbalance and stutters, in particular. In effect, these approaches acknowledged that peak heights and/or areas and/ contain valuable information for assigning evidential weight, but the use made is very limited and is subjective.

The binary nature of the decision means that once the decision is made, the results only include that binary decision. The underlying information is lost.

Previously, as exemplified in International Patent Application no PCT/GB2008/003882, a specification of a model for computing likelihood ratios (LRs) that uses peak heights taken from such DNA analysis has been provided. This quantified and modelled the relationship between peaks observed in analysis results. The manner in which peaks move in height (or area) relative to one another is considered. This makes use of a far greater part of the underlying information in the results.

## 2. Overview

The aim of this invention is to describe in detail the statistical model for computing likelihood ratios for single profiles while considering peak heights, but also taking into consideration allelic dropout and stutters. The invention then moves on to describe in detail the statistical model for computing likelihood ratios for mixed profiles which considering peak heights and also taking into consideration allelic dropout and stutters.

The present invention provides a specification of a model for computing likelihood ratios (LR's) given information of a different type in the analysis results. The invention is useful in its own right and in a form where it is combined with the previous model which takes into account peak height information.

One such different type of information considered by the present invention is concerned with the effect known as stutter.

Stutter occurs where, during the PCR amplification process, the DNA repeats slip out of register. The stutter sequence is usually one repeat length less in size than the main sequence. When the sequences are separated using electrophoresis to separate them, the stutter sequence gives a band at a different position to the main sequence. The signal arising for the stutter band is generally of lower height than the signal from the main band. However, the presence or absence of stutter and/or the relative height of the stutter peak to the main peak is not constant or fully predictable. This creates issues for the interpretation of such results. The issues for the interpretation of such results become even more problematic where the sample being considered is from mixed sources. This is because the stutter sequence from one person may give a peak which coincides with the position of a peak from the main sequence of another person. However, whether such a peak is in part and/or wholly due to stutter or is nothing to do with stutter is not a readily apparent position.

A second different type of information considered by the present invention is concerned with dropout.

Dropout occurs where a sequence present in the sample is not reflected in the results for the sample after analysis. This can be due to problems specific to the amplification of that sequence, and in particular the limited amount of DNA present after amplification being too low to be detected. This issue becomes increasingly significant the lower the amount of DNA collected in the first place is. This is also an issue in samples which arise from a mixture of sources because not everyone contributes an equal amount of DNA to the sample.

The present invention seeks to make far greater use of a far greater proportion of the information in the results and hence give a more informative and useful overall result.

To achieve this, the present invention includes the use of a number of components. The main components are:

1. An estimated PDF for homozygote peaks conditional on DNA quantity; discussed in detail in LR Numerator Quantification Category 1;

2. An estimated PDF for stutter heights conditional on the height of the parent allele; discussed in detail in LR Numerator Quantification Category 1;

3. An estimated joint probability density function (PDF) of peak height pairs conditional on DNA quantity; discussed in detail in LR Numerator Quantification Category 2. The peak heights are right censored by the limit of detection threshold $T_d$. Below this threshold it is not safe to designate alleles, as the peaks are too close to the baseline to be distinguished from other elements in the signal. Threshold $T_d$ can be different to the limit-of-detection threshold at 50 rfu suggested by the manufacturers of typical instruments analysing such results.

4. A latent variable $X$ representing DNA quantity that models the variability of peak heights across the profile. It does not consider degradation, but degradation can be incorporated by adding another latent variable Δ that discounts DNA quantity according to a numerical representation of the molecular weight of the locus.

5. The calculation of the LR is done separately for the numerator and the denominator. The overall joint PDF for the numerator and the denominator can be represented with Bayesian networks (BNs).

### 3. Detailed Description - Single Profile

### 3.1 - The Calculation of the Likelihood Ratio

The explanation provides:

a definition of the Likelihood Ratio, LR, to be considered;
then considers the numerator, its component parts and the manner in which they are determined;
then considers the denominator, its component parts and the manner in which they are determined;
then combines the position reached in a further discussion of the LR.

The explanation is supplemented by the specifics of the approach in particular cases.

An LR summarises the value of the evidence in providing support to a pair of competing propositions: one of them representing the view of the prosecution ($V_p$) and the other the view of the defence ($V_d$). The usual propositions are:

1) $V_p$: The suspect is the donor of the DNA in the crime stain;
2) $V_d$: Someone else is the donor of the DNA in the crime stain.

The possible values that a crime stain can take are denoted by $C$, the possible values that the suspect's profile can take are denoted by $G_s$. A particular value that $C$ takes is written as $c$, and a particular value that $G_s$ takes is denoted by $g_s$. In general, a variable is denoted by a capital letter, whilst a value that a variable takes is denoted by a lower-case letter.

We are interested in computing:

$$LR = \frac{p\left(c, g_s \middle| V_p\right)}{p\left(c, g_s \middle| V_d\right)} = \frac{f\left(c, g_s \middle| V_p\right)}{f\left(c, g_s \middle| V_d\right)}$$

In effect $f$ is a model of how the peaks change with different situations, including the different situations possible and the chance of each of those.

The crime profile c in a case consists of a set of crime profiles, where each member of the set is the crime profile of a particular locus. Similarly, the suspect genotype $g_s$ is a set where each member is the genotype of the suspect for a particular locus. We use the notation:

$$c = \left\{c_{L(i)} : i = ,2,...,n_{Loci}\right\} \quad \text{and} \quad gs = \left\{gs_{,L(i)} := 1,2,...,n_{Loci}\right\}$$

where $n_{Loci}$ is the number of loci in the profile.

### 3.2 - The LR Numerator Form

The calculation of the numerator is given by:

$$L_p = f\left(c \middle| g_s, V_p\right)$$

Because peak height is dependent between loci and needs to be rendered independent, the likelihood $L_p$ is factorised conditional on DNA quantity $\chi$. This is because the peak height between loci is also dependent on DNA quantity. This gives:

$$L_p = f\left(c_h \mid g_s, h, \chi, V_p\right)$$

It will be recalled, that $c$ is a crime profile across loci consisting of per locus profiles, so for a three locus form $c = \{c_{L(1)}, c_{L(2)}, c_{L(3)}\}$ and similarly for $g_s$. We can therefore write the initial equation as:

$$L_p = f\left(c_{L(1)}, c_{L(2)}, c_{L(3)}, \mid g_{s,L(1)}, g_{s,L(2)}, g_{s,L(3)}, V_p\right)$$

The combination of the two previous equations, to give conditioning on quantity and expansion per locus gives:

$$\mathrm{L}_p = f\left(c_{L(1)} \mid g_{s,L(1)}, \chi_i, V_p\right) x f\left(c_{L(2)} \mid g_{s,L(2)}, \chi_i, V_p\right) x f\left(c_{L(3)} \mid g_{s,L(3)}, \chi_i, V_p\right)$$

which can be stated as:

$$L_p = \sum_{\chi_i} L_{p,L(1)}(\chi_i) \times L_{p,L(2)}(\chi_i) \times L_{p,L(3)}(\chi_i) \times p(\chi_i)$$

where $L_{P,L(j)}(\chi_i)$ is the likelihood for locus $j$ conditional on DNA quantity, this assumes the abstracted form:

$$L_{p,L(j)}(\chi) = f\left(c_{L(j)} \mid g_{s,L(j)}, V_p, \chi_j\right)$$

or:

$$L_{p,L(j)}(\chi) = f\left(c_{L(j)} \mid g_{h(j)}, V, \chi_j\right)$$

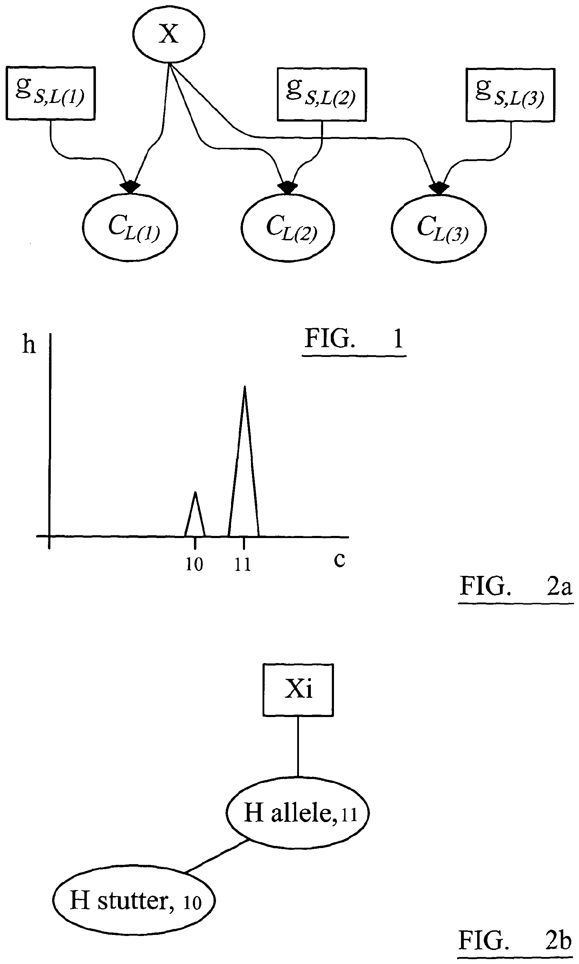

A pictorial description of this calculation is given by the Bayesian Network illustrated in Figure 1. The Bayesian network is for calculating the numerator of the likelihood ratio; hence, the network is conditional on the prosecution view $V_p$. The rectangles represent know quantities. The ovals represent probabilistic quantities. Arrows represent probabilistic dependencies, e.g. the PDF of $C_{L(1)}$ is given for each value of $g_{s,L(1)}$ and $\chi$.

Here we assume that the crime profile $C_{L(i)}$ is conditionally independent of $C_{L(j)}$ given DNA quantity X for $i \neq j$, $i, j \in \{1,2,...,n_L\}$. It can be written as:

$$C_{L(1)} \amalg C_{L_j} \mid X$$

In the Bayesian Network we can see that a path from $C_{L(1)}$ to $C_{L(2)}$ passes through $\chi$.

We also assume that is sufficient to use a discrete probability distribution on DNA quantity as an approximation to a continuous probability distribution. This discrete probability distribution is written as $\{\Pr (\chi=\chi_i): i = 1,2,...,n_x\}$. It can be written simply by $\{p(\chi_i): i=1,2,...,n_\chi\}$.

The likelihood in $L_{p,L(j)}(\chi) = f(c_{L(j)}|g_{s,L(j)}, V_p, X)$ specified a likelihood of the heights in the crime profile given the genotype of a putative donor, and so, they can be written as:

$$L_{L(j)}(\chi) = f\left(c_{L(j)}|g_{L(j)}, V, \chi\right)$$

where V states that the genotype of the donor of crime profile $c_{L(i)}$ is $g_{L(j)}$. The calculation of the likelihood is discussed below after the discussion of the denominator.

In general terms, the numerator can be stated as:

$$\sum_{\chi_i} \left[ \Pi_j^{n\,loci} f\left( c_{h(j)} \middle| g_{s,L(j)}, V_p, \chi_i \right) \right] p(\chi_i)$$

where the consideration is in effect, the genotype ($g_s$) is the donor of ($c_{h(j)}$) given the DNA quantity ($\chi_i$).

The general statements provided above for the numerator enable a suitable numerator to be established for the number of loci under consideration.

### 3.3 - The LR Numerator Quantification

All LR calculations fall into three categories. These apply to the numerator and, as discussed below, the denominator. The genotype of the profile's donor is either:

1) a heterozygote with adjacent alleles; or
2) a heterozygote with non-adjacent alleles; or
3) a homozygote.

A Bayesian Network for each of these three forms is shown in Figure 7; left to right, homozygote; non-adjacent heterozygite; adjacent heterozygote.

#### 3.3.1 - Category 1: homozygous donor

#### 3.3.1.1 - Stutter

Figure 2a illustrates an example of such a situation. The example has a profile, $c_{L(3)} = \{h_{10}, h_{11}\}$ arising from a genotype, $g_{L(3)} = \{11,11\}$. The consideration is of a donor which is homozygous giving a two peak profile, potentially due to stutter.

This position can be stated in the Bayesian Network of Figure 2b. The stutter peak height for allele 10, $H_{stutter,10}$, is dependent upon the allele peak height 11, $H_{allele,11}$, which in turn is dependent upon the DNA quantity, $\chi$.

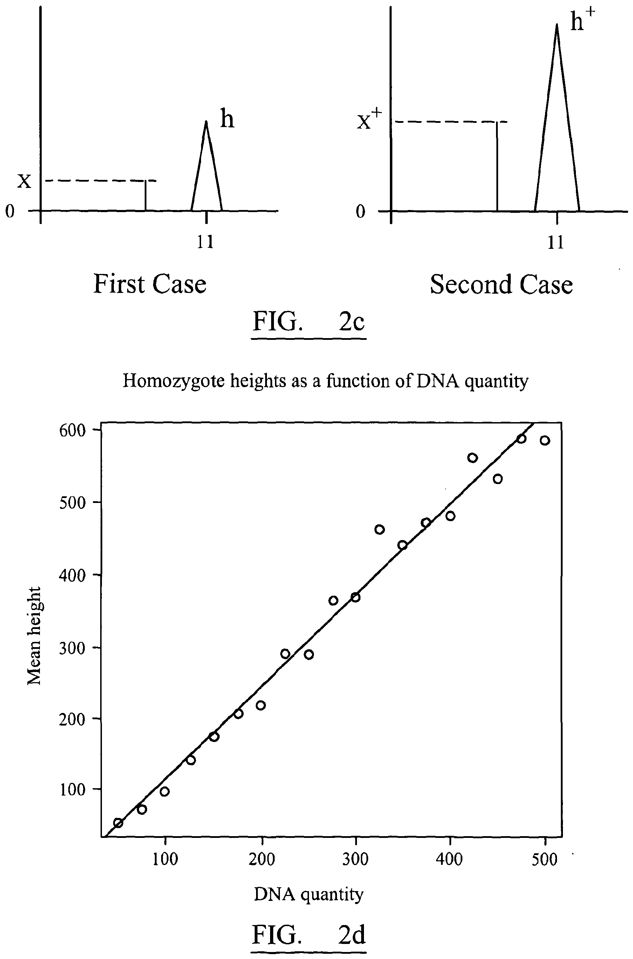

In this context, $\chi$, is assumed to be a known quantity. $H_{stutter,10}$ is a probability distribution function, PDF, which represents the variation in height of the stutter peak with variation in height of the allele peak, $H_{allele,11}$. $H_{allele,11}$ is a probability distribution, PDF, which represent the variation in height of the allele peak with variation in DNA quantity. In effect, there is a PDF for stutter peak height for each value within the PDF for the allele peak height. The concept is illustrated in Figure 2c. In the first case shown in Figure 2c, the allele peak has a height h and the stutter PDF has a range from 0 to x. In the second case shown, the allele peak has a greater height, h+ and the stutter PDF has a range of 0 to x+. Different values within the range have different probabilities of occurrence.

#### 3.3.1.2 - PDF for allele peak height with DNA quantity- details

The PDF for allele peak height, $H_{allele,11}$ in the example, can be obtained from experimental data, for instance by measuring allele peak height for a large number of different, but known DNA quantities.

The model for peak height of homozygote donors is achieved using a Gamma distribution for the PDF, $f(h|\chi)$, for peak heights of homozygote donors given DNA quantity $\chi$.

A Gamma PDF is fully specified through two parameter: the shape parameter $\alpha$ and the rate parameter $\beta$. These parameters are specified through two parameters: the mean height $\bar{h}$, which models the mean value of the homozygote peaks, and parameter $k$ that models the variability of peak heights for the given DNA quantity $\chi$.

The mean value $\bar{h}$ is calculated through a linear relationship between mean heights and DNA quantity, as shown in Figure 2d. The equation of the straight line is given by:

$$\bar{h} = -12.94 + 1.27 \times \chi$$

The line was estimated and plotted using fitHomPDFperX.r. The plot was produced with plot_HoniHgivenXPDFs.r.

The variance is modelled with a factor k which is set to 10. The parameters $\alpha$ and $\beta$ of the Gamma distribution are:

$$\alpha = \bar{h}\Big/ k \quad \text{and} \quad \beta = \alpha\Big/ \bar{h}$$

### 3.3.1.3 - PDF for stutter peak height with allele peak height - details

The PDF for stutter peak height, $H_{stutter,10}$ in the example, can also be obtained from experimental data, for instance by measuring the stutter peak height for a large number of different, but known DNA quantity samples, with the source known to be homozygous. These results can be obtained from the same experiments as provide the allele peak height information mentioned in the previous paragraph.

For each parent height there is a Beta distribution describing the probabilistic behaviour of the stutter height. The generic formula for a Beta PDF is:

$$f\big(y\big|\alpha,\beta\big) = \frac{\Gamma(\alpha+\beta)}{\Gamma(\alpha)\Gamma(\beta)} y^{\alpha-1}\big(1-y\big)^{\beta-1}$$

The conditional PDF $f_{H_s}\big|_H$ is in fact specified through the parameters of the Beta distribution that models stutter proportions, that is, stutter height divided by parent allele height. More specifically

$$f_{H_s}\big|_H\big(h_s\big|h\big) = \frac{1}{h} \times f\pi_s\big|_H\big(\pi_s\big|\alpha(h),\beta(h)\big)$$

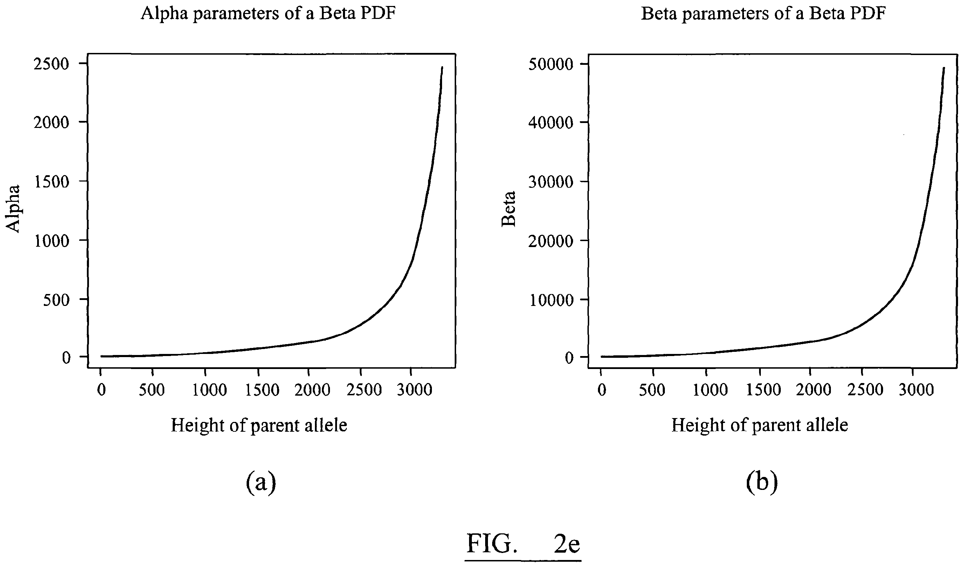

where $\alpha(h)$ and $\beta(h)$ are the parameters of a Beta PDF. Notice that $\alpha(h)$ and $\beta(h)$ are dependent, or functions of the height $h$ of the parent allele. Figure 2e shows a plot of the parameters as a function of $h$. These values will be stored digitally.

### 3.3.1.4 Further details

The methodology can be applied with a PDF for allele height for all loci, but preferably with a separate PDF for allele height for each locus considered. A separate PDF for each allele at each locus is also possible. The methodology can be applies with a PDF for stutter height for all loci, but preferably with a separate PDF for stutter height at each locus considered. A separate PDF for each allele at each locus is also possible.

In an example where locus three is under consideration and the allele peak is 11 and stutter peak is 10, the PDF for this case is given by the formula:

$$f_{L(3)}\big(h_{10},h_{11}\big) = f_s\big(h_{10}\big|h_{11}\big)f_{\text{hom}}\big(h_{11}\big)$$

This formula can be abstracted to give the generic form:

$$f_{L(j)}\big(h_{stutter},h_{allele}\big) = f_s\big(h_{stutter}\big|h_{allele}\big)f_{\text{hom}}\big(h_{allele}\big)$$

with the manner for obtaining the PDF's as described above.

### 3.3.1.5 - Extension to possible dropout

The formula $f_{L(3)}(h_{10},h_{11})$, more generically, $f_{L(j)}(h_{allele1},h_{allele2})$, gives density values for any positive value of the arguments. In many occasions either technical dropout or dropout has occurred and therefore we need to perform some integrations. Three possible cases are considered.

*Possible Case One* - $h_{10} \geq T_d, h_{11} \geq T_d$

If both heights in $c_{L(3)}$ are taller than the limit of detection threshold $T_d$, then the numerator is given by

$$L_{L(3)}(\chi) = f_{L(3)}(h_{10}, h_{11})$$

Or generically as:

$$L_{L(j)}(\chi) = f_{L(j)}(h_{allele1}, h_{allele2})$$

*Possible Case Two - $h_{10} \langle T_d, h_{11} \geq T_d$*

In this case the height of the stutter is less than the limit-of-detection threshold and so, we need to perform one integral.

$$L_{L(3)}(\chi) = \int_0^{T_d} f_{L(3)}(h_{10}, h_{11}) dh_{10}$$

It can be approximated by:

$$L_{L(3)}(\chi) \approx \sum_{h_{10}=1}^{T_d} f_{L(3)}(h_{10}, h_{11})$$

Or more generically as:

$$L_{L(j)}(\chi) \approx \sum_{h_{allele1}=1}^{T_d} f_{L(j)}(h_{allele1}, h_{allele2})$$

*Possible Case Three - $h_{10} \langle T_d, h_{11} \langle T_d$*

In this case, the height of both the peaks is less than the limit of detection threshold.

$$L_{L(3)}(\chi) = \int_0^{T_d} \int_{h_{10}}^{T_d} f_{L(3)}(h_{10}, h_{11}) dh_{10} dh_{11}.$$

It can be approximated by:

$$L_{L(3)}(\chi) \approx \sum_{h_{10}=1}^{T_d} \sum_{h_{11}=h_{10}+1}^{T_d} f_{L(3)}(h_{10}, h_{11})$$

Or more generically as:

$$L_{L(j)}(\chi) \approx \sum_{h_{allele1}=1}^{T_d} \sum_{h_{allele2}=h_{allele1}+1}^{T_d} f_{L(j)}(h_{allele1}, h_{allele2})$$

### 3.3.2 - Category 2: heterozygous donor with non-adjacent alleles

#### 3.3.2.1 - Stutter

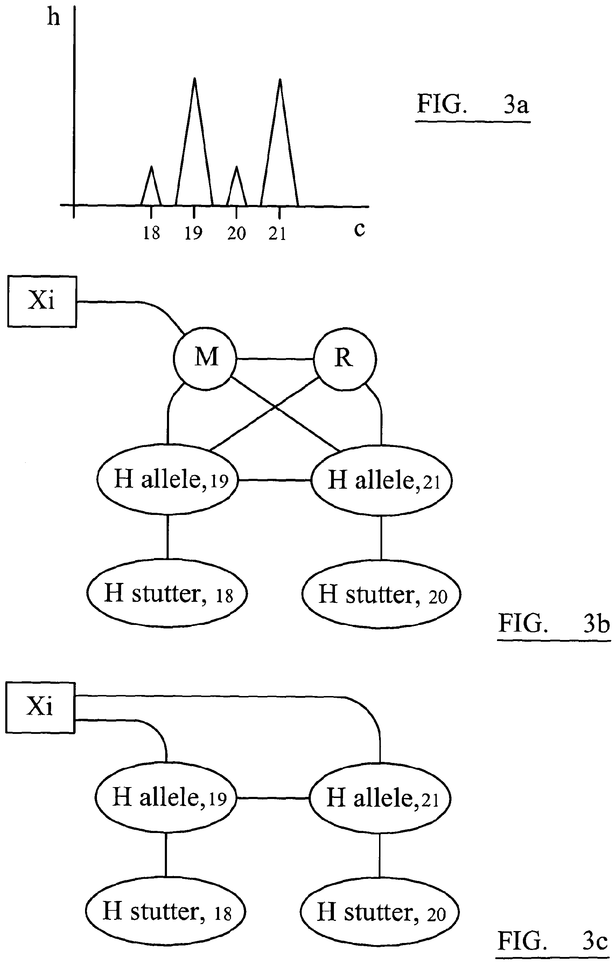

Figure 3a illustrates an example of such a situation. The example has a profile, $c_{L(2)} = \{h_{18}, h_{19}, h_{20}, h_{21}\}$, arising from a genotype, $g_{L(2)} = \{19,21\}$. The consideration is of a donor which is heterozygous, but the peaks are spaced such that a stutter peak cannot contribute to an allele peak. The same approach applies where the allele peaks are separated by two or more allele positions.

This position can be stated as in the Bayesian Network of Figure 3b. The stutter peak height for allele 18,

$H_{stutter,18}$ is dependent upon the allele peak height for allele 19, $H_{allele,19}$, which is in turn dependent upon the DNA quantity, $\chi$. The stutter peak height for allele 20, $H_{stutter,20}$, is dependent upon the allele peak height for allele 21, $H_{allele,21}$, which is in turn dependent upon the DNA quantity, $\chi$.

In this context, x, is assumed to be a known quantity. $H_{stutter,18}$ is a probability distribution function, PDF, which represents the variation in height of the stutter peak with variation in height of the allele peak, $H_{allele,19}$. $H_{allele,19}$ is a probability distribution, PDF, which represent the variation in height of the allele peak with variation in DNA quantity. $H_{stutter,20}$ is a probability distribution function, PDF, which represents the variation in height of the stutter peak with variation in height of the allele peak, $H_{allele,21}$. $H_{allele,21}$ is a probability distribution, PDF, which represent the variation in height of the allele peak with variation in DNA quantity.

These PDF's can be the same PDF's as described above in category 1, particularly where the same locus is involved. As previously mentioned, the PDF's for these different alleles and/or PDF's for these different stutter locations may be different for each allele.

The consistent nature of the PDF's with those described above means that a similar position to that illustrated in Figure 2c occurs. Equally, these PDF's too can be obtained from experimental data.

Figure 8b provides a further illustration of the variation in mean height with DNA quantity (similar to Figure 2d). Whilst Figure 8a provides an illustration of such variance modelling, with the value of profile mean plotted against profile standard deviation.

In addition, the Bayesian Network of Figure 3b indicates that both the allele peak height for allele 19, $H_{allele,19}$, and the allele peak height for allele 21, $H_{allele,21}$, are dependent upon the heterozygous imbalance, R and the mean peak height, M, with those terms also dependent upon each other and upon the DNA quantity, $\chi$.

The heterozygous imbalance is defined as:

$$r = \frac{h_{19}}{h_{21}}$$

or generically as:

$$r = \frac{h_{allele1}}{h_{allele2}}$$

The mean height is defined as:

$$m = \frac{h_{19} + h_{21}}{2}$$

or generically as:

$$m = \frac{h_{allele1} + h_{allele2}}{2}$$

The PDF for $f(h_{19}, h_{21})$ is defined as:

$$f(h_{19}, h_{21}) = |J| . f(r|m) . f(m)$$

with the heterozygous imbalance, r, having a PDF of the lognormal form, for each value of m, so as to give a family of lognormal PDF's overall; and with the mean, m, having a PDF of gamma form, for each value of $\chi$, with a series of discrete values for $\chi$ being considered.

Figure 9 illustrates a PDF for R|M=m using such an approach.

### 3.3.2.2 - Joint PDF for peak pair heights - details

Providing further detail on this, the specification of a joint distribution of pairs of peak heights $h_1$ and $h_2$ is described.

The specification is done by the specification of a joint distribution of mean height $m$ and heterozygote imbalance, which is given by

$$(h_1, h_2) \mapsto \left( m = \frac{h_1 + h_2}{2}, r = \frac{h_1}{h_2} \right)$$

If we specify a joint PDF for mean height $M$ and heterozygote imbalance $R$ we can obtain a joint PDF for peak heights $H_1$ and $H_1$ using the formula:

$$f_{H1,H2}(h_1, h_2 | \chi) = \frac{1}{h_2^2} \left( \frac{h_1 + h_2}{2} \right) \times f_{M,R}(m, r | \chi)$$

In fact we specify the joint distribution of $M$ and $R$ through the marginal distribution of $M, f_M(m|\chi)$, and the conditional distribution of $R$ given $M$, $f_{F|M}(r|m)$. With these considerations the joint PDF for heights is given by the formula:

$$f_{H1,H2}(h_1, h_2 | \chi) = \frac{1}{h_2^2} \left( \frac{h_1 + h_2}{2} \right) \times f_{R,M}(r|m) f_M(m|\chi)$$

Notice that the PDF for $M$ is conditional on DNA quantity $X$. This is a feature in the model that allow for dependence among peak heights in a profile.

In the following description we specify the PDF's for $M$ and $R|M = m$.

### 3.3.2.3 - PDFs for mean height given DNA quantity - details

The PDF $f_M(m|\chi)$ represents a family of PDF's for mean height, one for each value of DNA quantity. This model the behaviour of peak heights in a profile: the more DNA, the higher the peaks, of course, up to some variability.

The Gamma PDF is given by the formula:

$$f(x\alpha, \beta) = \frac{1}{s^\alpha \Gamma(\alpha)} x^{\alpha-1} e^{-x/s}$$

where $s = 1/\beta$. Parameter $\alpha$ is the shape parameter, $\beta$ is the rate parameter and so, $s$ is the scale parameter.

Therefore, the specification of the Gamma PDF's is achieved through the specification of the parameter $\alpha$ and $\beta$ parameters as a function of DNA quantity $\chi$. We achieve this through two intermediary parameters $\overline{m}$ and $k$ that model the mean value and the variance of $M$, respectively. The mean of the Gamma distributions is given by a linear function displayed in Figure 3d. The equation of the line is:

$$\overline{m} = -8.69 + 0.66 \times \chi$$

The variance is controlled by a factor $k$, which is set to 10 although it will change in the future.

Now that we have the parameters $\overline{m}$ and $k$, we can compute the parameters of $\alpha$ and $\beta$ of a Gamma distribution using the formula:

$$\alpha = \overline{m} / k, \quad \beta = \alpha / \overline{m}$$

For illustrative purposes, a selection of the Gamma distributions is shown in Figure 3e.

### 3.3.2.4 - PDFs for heterozygote imbalance given mean height - details

The conditional PDFs of heterozygote imbalance are modelled with lognormal PDFs whose PDF is given by

$$f_R\left(r|\mu,\sigma\right) = \frac{1}{r \times \sigma(m)\sqrt{2\pi}}\exp^{\frac{-(\ln(r)-\mu)^2}{2\sigma(m)}}$$

A Lognormal PDF is fully specified through parameters $\mu$ and $\sigma(m)$. The latter parameter is dependent on the mean height $m$ by the plot in Figure 3f. The transfer of the actual values can be done digitally. Currently the parameters are stored in logNPars.rData.

### 3.3.2.5 - Further details

As a result, PDF's have been determined for the six dependents in Figure 3b.

Given the above, the Bayesian Network of Figure 3b can be simplified to the form of Figure 3c.

In an example where locus 2 is under consideration and the allele peaks are at 19 and 21 and the stutter peaks are at 18 and 20, the generic PDF for this calculation is given by the formula:

$$f_{L(2)}\left(h_{18},h_{19},h_{20},h_{21}\right) = f_s\left(h_{18}|h_{19}\right)f_s\left(h_{20}|h_{21}\right)f_{het}\left(h_{19}|h_{21}\right)$$

This formula can be abstracted to give the generic form:

$$f_{L(j)}\left(h_{stutter1},h_{allele1},h_{stutter2},h_{allele2}\right) = f_{stutter}\left(h_{stutter1}|h_{allele1}\right)f_{stutter}\left(h_{stutter2}|h_{allele2}\right)f_{het}\left(h_{allele1}|h_{allele2}\right)$$

The manner for obtaining the PDF's is as described above with respect to the simplified form too.

### 3.3.2.6- Extension to possible dropout

The formula $f_{L(2)}(h_{18},h_{19},h_{20},h_{21})$, more generically $f_{L(j)}(h_{stutter1},h_{allele1},h_{stutter2},h_{allele2})$, gives density values for any positive value of the arguments. In many occasions either technical dropout, where a peak is smaller than the limit-of-detection threshold $T_d$, or dropout, where a peak is in the baseline, have occurred and therefore we need to perform some integrations. Eight possible cases are considered.

**Possible Case One** - $h_{18} \geq T_d$, $h_{19} \geq T_d$, $h_{20} \geq T_d$, $h_{21} \geq T_d$

In this case we do not need to compute any integration and

$$L_{L(2)}\left(\chi\right) = f_{L(2)}\left(h_{18},h_{19},h_{20}h_{21}\right)$$

Or more generically:

$$L_{L(j)}\left(\chi\right) = f_{L(j)}\left(h_{stutter1},h_{allele1},h_{stutter2}h_{allele2}\right)$$

**Possible Case Two** - $h_{18} \geq T_d$, $h_{19} \geq T_d$, $h_{20}\langle T_d$, $h_{21}\langle T_d$

In this case we need to compute two integrations:

$$L_{L(2)}\left(\chi\right) = \int_0^{T_d}\int_{h_{20}}^{T_d} f_{L(2)}\left(h_{18},h_{19},h_{20}h_{21}\right)dh_{20}dh_{21}.$$

It can be approximated with the following summations:

$$L_{L(2)}\left(\chi\right) \approx \sum_{h_{20}=1}^{T_d}\sum_{h_{21}=h_{20}+1}^{T_d} f_{L(2)}\left(h_{18},h_{19},h_{20},h_{21}\right)$$

Or more generically:

$$L_{L(j)}(\chi) \approx \sum_{h_{stutter2}=1}^{T_d} \sum_{h_{allele2}=h_{stutter2}+1}^{T_d} f_{L(j)}\left(h_{stutter1}, h_{allele1}, h_{stutter2}, h_{allele2}\right)$$

*Possible Case Three - $h_{18}\langle T_d, h_{19} \geq T_d, h_{20} \geq T_d, h_{21} \geq T_d$*

In this case we need only one integration:

$$L_{L(2)}(\chi) = \int_0^{T_d} f_{L(2)}\left(h_{18}, h_{19}, h_{20}h_{21}\right)dh_{18}$$

It can be approximated as summation:

$$L_{L(2)}(\chi) \approx \sum_{h_{18}=1}^{T_d} f_{L(2)}\left(h_{18}, h_{19}, h_{20}, h_{21}\right)$$

Or more generically as:

$$L_{L(j)}(\chi) \approx \sum_{h_{stutter1}=1}^{T_d} f_{L(j)}\left(h_{stutter1}, h_{allele1}, h_{stutter2}, h_{allele2}\right)$$

*Possible Case Four - $h_{18}\langle T_d, h_{19} \geq T_d, h_{20}\langle T_d, h_{21} \geq T_d$*

Two integrations are required. The likelihood is given by:

$$L_{L(2)}(\chi) = \int_0^{T_d} \int_0^{T_d} f_{L(2)}\left(h_{18}, h_{19}, h_{20}h_{21}\right)dh_{18}dh_{20}.$$

It can be approximated by:

$$L_{L(2)}(\chi) \approx \sum_{h_{18}=1}^{T_d} \sum_{h_{20}=1}^{T_d} f_{L(2)}\left(h_{18}, h_{19}, h_{20}, h_{21}\right)$$

Or more generically as:

$$L_{L(j)}(\chi) \approx \sum_{h_{stutter1}=1}^{T_d} \sum_{h_{stutter2}=1}^{T_d} f_{L(j)}\left(h_{stutter1}, h_{allele1}, h_{stutter2}, h_{allele2}\right)$$

*Possible Case Five - $h_{18}\langle T_d, h_{19} \geq T_d, h_{20}\langle T_d, h_{21}\langle T_d$*

We need three integrations.

$$L_{L(2)}(\chi) = \int_0^{T_d} \int_0^{T_d} \int_{h_{20}}^{T_d} f_{L(2)}\left(h_{18}, h_{19}, h_{20}, h_{21}\right)dh_{18}dh_{19}dh_{20}dh_{21}.$$

The likelihood is approximated with the summations:

$$L_{L(2)}(\chi) \approx \sum_{h_{18}=1}^{T_d} \sum_{h_{20}=1}^{T_d} \sum_{h_{20}+1}^{T_d} f_{L(2)}(h_{18}, h_{19}, h_{20}, h_{21})$$

Or more generically:

$$L_{L(j)}(\chi) \approx \sum_{h_{stutter1}=1}^{T_d} \sum_{h_{stutter2}=1}^{T_d} \sum_{h_{stutter2}+1}^{T_d} f_{L(j)}(h_{stutter1}, h_{allele1}, h_{stutter2}, h_{allele2})$$

**Possible Case Six** - $h_{18}\langle T_d, h_{19}\langle T_d, h_{20} \geq T_d, h_{21} \geq T_d$

Two integrations are required.

$$L_{L(2)}(\chi) = \int_0^{T_d} \int_{h_{18}}^{T_d} f_{L(2)}(h_{18}, h_{19}, h_{20} h_{21}) dh_{18} dh_{19}.$$

The likelihood is approximated with the summations:

$$L_{L(2)}(\chi) \approx \sum_{h_{18}=1}^{T_d} \sum_{h_{19}=h_{18}+1}^{T_d} f_{L(2)}(h_{18}, h_{19}, h_{20}, h_{21})$$

Or more generically:

$$L_{L(j)}(\chi) \approx \sum_{h_{stutter1}=1}^{T_d} \sum_{h_{allele1}=h_{stutter1}+1}^{T_d} f_{L(j)}(h_{stutter1}, h_{allele1}, h_{stutter2}, h_{allele2})$$

**Possible Case Seven** - $k_{18}\langle T_d, h_{19}\langle T_d, h_{20}\langle T_d, h_{21} \geq T_d$

We need three integrations.

$$L_{L(2)}(\chi) = \int_0^{T_d} \int_{h_{18}}^{T_d} \int_0^{T_d} f_{L(2)}(h_{18}, h_{19}, h_{20}, h_{21}) dh_{18} dh_{19} dh_{20}.$$

The likelihood is approximated with the summations:

$$L_{L(2)}(\chi) \approx \sum_{h_{18}=0}^{T_d} \sum_{h_{19}=h_{18}+1}^{T_d} \sum_{h_{20}=0}^{T_d} f_{L(2)}(h_{18}, h_{19}, h_{20}, h_{21})$$

Or more generically:

$$L_{L(j)}(\chi) \approx \sum_{h_{stutter1}=0}^{T_d} \sum_{h_{allele1}=h_{stutter1}+1}^{T_d} \sum_{h_{stutter2}=0}^{T_d} f_{L(j)}(h_{stutter1}, h_{allele1}, h_{stutter2}, h_{allele2})$$

**Possible Case Eight** - $h_{18}\langle T_d, h_{19}\langle T_d, h_{20}\langle T_d, h_{21}\langle T_d$

We need four integrations.

$$L_{L(2)}(\chi) = \int_0^{T_d} \int_{h_{18}}^{T_d} \int_0^{T_d} \int_{h_{20}}^{T_d} f_{L(2)}(h_{18}, h_{19}, h_{20}, h_{21}) dh_{18} dh_{19} dh_{20} dh_{21}$$

The likelihood can be approximated with the summations:

$$L_{L(2)}(\chi) \approx \sum_{h_{18}=1}^{T_d} \sum_{h_{19}=h_{18}+1}^{T_d} \sum_{h_{20}=1}^{T_d} \sum_{h_{21}=h_{20}+1}^{T_d} f_{L(2)}(h_{18}, h_{19}, h_{20}, h_{21})$$

Or more generically:

$$L_{L(j)}(\chi) \approx \sum_{h_{stutter1}=1}^{T_d} \sum_{h_{allele1}=h_{stutter1}+1}^{T_d} \sum_{h_{stutter2}=1}^{T_d} \sum_{h_{allele2}=h_{stutter2}+1}^{T_d} f_{L(j)}(h_{stutter1}, h_{allele1}, h_{stutter2}, h_{allele2})$$

### 3.3.3 - Category 3: heterozygous donor with adjacent alleles

### 3.3.3.1 - Stutter

Figure 4a illustrates an example of such a situation. The example has a profile, $c_{L(1)} = \{h_{15}, h_{16}, h_{17}\}$ arising from a genotype $g_{L(1)} = \{16,17\}$ where each height $h_i$ can be smaller than the limit-of-detection threshold $T_d$, situation $h_i \langle T_d$, or can be greater than this threshold, $h_i \geq T_d$ for $i \in \{15,16,17\}$. The consideration is of a donor which is heterozygous, but with overlap in position between allele peak and stutter peak.

The position can be stated in the Bayesian Network of Figure 4b. The stutter peak height for allele 15, $H_{stutter,15}$, is dependent upon the allele peak height for allele 16, $H_{allele,16}$, which is in turn dependent upon the DNA quantity, $\chi$. The stutter peak height for allele 16, $H_{stutter,16}$, is dependent upon the allele peak height for allele 17, $H_{allele,17}$, which is in turn dependent upon the DNA quantity, $\chi$. Additionally, the Bayesian Network needs to include the combined allele and stutter peak at allele 16, $H_{allele + stutter\,16}$, which is dependent upon the allele peak height for allele 16, $H_{allele,16}$, and is dependent upon the stutter peak height for allele 16, $H_{stutter,16}$.

In terms of the actual observed results, $H_{stutter,15}$, $H_{allele,17}$, and $H_{allele + stutter\,16}$, are observed and can be seen in Figure 4a, but $H_{allele,16}$, and $H_{stutter,16}$ are components within $H_{allele + stutter\,16}$ and so are not observed.

In addition, the Bayesian Network of Figure 4b indicates that both the allele peak height for allele 16, $H_{allele,16}$, and the allele peak height for allele 17, $H_{allele,17}$, are dependent upon the heterozygous imbalance, R and the mean peak height, M, with those terms also dependent upon each other and upon the DNA quantity, $\chi$.

In this context, $\chi$, is assumed to be a known quantity.

The overlap between stutter and allele contribution within a peak means that a different approach to obtaining the PDF's needs to be taken.

### 3.3.3.2 - PDF for allele + stutter peak height with allele peak height and stutter peak height - details

The PDF for $f(h_{allele1+stutter1}|h_{allele1}, h_{stutter1})=1$ if $h_{allele1=stutter1} = h_{allele1} + h_{stutter1}$ and has value = 0 otherwise. This is more clearly seen in the two specific examples:

$$f(h_{=200\,for\,allele1+stutter1}\big|h_{=150\,for\,allele1}, h_{=50\,for\,stutter1}) = 1$$

$$f(h_{=210\,for\,allele1+stutter1}\big|h_{=150\,for\,allele1}, h_{=50\,for\,stutter1}) = 0$$

This form is used to provide a PDF for $H_{allele + stutter\,16}$ in the above example.

### 3.3.3.3 - PDF's for other observed peaks

The PDF's for the other two observed dependents are obtained by integrating out $H_{allele,16}$, and $H_{stutter,16}$ in the above example; more generically, $H_{allele1}$, and $H_{stutter1}$. Integrating out avoids the need to consider a three dimensional estimation of the PDF's from experimental data.

The integrating out allows PDF's for the resulting components to be sought, for instance by looking at all the possibilities. This provides:

$$f(h_{allele16}, h_{allele17}|\chi) \times f(h_{stutter15}|h_{allele16}) \times f(h_{stutter16}|h_{allele17}) \times f(h_{allele+stutter16}|h_{allele16}, h_{stutter16})$$

Which equates to:

$$f\left(h_{allele16}, h_{allele17} \middle| \chi\right) \text{ x } f\left(h_{stutter15}, h_{allele16}, h_{stutter16}, h_{allele+stutter16}, h_{allele17}\right)$$

This comes together as the simplified Bayesian Network of Figure 4c. In an example where locus 1 is under consideration and the allele peaks are at 16 and 17 and the stutter peaks are at 15 and 16, we wish to calculate:

$$L_{L(1)}(\chi) = f\left(c_{L(1)} \middle| g_{L(1)}, V, \chi\right)$$

So, without considering $T_d$, the generic PDF is defined as:

$$f_{L(1)}(h_{15}, h_{16}, h_{17}) = \int_R f_s\left(h_{15} \middle| h_{a,16}\right) f_s\left(h_{s,16} \middle| h_{17}\right) f_{het}\left(h_{a,16}, h_{17}\right) dh_{a,16} dh_{s,16}$$

where $R = \{h_{a16}, h_{s,16} : h_{a,16} + h_{s,16} = h_{16}\}$; $f_s$ is a PDF for stutter heights conditional on parent height; and $f_{het}$ is a PDF of pairs of heights of heterozygous genotypes. The PDFs in these sections are given for any value $h_i$, including $h_i$ less than the threshold $T_d$.

The integral in the equation above can be computed by numerical integration or Monte Carlo integration. The preferred method for numerical integration is adaptive quadratures. The simplest method is integration by histogram approximation, which, for completeness, is given below.

The integral in the previous equation can be approximated with the summation:

$$f_{L(1)}(h_{15}, h_{16}, h_{17}) \approx \sum_{h_{a,16}=h_{15}+1}^{h_{16}} f_s\left(h_{15} \middle| h_{a,16}\right) f_s\left(h_{s,16} \middle| h_{17}\right) f_{het}\left(h_{a,16}, h_{17}\right)$$

where $h_{s,16} = h_{16} - h_{a,16}$. The step in the summation is one. It can be modified to have a larger increment, say $x_{inc}$, but then the term in the summation needs to be multiplied by $x_{inc}$. This is one possible numerical approximation. Faster numerical integrations can be achieved using adaptive methods in which the size of the bin is dynamically selected.

### 3.3.3.4 - Extending to dropout

The term $f_{L(1)}(h_{15}, h_{16}, h_{17})$ provides density values for each value of the arguments. However, in many occasions technical dropout has occurred, that is, a peak is smaller than the limit-of-detection threshold $T_d$. In this case we need to calculate further integral to obtain the required likelihoods. In the following sections we describe the extra calculations that need to be done for each of the six possible cases.

All integrals described in the sections below can be computed by numerical integration of Monte Carlo integration. The method described in these sections in the simplest way to compute a numerical integration through a hitogram approximation. They are included for the sale of completeness. An integration method based on adaptive quadratures is more efficient in terms of computational cost.

**Possible Case One** - $h_{15} \geq T_d$, $h_{16} \geq T_d$, $h_{17} \geq T_d$

If all the heights in $c_{L(1)}$ are taller than $T_d$ then the numerator of the LR for this locus is given by:

$$L_{L(1)}(\chi) = f_{L(1)}(h_{15}, h_{16}, h_{17}).$$

Or more generically:

$$L_{L(j)}(\chi) = f_{L(j)}\left(h_{stutter1}, h_{allele1+stutter2}, h_{allele2}\right)$$

**Possible Case Two** - $h_{15}\langle T_d, h_{16} \geq T_d, h_{17} \geq T_d$

If one of the heights are below $T_d$ we need to perform further integrations. For example if $h_{15}\langle T_d$ the numerator of the LR is given by the equation:

$$L_{L(1)}(\chi) = \int_{h_{15}\langle h_{16}} f_{L(1)}(h_{15}, h_{16}, h_{17}) dh_{15}.$$

A numerical approximation can be use to obtain the integral:

$$L_{L(1)}(\chi) = \sum_{h_{15}=1}^{T_d} f_{L(1)}(h_{15}, h_{16}, h_{17}).$$

Or more generically:

$$L_{L(j)}(\chi) = \sum_{h_{stutter1}=1}^{T_d} f_{L(j)}(h_{stutter1}, h_{allele1+stutter2}, h_{allele2})$$

**Possible Case Three** - $h_{15}\langle T_d, h_{16}\langle T_d, h_{17} \geq T_d$

In this case we need to compute two integrals:

$$L_{L(1)}(\chi) = \int_{h_{15}\langle T_d} \int_{h_{16}\langle T_d} f_{L(1)}(h_{15}, h_{16}, h_{17}) dh_{15}.$$

It can be approximated with:

$$L_{L(1)}(\chi) \approx \sum_{h_{15}=1}^{T_d} \sum_{h_{16}=h_{15}+1}^{T_d} f_{L(1)}(h_{15}, h_{16}, h_{17}) dh_{15} dh_{16}.$$

Or more generically by:

$$L_{L(j)}(\chi) \approx \sum_{h_{stutter1}=1}^{T_d} \sum_{h_{allele1+stutter2}=h_{stutter1}+1}^{T_d} f_{L(j)}(h_{stutter1}, h_{allele1+stutter2}, h_{stutter2}) dh_{stutter1} dh_{allele1+stutter2}$$

**Possible Case Four** - $h_{15}\langle T_d, h_{16} \geq T_d, h_{17}\langle T_d$

In this case we need to calculate two integrals:

$$L_{L(1)}(\chi) = \int_{h_{15}\langle T_d} \int_{h_{17}\langle T_d} f_{L(1)}(h_{15}, h_{16}, h_{17}) dh_{15} dh_{17}.$$

It can be approximated by

$$L_{L(1)}(\chi) \approx \sum_{h_{15}=1}^{T_d} \sum_{h_{17}=1}^{T_d} f_{L(1)}(h_{15}, h_{16}, h_{17})$$

Or more generically by:

$$L_{L(i)}(\chi) \approx \sum_{h_{stutter1}=1}^{T_d} \sum_{h_{allele2}=1}^{T_d} f_{L(j)}(h_{stutter1}, h_{allele1+stutter2}, h_{allele2})$$

**Possible Case Five -** $h_{15} \geq T_d, h_{16} \geq T_d, h_{17} \langle T_d$

In this case we need to calculate only one integral:

$$L_{L(1)}(\chi) = \int_{h_{17}\langle T_d} f_{L(1)}(h_{15}, h_{16}, h_{17}) dh_{17}.$$

The integral can be approximated using the summation:

$$L_{L(1)}(\chi) \approx \sum_{h_{17}=1}^{T_d} f_{L(1)}(h_{15}, h_{16}, h_{17})$$

Or more generically by:

$$L_{L(j)}(\chi) \approx \sum_{h_{allele2}=1}^{T_d} f_{L(j)}(h_{stutter1}, h_{allele1+stutter2}, h_{stutter2})$$

**Possible Case Six -** $h_{15} \langle T_d, h_{16} \langle T_d, h_{17} \langle T_d$

In this case we need to compute three integrals:

$$L_{L(1)}(\chi) = \int_{h\_15\langle T_d} \int_{h\_16\langle T_d} \int_{h\_17\langle T_d} f_{L(1)}(h_{15}, h_{16}, h_{17}) dh_{15} dh_{16} dh_{17}.$$

The integrals can be approximate with the summations,

$$L_{L(1)}(\chi) \approx \sum_{h_{15}=1}^{T_d} \sum_{h_{16}=h_{15}+1}^{T_d} \sum_{h_{17}}^{T_d} f_{L(1)}(h_{15}, h_{16}, h_{17})$$

Or more generically:

$$L_{L(j)}(\chi) \approx \sum_{h_{stutter1}=1}^{T_d} \sum_{h_{allele1+stutter2}=h_{stutter1}+1}^{T_d} \sum_{h_{allele2}}^{T_d} f_{L(j)}(h_{stutter1}, h_{allele+stutter2}, h_{allele2})$$

### 3.3.4 - LR Nominator Summary

The approach for the three different categories is summarised in the Bayesian Network of Figure 5. This presents the acyclic directed graph of a Bayesian Network in the case of three loci with the form:

- Locus L(1) :

  ○ $c_{L(1)} = \{h_{15}, h_{16}, h_{17}\}$ and
  ○ $g_{s,L(1)} = \{16, 17\}$

- Locus $L_{(2)}$:

  ○ $c_{L(2)} = \{h_{18}, h_{19}, h_{20}, h_{21}\}$ and
  ○ $g_{s,L(2)} = \{19, 20\}$

- Locus L(3) :

  ○ $c_{L(3)} = \{h_{10}, h_{11}\}$ and
  ○ $g_{s,L(3)} = \{11, 11\}$

The specification of the calculation of likelihood for this Bayesian Network is sufficient for calculating likelihoods for all loci of any number of loci.

### 3.4 The LR Denominator Form

The calculation of the denominator follows the same derivation approach. Hence, the calculation of the denominator is given by:

$$L_d = f(c|g_s, V_d)$$

As above, because the crime profile c extends across loci, for the three locus example, the initial equation of this section can be rewritten as:

$$L_d = f\left(c_{L(1)}, c_{L(2)}, c_{L(3)} \middle| g_{s,L(1)}, g_{s,L(2)}, g_{s,L(3)}, V_d\right)$$

Likelihood $L_d$ can be factorised according to DNA quantity and combined with the previous equation's expansion, to give:

$$L_d = \sum_{\chi_i} f\left(c_{L(1)} \middle| g_{L(1)}, V_d, \chi_i\right) f\left(c_{L(2)} \middle| g_{L(2)}, V_d \chi_i\right) f\left(c_{L(3)} \middle| g_{L(3)}, V_d \chi_i\right)$$

This can be abstracted to give:

$$f\left(c_{L(j)} \middle| g_{L(j)}, V_d, \chi_i\right)$$

As the expression $f(c_{L(j)}|g_{L(j)}, V_d, \chi_i)$ does not specify the donor of the crime stain, it needs to be expanded as:

$$f\left(c_{L(j)} \middle| g_{L(j)}, V_d, \chi_i\right) = \sum_{g_{U,L(j)}} f\left(c_{L(j)} g_{U,L(j)}, V_d, \chi \middle|\right) \times p\left(g_{U,L(j)} \middle| g_{S,L(j)}, V_d\right)$$

The first term on the right hand side of this definition corresponds to a term of matching form found in the numerator, as discussed above and expressed as:

$$L_{L(j)}(\chi) = f\left(c_{L(j)} g_{L(j)}, V, \chi\right)$$

The second term in the right-hand side is a conditional genotype probability. This can be computed using existing formula for conditional genotype probabilities given putative related and unrelated contributors with population structure or not, for instance see J.D. Balding and R. Nichols. DNA profile match probability calculation: How to allow for population stratification, relatedness, database selection and single bands. Forensic Science International, 64:125-140, 1994.
We denote the first term with the expression:

$$L_{d,L(j)}(\chi) = f\left(c_{L(j)} \middle| g_{U,L(j)}, V_d, \chi\right)$$

with the likelihood in this specified as a likelihood of the heights in the crime profile given the genotype of a putative donor, and so, they can be written as:

$$L_{L(j)}(\chi) = f\left(c_{L(j)} \big| g_{L(j)}, V, \chi\right)$$

where V states that the genotype of the donor of crime profile $c_{L(j)}$ is $g_{L(j)}$.

The Bayesian Network for calculating the denominator of the likelihood ratio is shown in Figure 5. The network is conditional on the defence hypothesis $V_d$. The ovals represent probabilistic quantities whilst the rectangles represent known quantities. The arrows represent probabilistic dependencies.

In general terms, the denominator can be stated as:

$$\sum_{\chi_i}\left[\Pi_j^{n\,loci}\sum f\left(c_{L(j)}\big|\ g_{u,\,L(j)}, V_d, \chi_i\right) \times p(g_{u,L(j)}\big|g_{s,L(j)})\ \right]p(\chi_i)$$

where the consideration is in effect, the genotype ($g_s$) is the donor of ($c_{h(j)}$) given the DNA quantity ($\chi_i$).

The general statements provided above for the denominator enable a suitable denominator to be established for the number of loci under consideration.

### 3.5 - The LR Denominator Quantification

In the denominator of the LR we need to calculate the likelihood of observing a set of heights giving any potential contributors. Most of the likelihoods would return a zero, if there is a height that is not explained by the putative unknown contributor. The presence of a likelihood of zero as the denominator in the LR would be detrimental to the usefulness of the LR.

In this section we provide with a method for generating genotype of unknown contributors that will lead to a non-zero likelihood.

For $c_{L(i)}$ there may be a requirement to augment with zeros to account for peaks that are smaller than the limit-of-detection threshold $T_d$. It is assumed that the height of a stutter is at most the height of the parent allele.

The various possible cases observed from a single unknown contributor are now considered. In the generic definitions, the allele number, stated as allele1, allele 2 etc refers to the sequence in the size ordered set of alleles, in ascending size.

### Possible Case 1 - Four peaks

For this to be a single profile we need the two pair of heights where each pair are adjacent. If the heights are $c_{L(i)} = \{h_1, h_2, h_3, h_4\}$, then the only possible genotype of the contributor is $g_U = \{2,4\}$. Crime profile $c_{L(i)}$ remains unchanged.

### Possible Case 2 - Three peaks with one allele not adjacent

In this cases, there are two sub-cases to consider:

- The larger two peaks are adjacent. If the peak heights are $c_{L(i)} = \{h_2, h_5, h_6\}$, then the only possible genotype is $g_{U,L(i)} = \{2,6\}$ and $c_{L(i)} = \{h_1, h_2, h_5, h_6\}$ where $h_1 = 0$.
- The smaller two peaks are adjacent. If the peak heights are $\{h_2, h_3, h_5\}$, the only possible genotype is $g_U = \{3,5\}$ and $c_{L(i)} = \{h_2, h_3, h_4, h_5\}$ where $h_4 = 0$.

### Possible Case 3 - Three adjacent peaks

The alleles heights can be written as $c_{L(i)} = \{h_2, h_3, h_4\}$. There are only two sub-cases to consider:

$g_{U,L(i)} = \{2,4\}$ or
$g_{U,L(i)} = \{3,4\}$.

- If $g_{U,L(i)} = \{2,4\}$, then $c_{L(i)} = \{h_1, h_2, h_3, h_4\}$ where $h_1 = 0$.
- If $g_{U,L(i)} = \{3,4\}$, then $c_{L(i)}$ remains unchanged.

### *Possible Case 4 - Two non-adjacent peaks*

If allele heights are $c_{L(i)} = \{h_2, h_4\}$, then the only possible genotype is $g_{U,L(i)} = \{2,4\}$ and $c_{L(i)} = \{h_1, h_2, h_3, h_4\}$ where $h_1 = 0$ and $h3 = 0$.

### *Possible Case 5 - Two adjacent peaks*

If allele heights are $c_{L(i)} = \{h_2, h_3\}$ then four possible genotypes need to be considered:

$g_{U,L(i)} = \{2,3\}$
$g_{U,L(i)} = \{3,3\}$
$g_{U,L(i)} = \{3,4\}$ or
$g_{U,L(i)} = \{3,Q\}$
where $Q$ is any other allele different than alleles 2, 3 and 4.

- if $g_{U,L(i)} = \{2,3\}$, then $c_{L(i)} = \{h_1, h_2, h_3\}$ where $h_1 = 0$
- if $g_{U,L(i)} = \{3,3\}$, then $c_{L(i)} = \{h_2, h_3\}$ remains unchanged
- if $g_{U,L(i)} = \{3,4\}$, then $c_{L(i)} = \{h_2, h_3, h_4\}$ where $h_4 = 0$
- if $g_{U,L(i)} = \{3,Q\}$, then $c_{L(i)} = \{h_2, h_3, h_{s,Q}, h_Q\}$ where $h_{s,Q} = h_Q = 0$.

### *Possible Case 6 - One peak*

If the peak is denoted by $c_{L(i)} = \{h_2\}$, then three possible genotypes need to be considered:

$g_{U,L(i)} = \{2,2\}$
$g_{U,L(i)} = \{2,3\}$ or
$g_{U,L(i)} = \{2,Q\}$
where $Q$ is any allele other than 2 and 3.

- if $g_{U,L(i)} \{2,2\}$, then $c_{L(i)} = \{h_1, h_2\}$ where $h_1 = 0$
- if $g_{U,L(i)} = \{2,3\}$, then $c_{L(i)} = \{h_1, h_2, h_3\}$ where $h_1 = h_3 = 0$
- if $g_{U,L(i)} = \{2,Q\}$, then $c_{L(i)} = \{h_1, h_2, h_{s,Q}, h_Q\}$ where $h_1 = h_{s,Q} = h_Q = 0$.

### *Possible Case 7 - No peak*

If this case the LR is one and therefore, there is no need to compute anything.

### 4. Detailed Description - Mixed Profile

### 4.1 - The Calculation of the LR

The aim of this section is to describe in detail the statistical model for computing likelihood ratios for mixed profiles while considering peak heights, allelic dropout and stutters.

In considering mixtures, there are various hypotheses which are considered. These can be broadly grouped as follows:

Prosecution hypotheses:

$V_p(S+V)$: The DNA came from the suspect and the victim;

$V_p(S_1+S_2)$: The DNA came from suspect 1 and suspect 2;

$V_p(S+U)$ : The DNA came from the suspect and an unknown contributor;

$V_p(V+U)$: The DNA came from the victim and an unknown contributor.

Defence hypotheses:

$V_d(S+U)$ : The DNA came from the suspect and an unknown contributor;

$V_d(V+U)$: The DNA came from the victim and an unknown contributor;

$V_d(U+U)$: The DNA came from two unknown contributors.

The combinations that are used in casework are:

$V_p(S+V)$ and $V_d(S+U)$;

$V_p(S+V)$ and $V_d(V+U)$;

$V_p(S+U)$ and $V_d(U+U)$;

$V_p(V+U)$ and $V_d(U+U)$;

$V_p(S_1+S_2)$ and $V_d(U+U)$.

If we denote by $K_1$ and $K_2$ the person whose genotypes are known, there are only three generic pairs of propositions:

$V_p(K_1+K_2)$ and $V_d(K_1+U)$;

$V_p(K_1+U)$ and $V_d(U+U)$ ;

$V_p(K_1+K_2)$ and $V_d(U+U)$.

The likelihood ratio (LR) is the ratio of the likelihood for the prosecution hypotheses to the likelihood for the defence hypotheses. In this section, that means the LR's for the three generic combinations of prosecution and defence hypotheses listed above.
Throughout this section $p(w)$ denotes a discrete probability distribution for mixing proportion $w$ and $p(x)$ denotes a discrete probability distribution for $x$.

**4.4.1- Proposition one** - $V_p(K_1+K_2)$ and $V_d(K_1 + U)$

The numerator of the LR is:

$$num = \sum_x \sum_w \prod_i^{n\,loci} f\left(C_{L(i)} \big| g1, L(i), g2, L_{(i)}, w, x\right) p\left(w\right) p\left(x\right)$$

where:

$g_1$ and $g_2$ are the genotypes of the known contributors $K_1$ and $K_2$ across loci;
c. is the crime profile across loci;

The subscript $L(i)$ means that the either the genotype of crime profile is for locus i or $n_{loci}$ is the number of loci.
The denominator of the LR is:

$$den = \sum_x \sum_w \prod_i^{n\,loci} \sum_{gU,L(i)} f\left(C_{L(i)} \big| g_1, L(i), gU, L(i), w, x\right) p\left(gU, L(i) \big| g_1, L(i), g_2, L(i)\right) p\left(w\right) p\left(x\right)$$

where:

gl,L(i) is the genotype of the known contributor in locus I;
g2,L(i) is a known genotype for locus i but it is not proposed as a genotype of the donor of the mixture;
gU,L(i) is the genotype of the unknown donor.

The conditional genotype probability in the right-hand-side of the equation is calculated using the Balding and Nichols model cited above.

The function in the left-hand side equation is calculated from probability distribution functions of the type described above and below.

### 4.4.2 - Proposition two - $V_p(K_1+U)$ and $V_d(U+U)$

The numerator is:

$$num = \sum_x \sum_w \prod_i^{n\ loci} \sum_{gU,L(i)} f\left(C_{L(i)} \middle| g_1, L_i, gu, L_{(i)}, w, x\right) p\left(gu, L(i) \middle| g1, L(i)\right) p(w)\ p(x)$$

where:

gl,L(i) is the genotype of the known contributor $K_1$ in locus i.

The denominator is

$$den = \sum_x \sum_w \prod_i^{n_{Loci}} \sum_{g_{U_1,\mathcal{L}(i)}, g_{U_2,\mathcal{L}(i)}} f\left(c_{\mathcal{L}(i)} \middle| g_{U_1,\mathcal{L}(i)}, g_{U_2,\mathcal{L}(i)}, w, x\right) p(g_{U_1,\mathcal{L}(i)}, g_{U_2,\mathcal{L}(i)} \middle| g_{1,\mathcal{L}(i)}, g_2, L(i)) p(w) p(x)$$

where:

gl,L(i) is the genotype of the known contributor $K_1$ in locus i ; and

$g_{U1}$,L(i) and $g_{U2}$,L(i) are the genotypes for locus i of the unknown contributors.

The second factor is computed as:

$$p(g_{U_1,\mathcal{L}(i)}, g_{U_2,\mathcal{L}(i)} \middle| g_{1,\mathcal{L}(i)}) = p(g_{U_1,\mathcal{L}(i)} \middle| g_{1,\mathcal{L}(i)}, g_{U_2,\mathcal{L}(i)}) p(g_{U_2,\mathcal{L}(i)} \middle| g_{1,\mathcal{L}(i)})$$

The factors in the right-hand-side of the equation are computed using the model of Balding and Nichols cited above.

### 4.4.3 - Proposition three - $V_p(K_1+K_2)$ and $V_d(U+U)$

The numerator is the same as the numerator for the first generic pair of hypotheses. The denominator is almost the same as the denominator for the second generic pair of propositions except for the genotypes to the right of the conditioning bar in the conditional genotype probabilities. The denominator of the LR for the generic pair of propositions in this section is:

$$den = \sum_x \sum_w \prod_i^{n_{Loci}} \sum_{g_{U_1,\mathcal{L}(i)}, g_{U_2,\mathcal{L}(i)}} f\left(c_{\mathcal{L}(i)} \middle| g_{U_1,\mathcal{L}(i)}, g_{U_2,\mathcal{L}(i)}, w, x\right) p(g_{U_1,\mathcal{L}(i)}, g_{U_2,\mathcal{L}(i)} \middle| g_{1,\mathcal{L}(i)}, g_{2,\mathcal{L}(i)}) p(w) p(x)$$

where:

gl,L(i) and g2,L(i) are the genotypes of the known contributors $K_1$ and $K_2$ in locus i;

$g_{U_1}$,L(i) and $g_{U_2}$,L(i) are the genotypes for locus i of the unknown contributors.

The second factor is computed as:

$$p(g_{U_1,\mathcal{L}(i)}, g_{U_2,\mathcal{L}(i)} \middle| g_{1,\mathcal{L}(i)}, g_{2,\mathcal{L}(i)}) = p(g_{U_1,\mathcal{L}(i)} \middle| g_{1,\mathcal{L}(i)}, g_{2,\mathcal{L}(i)}, g_{U_2,\mathcal{L}(i)}) p(g_{U_2,\mathcal{L}(i)} \middle| g_{1,\mathcal{L}(i)}, g_{2,\mathcal{L}(i)})$$

The factors in the right-hand-side of the equation are computed using the model of Balding and Nichols cited above.

### 4.2 - Density value for crime profile given two putative donors

The terms in the calculations above are put together using per locus conditional genotype probabilities and density values of per locus crime profiles given putative per locus genotypes of two contributors. The conditional genotype probabilities are calculated using the model of Balding and Nichols cited above. In this section we focus on the density values of per locus crime profiles.

For the sake of clarity and brevity of explanation, the method for calculating the density value $f(cL(i)|g1,L(i),g2,L(i),w,x)$ is explained through an example.

*Example*

The genotypes and crime profiles are: $g_{1,\mathcal{L}(i)} = \{16,17\}$  $g_{2,\mathcal{L}(i)} = \{18,20\}$

$c_{\mathcal{L}(i)} = \{h_{*,15},h_{*,16},h_{*,17},h_{*,18},h_{*,19},h_{*,20}\}$ .

We first obtain an intermediate probability density function (PDF) defined as the product of the factors:

1.

$$f(h_{1,15},h_{1,16},h_{1,17} \mid g_{1,\mathcal{L}(i)} = \{16,17\}, w \times x)$$

2.

$$f(h_{2,17},h_{2,18},h_{2,19} \mid g_{2,\mathcal{L}(i)} = \{18,28\}, (1-w) \times x)$$

3. $\delta_S(h_{17}|h_{1,17},h_{2,17})$

The first factor has been already defined as a PDF for a single contributor: in this case the donor is g1,L(i)= {16,17} and DNA quantity $w$ x $x$. The second factor has also being defined as a PDF for a single contributor: the donor in this case is g2,L(i)={18,28} and DNA quantity $(1-w)$x $x$. The third factor is a degenerated PDF defined by: $\delta_s(h_{17}|h_{1,17},h_{2,17}) = 1$ *if* $h_{17} = h_{1,17} + h_{2,17}$ and zero otherwise. The intermediate PDF is denoted by f($h_{1,15},h_{1,16},h_{1,17},h_{17},h_{2,17}h_{2,18},h_{2,19}$). The required density value is obtained by integration:

$$f(h_{*,15},h_{*,16},h_{*,17},h_{*,18},h_{*,19}) = \int f(h_{*,15},h_{*,16},h_{1,17},h_{*,17},h_{2,17},h_{*,18},h_{*,19})dh_{1,17}dh_{2,17}$$

where $f(h_{*,15},h_{*,16},h_{*,17},h_{*,18},h_{*,19}) = f\left(c_{\mathcal{L}(i)} \mid g_{1,\mathcal{L}(i)},g_{2,\mathcal{L}(i)},w,x\right)$ in this example.

Notice that $h_{1,15}$ has been replaced by the observed height in the crime profile $h_{*,15}$. This is because $h_{1,15}$ represents a generic variable and $h_{*,15}$ represent an observed height. (For example, cosine(y) represents a generic function but cosine($\pi$) represent the evaluation of the function cosine for the value $\pi$.). Notice as well that the height h*,15 is only explained by the stutter of allele 16.

In contrast, $h_{1,17}$ and $h_{2,17}$ are not replaced by $h_{*,17}$ because $h_{*,17}$ is form as the sum of $h_{1,17}$ and $h_{2,17}$. We do not know the observed values but only the sum of them. (If we observe number 10 and we are told that it is the sum of two numbers, there are many possibilities for the two numbers: 1 and 9, 2 and 8, 1.1 and 8.9, etc.). The integration considers all of the possible $h_{1,17}$ and $h_{2,17}$. The variable that take these values is known as a hidden, latent or unobserved variable.

The integration can be achieved using any type of integration, including, but not limited to, Monte Carlo integration, and numerical integration. The preferred method is adaptive numerical integration in one dimension in this example, and in several dimensions in general.

The general methods is to generate an intermediate PDF using the PDF of the contributor and by introducing $\delta_s$ PDFs for the height pairs that fall in the same position. There can be cases when more than one pair of heights fall in the same position. For example if g1,L(i)={16,17} and g2,L(i)={16,17}, then there are three pairs of heights falling in the same position: one in position 15, another in position 16 and the third in position 17.

If one of the observed heights is below the limit-of-detection threshold $T_d$, we need to perform further integration to consider all values. For example if h{*,15} is reported as below the limit-of-detection threshold $T_d$ and all other heights are greater than the limit-of-detection threshold, the PDF value that we are interested become a likelihood given by:

$$f(h_{*,15} < T_d, h_{*,16}, h_{*,17}, h_{*,18}, h_{*,19}) = \int_{h_{*,15} < T_d} f(h_{15}, h_{*,16}, h_{*,17}, h_{*,18}, h_{*,19}) dh_{15}$$

The integral consider all the possibilities for $h_{15}$. In general we need to perform an integration for each height that is smaller than $T_d$. Any method for calculating the integral can be used. The preferred method is adaptive numerical integration.

## 5 Detailed Description - Intelligence Uses

### 5.1 - Use in Intelligence Applications

In an intelligence context, a different issue is under consideration to that approached in an evidential context. The intelligence context seeks to find links between a DNA profile from a crime scene sample and profiles stored in a database, such as The National DNA Database® which is used in the UK. The process is interested in the genotype given the collected profile.

Thus in this context, the process starts with a crime profile $c$, with the crime profile consisting of a set of crime profiles, where each member of the set is the crime profile of a particular locus. The method is interested in proposing, as its output, a list of suspect's profiles from the database. Ideally, the method also provides a posterior probability (to observing the crime profile) for each suspect's profile. This allows the list of suspect's profiles to be ranked such that the first profile in the list is the genotype of the most likely donor.

Where the profile is from a single source, a single suspect's profile and posterior probability is generated. Where the profile is from two sources, a pair of suspect profiles and a posterior probability are generated.

### 5.2 - Intelligence Application - Single Profile

As described above, the process starts with a crime profile $c$, with the crime profile consisting of a set of crime profiles, where each member of the set is the crime profile of a particular locus. The method is interested in proposing a list of single suspect profiles from the database, together with a posterior probability for that profile. This task is usually done by proposing a list of genotypes $\{g_1, g_2, ..., g_m\}$ which are then ranked according the posterior probability of the genotype given the crime profile.

The list of genotypes is generated from the crime scene $c$. For example if $c = \{h_1, h_2\}$, where both $h_1$ and $h_2$ are greater than the dropout threshold, $t_d$, then the potential donor genotype is generated according to the scenarios described previously. Thus, if the peaks are not adjoining, then the lower size peak is not a possible stutter and g = {1,2}. If the peaks are adjoining, then g = {1, 2} and g = {stutter2, 2} are possible, and so on.

The quantity to be computed is the posterior probability, $p(g_i|c)$, for all possible genotypes across the profile, $g_i$. This quantity can be defined as:

$$p(g_i | c) = \frac{f(c | g_i) p(g_i)}{\sum_{g_j} f(c | g_j) p(g_j)}$$

where $p(g_i)$ is a prior distribution for genotype $g_i$, preferably computed from the population in question.

The likelihood $f(c|g)$ can be computed using the approach of section 3.2 above, but with the modification of replacing the suspect's genotype by one of the generated $g_i$.

Thus the computation uses:

$$L_p = \sum_{\chi_i} L_{p,L(1)}(\chi_i) \times L_{p,L(2)}(\chi_i) \times L_{p,L(3)}(\chi_i) \times p(\chi_i)$$

Where $L_{p,L(j)}(\chi_i)$ is the likelihood for locus $j$ conditional on DNA quantity, this assumes the abstracted form:

$$L_{p,L(j)}(\chi) = f\left(c_{L(j)} \mid g_{s,L(j)}, V_p, \chi_j\right)$$

or:

$$L_{p,L(j)}(\chi) = f\left(c_{L(j)} \mid g_{h(j)}, V, \chi_j\right).$$

or:

$$\sum_{\chi_i}\left[\Pi_j^{n\ loci} f\left(c_{h(j)} \mid g_{s,L(j)}, V_p, \chi_i\right)\right]p(\chi_i)$$

The prior probability $p(g_i|c)$ is computed as:

$$p(g_i) = \prod_{k=1}^{n_{loci}} p\left(g_{i,L(k)}\right)$$

Each factor in this product can be computed using the following approach.
The approach inputs are:

> $g$ - a genotype;
> $AlleleList$- a list of observed alleles - this may include allele repetitions, such as {15,16;15,16};
> $locus$ - an identifier for the locus;
> $theta$ - a co-ancestry or inbreeding coefficient - a real number in the interval [0,1];
> $eaGroup$ - ethnic appearance group - an identifier for the ethnic group appearance, which can change from country to country;
> $alleleCountArray$ - an array of integers containing counts corresponding to a list of alleles and loci.

The approach outputs are:
$Prob$ - a probability - a real number with interval [0,1].
The algorithmical description becomes:

> a) if g is a heterozygote, then multiply by 2;
>
> b)

$$N = length(g) + length(allelelist);$$

> c)

$$den = [1 + (N - 2)\theta][1 + (N - 3)\theta];$$

> d) $n_1$ is the number of times that the first allele $g(1)$ is present in allelelist $\cup$ $g(2)$;
>
> e) $n_2$ is the number of times that the second allele $g(2)$ is present in the list $alleleList$.
>
> f)

$$num = \left[(n_1 - 1)\theta + (1-\theta)*p_1\right]\left[(n_2 - 1)\theta + (1-\theta)*p_2\right]$$

where $p_1$ is the probability of allele $g(1)$ and $p_2$ is the probability of allele $g(2)$.

### 5.3 - Intelligence Application - Mixed Profile

In the mixed profile case, the task is to propose an ordered list of pairs of genotypes $g_1$ and $g_2$ per locus (so that the first pair in the list are the most likely donors of the crime stain) for a two source mixture; an ordered list of triplets of genotypes per locus for three source sample, and so on.

The starting point is the crime stain profile $c$. From this, an exhaustive list $\{g_{1,i}, g_{2,i}\}$ of pairs of potential donors are generated. The potential donor pair genotypes are generated according to the scenarios described previously taking into account possible stutter etc.

For each of theses pairs, a probability distribution for the genotypes is calculated using the formula:

$$p\left(g_1, g_2 \mid c\right) = \frac{f\left(c \mid g_1, g_2\right) p\left(g_1, g_2\right)}{\sum_{gi, gj} f\left(c \mid g_i, g_j\right) p\left(g_i, g_j\right)}$$

where $p\{g_1, g_2\}$ and/or $p(g_i, g_j)$ are a prior distribution for the pair of genotypes inside the brackets that can be set to a uniform distribution or computed using the formulae introduced by Balding et al.

In practice, there is no need to compute the denominator as the computation extends to all possible genotypes. The term can be normalised later. As described above for evidential uses, for instance, the core term is the calculation of the likelihood $f(c|g_1, g_2)$. This can be computed according to the formula:

$$f\left(c \mid g_1, g_2\right) = \sum_x \sum_w \prod_i^{n_{loci}} f\left(c_{L(i)} \mid g_{1, L(i)}, g_{2, L(i)}\right) p(w) p(x)$$

where the term:

$$p\left(g_1, g_2\right) = \prod_i^{n_{loci}} p\left(g_{1, L(i)} \mid g_{2, L(i)}\right) p\left(g_{1, L(i)}\right)$$

Each factor in this product can be computed using the approach described in section 5.2 above.

### 6 Extension to Include Variable Peak Height Impact Effects in the Model

### 6.1 - Background

In practice, there are a variety of effects which impact upon the way in which different allele sizes and/or different loci sizes are observed in the results.

For instance, degradation of DNA samples occurs with time due to various factors. When the effect occurs it impacts by resulting in a reduction in the observed peak height of an allele as the degraded DNA does not contribute to that peak (or any of the peaks) within the analysis. However, the impact of degradation is not consistent across all loci. Higher molecular weight loci are subjected to greater levels of degradation than lower molecular weight loci within a sample.

Another instance of an effect having a variable impact is variations in amplification efficiency within and/or between loci. Lower amplification efficiency effects will impact in terms of lower peaks for the quantity of DNA present than is the case for higher amplification efficiency effects.

Another instance is sampling effects, where because the number of molecules of DNA forming the starting point for amplification is small, any variation in the number of molecules when the sub-samples of the DNA sample are generated will have a material effect on the peak heights.

In general, the effect can be considered as any effect which has the impact of causing peak imbalance in the results.

### 6.2 - Experimental determination of the information

In the technique which follows, there is a need to have available information on the mean height of an allele for a locus and the variance thereof.

This information could be generated by a model of the results observed for various alleles under various conditions. In this instance, however, experimentally derived information is used.

### 6.2.1 - Profile Generation

A total of 865 profiles were produced from 15 volunteers who donated three buccal scrapes (using Whatman®OmniSwab™). Nineteen DNA templates were targeted through a dilution series from 50 to 500 picograms per microlitre ($pg/\mu l$) in increments of 25 $pg/\mu l$, covering a template range where allelic dropout is possible. To cover current protocols used in the FSS, three combination of amplification and detection were selected that represent current of casework samples: (a) Tetrad and 3100; (b) Tetrad and 3130x1 and (c) 9700 and 3130x1. The protocol used is now described.

### 6.2.2 - Extraction

The serrated collection area of each swab was deposited into a micro test tube (Eppendorf Biopur Safe-Lock, 1.5 $\mu l$, individually sealed). DNA was purified from the buccal scrapes using a Qiagen EZ1 and the EZ1 DNA tissue kit. Each donor's three purified DNA samples were hen pooled into a single sample to ensure that a sufficient volume of high concentration DNA was available.

### 6.2.3 - Dilution Series

The DNA in each pooled sample was measured in duplicate using the 7500 Real Time PCR System (Applied Biosystems) and the Quantifiler Human DNA Quantification kit (Applied Biosystems). Each pooled extract was first used to create stock volumes of 100 pg/l, 250 pg/l and 500 pg/l. The stock volumes were then used to generate diluted volumes such that the addition of 101 to the amplification reaction will provide each of the 19 target template levels in the dilution series.

### 6.2.4 - Amplification

Amplification was performed for each donor at each template level using the AmpFSTR SGM Plus PCR Amplification Kit (Applied Biosystems) on theremocycler MJ Research PTC-225 Tetrad. A reaction volume of 25 1 was used for each amplification. Protocols that use a reaction volume of 25 I have been tested in the FSS and they produce comparable profiles to protocols that use 50 l.

### 6.2.5 - Detection

Two genetic analyzers were used: (1) the 3100 Genetic Analyzer (Applied Biosystems) using POP4TM (Applied Biosystems) and injection parameters of 1 kV for 22 seconds. (2) the 3130x1 Genetic analyzer using injection parameters of 1.5 kv for 10 seconds and 3 kv for 10 seconds.

### 6.2.6 - Interpretation

Analysis and genotyping of the run files was carried out using GeneMapper® ID v3.2 (Applied Biosystems). A series of peak positions and heights were thus obtained for the allele or alleles present at each locus for each sample.

### 6.2.7 - Data Generation

### 6.2.7.1 - Allele Mean v Quantity Distribution Fitting

All the heights within a profile were added together (except the Amelogenin height) to give the $\chi$ value; DNA quantity. The sum of the heights $\chi_{l(i)}$ in locus $l(i)$ was then computed from the same basic data and the mean height of allele obtained. This information formed the data points for a plot of mean peak height against DNA quantity x scaling factor. In the case of the Figure 11a illustration, mean peak height is plotted against DNA quantity x 10 (as a scaling factor).

A linear Gamma distribution (the line shown) is then fitted to these points. The allele mean for locus $l(i)$, denoted by $\mu_{a,l(i)}$ is modelled with a regression line through the origin: $\mu_{a,l(i)} = \kappa_{1,a,l(i)} x \chi$ where $\kappa_{1,a,l(i)}$ is the amplified DNA proxy $\chi$, by summing all peak heights above the limit of detection threshold $T_d$ = 30rfu in the profile, except for Amelogenin.

*6.2.7.2 - Stutter Mean v Quantity Distribution Fitting*

In a similar manner, a plot of mean stutter peak height against DNA quantity x scaling factor can be obtained. The stutter mean model for locus *l(i)*, denoted by $\mu_{s,l(i)}$ is also modelled with a regression line through the origin: $\mu_{s,l(i)} = \kappa_{1,s,l(i)} x \chi$.

*6.2.7.3 - Allele Variance Distribution Fitting*

In the next step of the data generation, the approach goes on to consider the variances for the alleles based upon the expected mean and the observed means. By plotting variance against the mean height, a plot of the type shown in Figure 11b can be obtained.

Again a Gamma distribution can be fitted to it; in this case two different distributions are fitted to the two different sections, with a knot joining them. The allele variance is modelled with two quadratic polynomials joined in a chosen knot. A knot is chosen through experimenting with several candidates and selecting candidates that give a good fit. The result is stated as, if $\mu \leq knot$:

$$\sigma^2 = \kappa_{2,1,l(i)} x \mu + \kappa_{3,1l(i)} x \mu^2$$

If $\mu > knot,$

$$\sigma^2 = \kappa_{2,2,l(i)} x \mu + \kappa_{3,2l(i)} x \mu^2$$

The allele variance model is used for stutter because stutter heights are smaller than allele heights and are more affected by the censoring of 30rfu. Peak heights of alleles and stutters are assumed to follow a Gamma distribution where the parameters $\alpha$ and $\beta$ are calculated from the mean and variance specified above.

*6.2.7.4 - Examples for Loci*

The process is repeated for all the loci of interest.

In Figures 12a and 12b the allele and stutter results are shown for the D3 locus. Figures 13a and 13b show the allele and stutter results for the vWA locus. Figures 14a and 14b show the allele and stutter results for the D16 locus. Figures 15a and 15b show the allele and stutter results for the D2 locus. Figures 16a and 16b show the allele and stutter results for Amelogen. Figures 17a and 17b show the allele and stutter results for the D8 locus. Figures 18a and 18b show the allele and stutter results for the D21 locus. Figures 19a and 19b show the allele and stutter results for the D18 locus. Figures 20a and 20b show the allele and stutter results for the D19 locus. Figures 21a and 21b show the allele and stutter results for the THO locus. Figures 22a and 22b show the allele and stutter results for the FGA locus.

*6.2.7.5 -Equivalent $\beta$ values*

In the next step, the approach provides for the system preference for the $\beta$ values for the Gamma distributions to be the same, and hence the requirement for a linear relationship: $\sigma^2 = k_2 x \mu$. Hence:

$$\beta = \frac{\mu}{\sigma^2} = \frac{\mu}{k_2 . \mu} = \frac{1}{k^2}$$

As a single estimate of $k_2$ is required for all $\mu$'s, if for example, there are three peaks with means $\mu_1 = 200$, $\mu_2 = 400$ and $\mu_3 = 600$, the model of Figure 11b can be used to give the variance values, $\sigma_1^2$, $\sigma_2^2$ and $\sigma_3^2$. A least-squares line is then estimated for these points, Figure 11c and the $k_2$ slope is obtained as a result.

*6.2.8 - Estimate of the allele model*

The EM algorithm is used to estimate the parameters of the mean and variance models. The values of the parameters in iteration *m* is denoted by:

$$\kappa_{1,a,l(i)}\left[m\right],\kappa_{2,1,l(i)}\left[m\right],\kappa_{3,1,l(i)}\left[m\right],\kappa_{2,2,l(i)}\left[m\right],\kappa_{3,2l(i)}\left[m\right],$$

In the first iteration we ignore zeros, i.e. heights smaller than $T_d$ = 30. From the second iteration onwards, the zeros are replaced by samples obtained from the tail of the Gamma pdf's estimated in the previous step. More specifically,

1. Parameter $k_{1,a,l(i)}[1]$ is estimated using standard linear regression methods where the response variable are non-zero allele heights and the covariate is the corresponding $\chi$.

2. Parameters $k_{2,1,l(i)}[1]$, $k_{3,1,l(i)}[1]$, $k_{2,2,l(i)}[1]$, $k_{3,2,l(i)}[1]$ are estimated by using the estimated mean $\mu_{a,l(i)}$ as the mean and computing the variance of the heights around these mean according to a window size.

3. In iteration $m$, zeros are replaced by samples taken from the family of Gamma distribution estimated in the previous iteration. In more detail, for a zero corresponding to $\chi$, we first obtain a $\mu_{a,l(i)}[m$ - $1]$ from $\chi$ and $\sigma^2_{a,l(i)}\left[m-1\right]$ from $\mu_{a,l(i)}[m$ - $1]$. Parameter $\alpha[m$ - $1]$ and $\beta[m$ - $1]$ can be computed from $\mu_{a,l(i)}[m$ - $1]$ and $\sigma^2_{a,l(i)}\left[m-1\right]$. A sample is then taken in the interval $(0, 30)$ from the tail of the distribution using the CDF inverse method using uniform samples in the interval $(0, F(30, \alpha[m$ - $1], \beta[m$ - $1]))$ where F is the CDF of a Gamma distribution.

4. Parameter $k_{1,a,l(i)}[m]$ is also estimated using standard linear regression methods where the response variable are allele heights and the covariate are the corresponding $\chi$'s. Zeros are replaced using the method described above.

5. Parameters $k_{2,1,l(i)}[m]$, $k_{3,1,l(i)}[m]$, $k_{2,2,l(i)}[m]$, $k_{3,2,l(i)}[m]$ are estimated by using the estimated mean $\mu_{a,l(i)}[m]$ as the mean and computing the variance of the heights around these mean according to a window size.

6. The process is repeated until the parameters converge according to the rules:

(a) $|k_{1,a,l(i)}[m]$ - $k_{1,a,l(i)}[m$-$1]|<0.0001$;
(b) $|k_{2,a,l(i)}[m]$ - $k_{2,a,l(i)}[m$-$1]|<0.01$; and
(c) $|k_{3,a,l(i)}[m]$- $k_{3,a,l(i)}[m$-$1]|<0.001$.

*6.2.9 - Estimation of the Stutter Model*

The same methodology is used for estimating the parameters of the stutter model except that we have more zeros: stutter peaks are much smaller than allele peaks. To alleviate the extra variability introduced by the zeros we use the variance model for the allele and iterate only for the stutter mean.

*6.2.10 - Variances for means exceeding the maximum*

If an $\chi$ is larger than the one support by the data, we use the regression line to extrapolate a value for the allele and stutter means. Extrapolation of the variance is a more involved process.

The profiles provide estimates for variances up to a maximum value for the mean value denoted by $\mu_{max}$. To extrapolate the variances we use the coefficient of variation, denoted here by $v$, i.e the standard deviation divided by the mean ($v = \sigma/\mu$). The coefficient of variation decreases as the mean increases, however, its rate of reduction also decreases. Figures 12d to 22d show the coefficient of variation for each locus. We therefore use the last value of the coefficient of variation, denoted by $v_{max}$. For a given mean $\mu$ larger than $\mu_{max}$, we can compute the variance as: $\sigma^2 = (v_{max} \times \mu)^2$.

## 6.3 - **Single Source Profiles**

Having established the background information needed, the manner in which the approach is implemented for a variety of sample situations can be discussed, starting with samples from a single source.

### 6.3.1 Evidential Uses

As mentioned above, in the context of evidential uses, there is consideration of the term:

$$LR = \frac{f(c|g_s, V_p)}{f(c|g_s, V_d)}$$

with the crime profile c in a case consists of a set of crime profiles, where each member of the set is the crime profile of a particular locus. Similarly, the suspect genotype $g_s$ is a set where each member is the genotype of the suspect for a particular locus. As a result, the notation used was:

$$c = \left\{ c_{L(i)} : i =, 2, ..., n_i \right\} \quad \text{and} \quad g_s = \left\{ g_{s,L(i)} := 1, 2, ..., n_i \right\}$$

where $n_i$ is the number of loci in the profile.

As a result, this provides for the height of the crime scene profile at the locus being considered, but then being summed together with the heights of all the loci. The sum was used in the subsequent considerations and the heights at the individual loci were not made any further use of.

However, as mentioned in section 6.1, peak imbalance effects (such as degradation effects) are locus and even allele dependent in the occurrence and extent. For example, locus vWA undergoes greater extents of degradation than locus D3 in the same sample.

In accounting for peak imbalance, therefore, the model moves to condition on the sum per locus; $\chi_{l(i)}$, the sum of peak heights in a locus. In effect, this considers the Bayesian Network shown in Figure 10 which represents the position for two of the loci L under consideration.

## 6.3.1.1 - Numerator

On this basis, the denominator in the LR can be expressed as:

$$Num = f\left( c \middle| g_s, H_p \right) = \prod_{i=T}^{n_l} f(C_{l(i)} \middle| g_{s,l(i)}, H_P) \chi_{l(i)}, \delta)$$

where the peak heights are summed for loci i and $\delta$ is a parameter, peak imbalance parameter or EAQ, that takes into account effects within a locus (and which is discussed further in section 6.5.

The right-hand side factor of the above equation, $f(C_{l(i)}|g_{s,l(i)},H_P,\chi_{l(i)},\delta)$ can be written as:

$$f(C_{l(i)} \middle| g_{l(i)}, \chi_{l(i)}, \delta)$$

where it is assumed that $g_{l(i)}$ is the genotype of the donor of $C_{l(i)}$ (the donor varying according to the prosecution hypothesis and the defense hypothesis). This is a core pdf in the considerations made by the invention and is discussed further below.

## 6.3.1.2 - Denominator

The denominator can be expressed as:

$$Den = f\left( c \middle| g_s, H_d \right) = \prod_{i=T}^{n_l} f(C_{l(i)} \middle| g_{s,l(i)}, H_d, \chi_{l(i)}, \delta)$$

The right-hand side factor of the above equation, $f(C_{l(i)}|g_{s,l(i)},H_d,\chi_{l(i)},\delta)$ can be written as:

$$f(C_{l(i)} \middle| g_{s,l(i)}, \chi_{l(i)}, H_d, \delta) = \sum_i f(C_{l(i)} \middle| g_{u(i)}, \chi_{l(i)}, H_d, \delta, g_{u,l(i)}) \Pr(g_{u,l(i)} \middle| g_{s,l(i)})$$

where the function $f(C_{l(i)}|g_{u(i)},\chi_{l(i)},H_d,\delta,g_{u,l(i)})$ can be written as:

$$f(C_{l(i)} \middle| g_{l(i)}, \chi_{l(i)}, \delta)$$

where we assume that $g_{l(i)}$ is the genotype of the donor of $C_{l(i)}$. This is a pdf of the same form as referenced above as core to the invention.

Once again, the right-hand term is a conditional genotype probability. This can be computed using existing formula for conditional genotype probabilities given putative related and unrelated contributors with population structure or not, for instance using the approach defined in J.D. Balding and R. Nichols. DNA profile match probability calculation: How to allow for population stratification, relatedness, database selection and single bands. Forensic Science International, 64:125-140, 1994.

### 6.3.2 - Intelligence Uses

In this use, the task is to compute posterior probabilities of the genotype given the crime profile for locus i. Given the crime stain, quantity of DNA and peak imbalance/EQA parameter, the use assigns probabilities to the genotypes which could be behind it. The term $\chi_{l(i)}$ denotes the sum of peak heights in locus i bigger than reporting threshold $T_r$. The term $\delta$ denotes the EAQ factor, described in below.

The posterior genotype probability for $g_{u,J(i)}^*$ given $C_{l(i)}$, $\chi_{l(i)}$ and $\delta$ is calculated using Bayes theorem:

$$p(g_{u,J(i)}^* \big| C_{l(i)}, \chi_{l(i)}, \delta) = \frac{f\left(c_{l(i)} \big| g_{u,J(i)}^*, \chi_{l(i)}, \delta\right) p\left(g_{u,J(i)}^*\right)}{\sum_{g_{U,J(i)}} f\left(c_i \big| g_{U,J(i)}, \chi_{l(i)}, \delta\right) p\left(g_{U,J(i)}\right)}$$

where $p(g_{U,l(i)})$ is the probability of genotype $g_{U,l(i)}$ prior to observing the crime profile. In this version of the method we chose to set a uniform prior to all genotypes so that only the effect of the crime profile is considered. The formula above is simplified to:

$$p(g_{u,J(i)}^* \big| C_{l(i)}, \chi_{l(i)}, \delta) = \frac{f\left(c_{l(i)} \big| g_{u,J(i)}^*, \chi_{l(i)}, \delta\right)}{\sum_{g_{U,J(i)}} f\left(c_i \big| g_{U,J(i)}, \chi_{l(i)}, \delta\right)}$$

As above in the evidential uses, both numerator and denominator can be presented in a form based around the core pdf:

$$f(C_{l(i)} \big| g_{l(i)}, \chi_{l(i)}, \delta)$$

where we assume that $g_{l(i)}$ is the genotype of the donor of $C_{l(i)}$.

In this use, it is not necessary to compute all possible genotypes in a locus: most of the probabilities would be zero. Instead we generate genotypes that may lead to a non-zero posterior probability. Starting with the crime profile $C_{l(i)}$ in this locus, peaks are designated either as a stutter or alleles. The set of designated alleles is used for generating the possible genotypes. There are only three possibilities:

1. No peaks bigger than reporting threshold $T_r$. No genotype is generated as there is no peak height information to inform them.
2. One peak bigger than $T_r$ as allele, denoted by *a*. The possible genotypes are {a,a} and {a, Q} where *Q* denotes any allele other than *a*.
3. Two peaks bigger than $T_r$ designated as alleles, denoted by *a* and *b*. In this case the only possible genotype is {a,b}.

### 6.3.3 - The function $f(C_{l(i)}|g_{l(i)}, \chi_{l(i)}, \delta)$

In the above sections, the pdf: $f(C_{l(i)}|g_{l(i)}, \chi_{l(i)}, \delta)$ is explained as important to the calculation of the likelihood ratio, LR in each case.

In this first consideration, we consider the position where allele dropout is not involved given the suspect's genotype.

In a second consideration, below, we consider the position where allele dropout is involved given the suspect's genotype.

### 6.3.3.1 - First Consideration

The first consideration opens with those cases where all the expected peaks given the genotype, including any stutter peaks present, are above the detection threshold limit T. The genotype is denoted as:

$g_{l(i)} = \{a_{1,l(i)}, a_{2,l(i)}\}$ where the alleles a may be the same (homozygous) or different (heterozygous) in that locus, i. The pdf is constructed in seven steps.

**Step 1** The peak-height sum is denoted by $\chi_{l(i)}$. Let's denote the corresponding means for the peak heights of the alleles and the stutters of the putative donor $g_{l(i)}$ by $\mu_{a,1,l(i)}$ and $\mu_{s,1,l(i)}$ respectively. They are a function of $\chi_{l(i)}$ and obtained as described elsewhere. For each allele a of the donor, we assign the allele mean $\mu_{a,1,l(i)}$ to the position of allele a, and the stutter mean $\mu_{s,1,l(i)}$ to a position a-1. If the donor is homozygote, we do the assignment twice.

**Step 2** If the donor is a heterozygote, the means are modified using the EAQ factor $\delta$ to take into account factors, such as PCR efficiency and degradation, that affect the resulting peak heights. For example, if the donor is a heterozygote in locus l(i) the mean for his/her alleles and stutters are: $\delta_1 x \mu_{a,1,l(i)}$ and $\delta_1 x \mu_{s,1,l(i)}$ for the low-molecular-weight allele and $\delta_2 x \mu_{a,1,l(i)}$ and $\delta_2 x \mu_{a,1,l(i)}$ for the high-molecular-weight allele. We give a detailed description of the method for calculating $\delta_1$ and $\delta_2$ elsewhere.

**Step 4** The variances for each allele and stutter are obtained as a function of their corresponding means and obtained using the method described above. In alter step we need to add random Gamma variables. A condition for a close form calculation of this addition is that the $\beta$-parameters are the same. Also in a later step, we divide each Gamma by the overall sum of peak height to account for using the sum of peak heights in this locus. A closed form calculation can be done if all $\beta$ parameters are the same. The conditioned on the $\beta$-parameters can be obtained by estimating a line between the points form by the means, in the x-axis, and the variances, in the y-axis. A regression line with zero intercept is fitted to obtain:

$$\hat{\sigma}^2 = \kappa_2 x \mu$$

So, if peak i has mean and variance $(\mu_i, \sigma_i^2)$,

$$\beta = \mu_i / \sigma_i^2 = \mu_i / (\kappa_2 x \mu_i) = 1/\kappa_2$$

regardless of the value of $\mu_1$.

**Step 5** The shape ($\alpha$) and rate ($\beta$) parameters are obtained from the mean and the variances.

**Step 6** The alpha parameters for alleles and stutters in the same allele position are added to obtain an overall $\alpha$ for that allele position. Now we have the parameters of a Gamma distribution for each allele position.

**Step 7** To account for using the sum of peak height in the locus, the collection of Gamma pdfs whose peak heights are above the peak-height reporting limit are converted to a Dirichlet pdf. This is achieved in closed form because all $\beta$'s are the same. The resulting Dirichlet pdf inherit the $\alpha$ parameters of the Gammas.

### 6.3.3.2 Second Consideration

In the second consideration, allele dropout is invoked given the suspects genotype, the consideration has to reflect one or more of the heights in the profile being below the threshold T. In such a case, the peak which is below the threshold does not form part of the value of $\chi_{l(i)}$ and the correction is only applied to those peaks above the threshold.
For example, in the case of a non-adjacent heterozygous alleles case, when $h_{s,1,l(i)} < T$ then the PDF is given by:

$$f\left(h_{s,1,l(i)} < T, h_{a,1,l(i)}, h_{s,2,l(i)}, h_{a,2,l(i)} \middle| g_{l(i)} = \{a_{1,l(i)}, a_{2,l(i)}\}, \chi_{l(i)}, \delta\right)$$

which can be expressed as:

$$F(T | \alpha_{s,1,l(i)}, \beta) f_{Dir}(\pi_{a,1,l(i)}, \pi_{s,2,l(i)}, \pi_{a,2,l(i)}, | \alpha_{a,1,l(i)}, \alpha_{s,2,l(i)}, \alpha_{a,2,l(i)},)$$

where F is the cdf of a gamma distribution with parameters $\alpha_{s,1,l(i)}$ and $\beta$.
If there is more than one peak below the threshold T, then there will be a corresponding number of f.

The approach for the second consideration is closely based on the approach for the first consideration, together with these revisions.

### 6.3.3.3 - Example - One allele

In this case, the donor is homozygous. Hence, the term $\alpha_{a,1,l(i)}$ is deployed twice for the allele and the term $\alpha_{s,1,l(i)}$ is deployed twice for the stutter (if present). The probability density for $c_{l(i)}$ is given by multiplying two Gamma pdf's. The first has parameters $2\alpha_{s,1,l(i)}$ and $\beta$, and the second has parameters $2\alpha_{a,1,l(i)}$ and $\beta$. Thereby giving the expression:

$$f(h_{s,1,l(i)}, h_{a,1,l(i)} \big| g_{l(i)} = \left\{ a_{1,l(i)}, a_{2,l(i)} \right\}, \chi_{l(i)}, \delta)$$

which can be expressed as:

$$f(h_{s,1,l(i)} \big| 2\alpha_{s,1,l(i)}, \beta) f(h_{a,1,l(i)} \big| 2\alpha_{a,1,l(i)}, \beta)$$

In the next step, the effect of conditioning on the sum of heights $\chi_{l(i)}$ is removed. Because the sum of the height is known, the contribution of the heights is only made through their contribution to the sum. So the PDF is replaced with:

$$f(h_{s,1,l(i)}, h_{a,1,l(i)} \big| g_{l(i)} = \left\{ a_{1,l(i)}, a_{a,l(i)} \right\}, \chi_{l(i)}, \delta$$

$$= f_{Dir}(\pi_{s,1,l(i)}, \pi_{a,1,l(i)} \big| 2\alpha_{s,1,l(i)}, 2\alpha_{a,1,l(i)})$$

where $\pi_{s,1,l(i)} = h_{s,1,l(i)}|g_{l(i)}$ and $\pi_{a,1,l(i)} = h_{a,1,l(i)}|g_{l(i)}$ and $f_{Dir}$ is a Dirichlet pdf.

### 6.3.3.4 - Example - Two alleles - non-adjacent positions

In this case, the donor is heterozygous and their alleles for this locus are not in adjacent positions. For instance, the alleles might be 16, 18.
In this case, four $\alpha$'s are deployed, with those being $\alpha_{s,1,l(i)}$, $\alpha_{a,1,l(i)}$, $\alpha_{s,2,l(i)}$ and $\alpha_{a,2,l(i)}$. The probability density for $c_{l(i)}$ is given by multiplying four Gamma pdfs. The $\alpha$ parameters are given by $\alpha_{s,1,l(i)}, \alpha_{a,1,l(i)}, \alpha_{s,2,l(i)}$ and $\alpha_{a,2,l(i)}$, with a single $\beta$. Thereby giving the expression:

$$f(h_{s,1,l(i)}, h_{a,1,l(i)} h_{s,2,l(i)}, h_{a,2,l(i)} \big| g_{l(i)} = \left\{ a_{1,l(i)}, a_{2,l(i)} \right\}, \chi_{l(i)}, \delta)$$

which can be expressed as:

$$f(h_{s,1,l(i)}, \big| \alpha_{s,1,l(i)}, \beta) f(h_{a,1,l(i)}, \big| \alpha_{a,1,l(i)}, \beta) f(h_{s,2,l(i)}, \big| \alpha_{s,2,l(i)}, \beta) f(h_{a,2,l(i)}, \big| \alpha_{a,2,l(i)}, \beta)$$

Once again, the conditioning on $\chi_{l(i)}$ is removed, to obtain:

$$= f_{Dir}(\pi_{s,1,l(i)}, \pi_{a,1,l(i)} \pi_{s,2,l(i)}, \pi_{a,2,l(i)} \big| \alpha_{s,1,l(i)}, \alpha_{a,1,l(i)} \alpha_{s,2,l(i)}, \alpha_{a,2,l(i)})$$

where the $\pi$'s are the ratios of their respective $h$'s divided by $\chi_{l(i)}$.

### 6.3.3.5 - Example - Two alleles - adjacent positions

In this case, the donor is heterozygous and their alleles for this locus are in adjacent positions. For instance, the alleles might be 16, 17. Because of their positions, the stutter for allele 2 is in the same position as allele 1.
In this case four $\alpha$'s are deployed, with those being $\alpha_{s,1,l(i)}$, $\alpha_{a,1,l(i)}$, $\alpha_{s,2,l(i)}$ and $\alpha_{a,2,l(i)}$. The probability density for $c_{l(i)} = \{h_{s,1,l(i)}, h_{a,1,l(i)}, h_{a,2,l(i)}\}$ is given by multiplying the Gamma pdf's having $\alpha$ parameters given by $\alpha_{s,1,l(i)}, \alpha_{a,1,l(i)} +$

$\alpha_{s,2,l(i)}$ and $\alpha_{a,2,l(i)}$, with a single $\beta$.

Taking the approach outlined above, and after having accounted for the conditioning on $\chi_{l(i)}$ the expression gives:

$$f\left(h_{s,1,l(i)},h_{a,1,l(i)},h_{a,2,l(i)}\Big|g_{l(i)}=\left\{a_{1,l(i)},a_{2,l(i)}\right\},\chi_{l(i)},\delta\right)$$

and hence:

$$=f_{Dir}\left(\pi_{s,1,l(i)},\pi_{a,1,l(i)},\pi_{a,2,l(i)}\Big|\alpha_{s,1,l(i)},\alpha_{a,1,l(i)}+\alpha_{s,2,l(i)},\alpha_{a,2,l(i)}\right).$$

### 6.3.4 - Use of the Approach

Given a putative genotype, the peaks in the crime profile are either bigger or smaller than the reporting threshold $T_r$, or not present at all. We treat missing peaks and peaks smaller than $T_r$ as peak that has dropped out. We partition the crime profile for a given pair of genotype as:

$$c_{l(i)}=\left\{h:h\in c_{l(i)},h<T_r\right\}\cup\left\{h:h\in c_{l(i)},h\geq T_r\right\}$$

The resulting pdf is given by:

$$f(c_{l(i)}\big|g_{1,l(i)},g_{2,l(i)},\chi_{l(i)},\delta,\omega)=f\left(\pi\big|\alpha\right)x\prod_{\{h:h\in c_{l(i)},h<T_r\}}F(T\big|\alpha_h,\beta_h)$$

$f(\pi|\alpha)$ is a Dirichlet pdf with parameters:

$$\alpha=\cup\left\{\alpha_h:h\in c_{l(i)},h\geq T_r\right\}$$

and

$$\pi=\cup\left\{h\Big/\chi_{l(i)}^l:h\in c_{l(i)},h\geq T_r\right\}$$

where $a_h$ is the alpha parameter of the associated Gamma pdf in the corresponding position of height $h$.

$$*\,\chi_{l(i)}^l=\sum\nolimits_{h\in c_{l(i)},h\geq T_r}$$

$h$ is the sum of peak heights bigger than reporting threshold $T_r$. $F(T_r|\alpha_h,\beta_h)$ is the CDF of a Gamma distribution with parameters $\alpha_h$ and $\beta_h$ for the peak in the position of $h$ calculated as described above.

### 6.4 - Peak Balance Parameter Model

#### 6.4.1- Overview

As mentioned above, the approach uses a peak imbalance parameter/ effective amplified quanitity (EQE) parameter, $\delta$, in the form of a set of $\delta$'s, such that there is one for each of the alleles. Each of the peak imbalance parameters in the set can be used to adjust the means for the alleles.

The approach models degradation and other peak imbalance effects prior to any knowledge of the suspect's genotype. For each locus, the molecular weight of the peaks in the profile is associated with the sum of the heights. So as the molecular weight of the locus increase, a reduction in the sum of the peak heights is estimated.

### 6.4.2 - Details

Following this approach, for locus l(i), there are a set of peak heights: $h_{l(i)} = \{h_{j,l(i)} : j = 1,......n_{l(i)}\}$. Each height has an associated base pair count: $b_{l(i)} = \{b_{j,l(i)} : j = 1,.....n_{l(i)}\}$. An average base pair count is used as a measure of molecular weight for the locus, weighted by peak heights. This is defined as:

$$\bar{b}_{l(i)} = \frac{\sum_{j=1}^{n} b_{j,l(i)} h_{j,l(i)}}{\chi_{l(i)}}$$

where

$$\chi_{l(i)} = \sum_{j=1}^{n_{l(i)}} h_{j,l(i)}$$

and so the degradation model is defined as:

$$\chi_{l(i)} = d_1 + d_2 \bar{b}_{l(i)}$$

where $d_1$ and $d_2$ are the same for all loci.

The parameters $d_1$ and $d_2$ can be calculated using the least squared estimation. As some loci may behave differently to degradation etc, the sum of the peak heights for these loci are treated as outliers.

To deal with these outliers, a Jacknife method is used. There are $n_L$ loci with peak height and base pair information. Hence, the approach:

1. fits a regression model $n_L$ times, removing the $i^{th}$ value of the sets $\{\chi_{l(i)} : i = 1,.......n_L\}$ and $\{\bar{b}_{l(i)} : i = 1,....n_L\}$.

2. uses the regression model to produce a prediction interval, $\hat{\chi}_{l(i)} = I - 2\sigma$ where $\sigma$ is the standard deviation of the residuals in the fitted regression line.

3. when the sum of the peak height $\chi_i$, which is not used for the estimation of the regression line, does not lie within the prediction interval, then consider it as an outler.

4. removes any outliers from the data set and refits the model, after the $n_L$ models have been produced. The values of $d_1$ and $d_2$ are extracted from the model estimated without outliers.

If the degradation etc in the profile is negligible. Peak height variability may cause the estimated value of $d_2$ to be greater than zero. In such cases, $d_2$ is set as 0 and $d_1$ as 1.

In the deployment of the degradation model, at locus l(i) there is a crime profile with peaks having allele designations $\alpha_{j,l(i)}$ and base pair counts $b_{l(i)} = \{b_{j,l(i)} : j = 1,....n_{l(i)}\}$. If degradation were not being accounted for, then given the sum of the peak heights $\chi_{l(i)}$ it is possible to obtain a mean and a variance from a Gamma distribution.

When considering degradation, the same Gamma distribution is used, but the degradation model is used to adapt the Gamma pdf to account for the molecular weight of the allele.

As previously mentioned, peak heights increase with the sum of peak heights $\chi_{l(i)}$ and therefore the mean and variance also increase accordingly. If an allele is of high molecular weight, a reduction of $\chi_{l(i)}$ results in a reduction in the mean and variance. The degradation model reduced or increases the $\chi_{l(i)}$ associated with an allele according to degradation by using an appropriate $\delta$ for that allele.

The appropriate $\delta$'s are calculated as follows using the degradation model $\chi_{l(i)} = d_1 + d_2 \bar{b}_{l(i)}$.

The degradation parameter associated with alleles $\alpha_{j,l(i)}$ is defined as $\delta_{j,l(i)}$ so that the sum of peak heights associated with this allele are $\delta_{j,l(i)} \cdot \chi_{l(i)}$.

For each allele the model is used to estimate the associated peak height sum:

$$\hat{\chi}_{j,l(i)} = d_1 + d_2 b_{j,l(i)}$$

The calculations of $\delta$ are made such that the ratio of the estimated peak height sums are preserved; that is:

$$\frac{\chi_{j,I(i)}}{\hat{\chi}_{j+1,I(i)}} = \frac{\delta_{j,I(i)} \cdot \chi_{I(i)}}{\delta_{j+1,I(i)} \cdot \chi_{I(i)}}$$

To do this, a set of $n_{I(i)}$-1 equations with $n_{I(i)}$ unknowns, are provided:

$$\frac{\hat{\chi}_{j,I(i)}}{\hat{\chi}_{j+1,I(i)}} = \frac{\delta_{j,I(i)}}{\delta_{j+1,I(i)}}$$

The ratios on the left-hand side are obtained from the degradation model and the $\delta$'s are the unknown variables. A restriction is set, such that the average peak height sum in the locus remains the same after the application of the $\delta$'s, that is:

$$\frac{\sum_{j=1}^{n_{I(i)}} \delta_{j,I(i)} \cdot \chi_{I(i)}}{n_{I(i)}} = \chi_{I(i)}$$

which gives a further equation with the $\delta$'s as unknown quantities. This allows a solution to be found as there are $n_{I(i)}$ equations in the system and $n_{I(i)}$ unknowns.

The ratio of the estimated peak height sum is denoted:

$$r_{j,I(i)} = \frac{\hat{\chi}_{j,I(i)}}{\hat{\chi}_{j+1,I(i)}}, j = 1, 2 \ldots \ldots n_{I(i)} - 1$$

$$r_{j,I(i)} = 1, j = n_{I(i)}$$

The degradation parameters $\delta$'s, are then given by:

$$\delta_{j,I(i)} = \frac{n_{I(i)} \prod_{k=j}^{n_{I(i)}} r_{k,I(i)}}{\sum_{k=1}^{n_{I(i)}} \prod_{k}^{n_{I(i)}} r_{k,I(i)}}$$

The stutter associated with an allele, will have the same degradation parameter $\delta$ as the allele because the starting DNA molecule is the same in each case.

### 6.4.3 - Example

In Figure 23, a table is shown which details profile information, DNA quantity for the loci and the weighted base pair count for the degraded profile observed.

From the linear model of degradation provided above, $\chi_{I(i)} = d_1 + d_2 \bar{b}_{I(i)}$, this gives:

$$\chi = 3629.882 - 12.225b.$$

In Figure 24, a plot of weighted base pair against DNA quantitiy per locus is provided, with the linear model overlaid. Figure 25 shows in the table two examples of the degradation model as deployed.

## 6.5 - Multiple Source Samples

Using the same background information provided above, and a similar approach to that taken on samples from a single source, it is possible to extend the approach to multiple source samples.

### 6.5.1 Evidential Uses

Applying the approach provided above for the single source samples, the numerator can be stated as:

$$Num = f\left(c \big| g_{U1}, g_{U2s}, H_p\right) = \prod_{i=T}^{n_I} f(c_{I(i)} \big| g_{U1,I(i)}, g_{U2,I(i)}, H_P, \chi_{I(i)}, \delta)$$

and the denominator as:

$$Den = f\left(c \big| g_{U1}, g_{U2}, H_d\right) = \prod_{i=T}^{n_I} f(C_{I(i)} \big| g_{U1,I(i)}, g_{U2,I(i)}, H_d, \chi_{I(i)}, \delta)$$

In both instances, the core pdf is of the type previously identified, namely:

$$f\left(c_{I(i)} \big| g_{U,1,I(i)}, g_{U,2,I(i)}, \chi_{I(i)}, \delta\right)$$

### 6.5.2 Intelligence Uses

The task is to compute the posterior probability $p(g_{U,1,I(i)}, g_{U,2,I(i)} | C_{I(i)}, \chi_{I(i)}, \delta)$ of pair of genotypes given the peak heights in the profile. This probability is computed using Bayes theorem.

$$p(g^*_{U,1,I(i)}, g^*_{U,2,I(i)} \big| C_{I(i)}, \chi_{I(i)}, \delta) = \frac{f\left(c_{I(i)} \big| g^*_{U,1,I(i)}, g^*_{U,2,I(i)}, \chi_{I(i)}, \delta\right) p\left(g^*_{U,1,I(i)}, g^*_{U,2,I(i)}\right)}{\sum_{g_{U,1,I(i)}, g_{U,2,I(i)}} f\left(c_{I(i)} \big| g_{U,1,I(i)}, g_{U,2,I(i)}, \chi_{I(i)}, \delta\right) p\left(g_{U,1,I(i)}, g_{U,2,I(i)}\right)}$$

We assume that the prior probability for the pair of genotypes is the same for any genotype combination in the locus, therefore the formula above simplifies to:

$$p(g^*_{U,1,I(i)}, g^*_{U,2,I(i)} \big| C_{I(i)}, \chi_{I(i)}, \delta) = \frac{f\left(c_{I(i)} \big| g^*_{U,1,I(i)}, g^*_{U,2,I(i)}, \chi_{I(i)}, \delta\right)}{\sum_{g_{U,1,I(i)}, g_{U,2,I(i)}} f\left(c_{I(i)} \big| g_{U,1,I(i)}, g_{U,2,I(i)}, \chi_{I(i)}, \delta\right)}$$

The pdf for the peak heights given a pair of putative genotypes is calculated using the formula below:

$$f\left(c_{I(i)} \big| g_{U,1,I(i)}, g_{U,2,I(i)}, \chi_{I(i)}, \delta\right) = \sum_{\omega} f\left(c_{I(i)} \big| g_{U,1,I(i)}, g_{U,2,I(i)}, \chi_{I(i)}, \delta, \omega\right) p\left(\omega\right)$$

where $\omega$ is the mixing proportion.

Again the core pdf function $f(c_{I(i)} | g_{U,1,I(i)}, g_{U,2,I(i)}, \chi_{I(i)}, \delta)$ features.

As with the single source sample, not all pair of genotypes will have a non-zero probability. We therefore use the crime profile to guess pair of genotypes that may have zero probability. Peaks in the crime profile are designated as alleles or stutters. The genotypes are produced based on the peaks designated as alleles. We describe all cases below:

1. No peaks are bigger than the reporting threshold $T_r$. No genotypes are generated.
2. One peak bigger than $T_r$ is designated as allele, denoted by $a$. There are two possible genotypes {a, a} and a, Q} where $Q$ denoted any allele other than $a$. Any pairing of these genotypes is possible: ({a, a}, {a, a}), ({a, a}, {a, Q}) and ({a, Q}, {a, Q}).

3. Two peaks bigger than $T_r$ designated as alleles, denoted by $a$ and $b$. The possible genotypes are {a, a}, {a, b}, {a, Q}, {b, b}, {b, Q} and {Q, Q} where $Q$ is any allele other than $a$ and $b$. Any combination of pair of genotypes whose union contains $a, b$ is a possible pair of genotypes: {a, a} with any genotype that contains $b;$ {a, b} with any genotype in the list; {a, Q} with any genotype that contains $b;$ {b, b} with any genotype that contains $a;$ {b, Q} with any genotype that contains $a;$ and {Q, Q} with {a, b}.

4. Three peaks bigger than $T_r$, denoted by $a$, $b$ and $c$. This case follows exactly the same logic as in the case above. There are 10 genotypes that are possible from allele set: $a, b, c$ and $Q$, where $Q$ denotes any allele other than $a, b$ and $c$. All genotype pairs whose union contains $a, b, c$.

5. Four peaks bigger than $T_r$ are designated as alleles, denoted by $a, b, c$ and $d$. In this case there are 10 possible pair of genotypes. Any genotype pair whose union is $a, b, c, d$ is considered as a possible genotype pair.

In practice the interest lies on genotype pairs such that the first and second genotype corresponds to the major and minor contributor respectively. The calculation of the posterior probabilities in this section is done for all possible combinations of genotypes and mixing proportions. Moving from all combinations of genotypes to major minor requires folding the space of all combinations of genotypes and mixing proportions in two. To explain this point we need to introduce further notation:

$G_j$: a random variable for the genotype of contributor $j$, $j$ = 1,2;
$g_j$: specific instances of a genotype, $j$ = 1,2; $g_1$ and $g_2$ can be the same or different genotype.
$G_M$: a random variable for the genotype of the major contributor;
$G_m$: a random variable for the genotype of the minor contributor.

We are interested in the probabilities:

$$\Pr\left(G_M = g_1, G_m = g_2 \middle| c_{l(i)}\right) = \sum_{\omega \in \Omega \geq 0.5} \Pr\left(G_M = g_1, G_m = g_2 \middle| c_{l(i)}, \omega\right) p(\omega)$$

where $\Omega \geq 0.5$ is a discreet set of mixing proportions greater or equal to 0.5. When $\omega > 0.5$ the first factor in the summation in the above equation is:

$$\Pr\left(G_M = g_1, G_m = g_2 \middle| c_{l(i)}\omega\right) = \Pr\left(G_1 = g_1, G_2 = g_2 \middle| c_{l(i)}, \omega\right)$$

$$+ \Pr\left(G_1 = g_2, G_2 = g_1 \middle| c_{l(i)}, 1 - \omega\right)$$

$$= 2x\Pr\left(G_1 = g_1, G_{2m} = g_2 \middle| c_{l(i)}, \omega\right)$$

If $\omega$ = 0.5:

$$\Pr\left(G_M = g_1, G_m = g_2 \middle| c_{l(i)}\omega\right) = \Pr\left(G_1 = g_1, G_2 = g_2 \middle| c_{l(i)}, \omega\right).$$

### 6.5.3 - Consideration of Mixing proportion

In mixed source samples, the mixing proportion comes into play. On the basis that major and minor contributors are considered, then the values are:

$$\omega \in = \left(0.5, 0.6, \ldots \ldots 0.9\right).$$

In fact, the posterior probability of the mixing proportion given the peaks heights across all loci is used, expressed as:

$$f\left(c_{l(i)} \middle| g_{U,1,l(i)}, g_{U,2,l(i)}\right) = \sum_{\omega} f\left(c_{l(i)} \middle| g_{U,1,l(i)}, g_{U,2,l(i)}, \omega\right) p\left(\omega \middle| c_{l(1)}, c_{l(2)}, \ldots\ldots c_{l(nLoci)}\right)$$

The method for obtaining the posterior probability of the mixing proportion given peak heights, the second factor in the summation, is described in section 6.5.4. The method for computing pdf in the first factor of the summation is given in section 6.5.5.

### 6.5.4 - Mixing proportion posterior distribution

For each locus $l(i)$ we generate a set of possible genotype pairs of potential contributors of the crime profile $c_{l(i)}$. The $j$-th instance of the genotype of the contributor 1 and 2 are denoted by $g_{U1,j,l(i)}$ and $g_{U2,j,l(i)}$, respectively, where $n_g$ is the number of genotype pairs. We are interested in calculating the posterior probability of pair of genotypes given the peak heights in the crime profile $c_{l(i)}$. For this calculation we need a probability distribution for mixing proportion. In this section we describe a sequential method for calculating the posterior distribution of mixing proportion given peak heights across loci.

The mixing proportion is a continuous quantity in the interval $(0, 1)$. However, for practical purposed, we use a discrete probability distribution. Assume that we have mixing proportions $\omega = \{\omega_k : k = 1, \ldots, n_\omega\}$, where $n_\omega$ is the number of mixing proportions considered. We set a prior distribution for mixing proportion as uniform over the discrete values. Using Bayes theorem, the posterior distribution for mixing proportion given the peak heights in locus i is:

$$p(\omega_k \middle| c_{l(i)}, \chi_{l(i)}, \delta) = \frac{f\left(c_{l(1)} \middle| \chi_{l(1)}, \omega_k, \delta\right) p(\omega_k)}{\sum_{m=1}^{n_\omega} f\left(c_{l(1)} \middle| \chi_{l(1)}, \delta, \omega_m\right) p\left(\omega_m\right)}$$

The posterior distribution of mixing proportion for locus i, i = 2, 3, ...,$n_L$ is given by:

$$p(\omega_k \middle| c_{l(1)}, \ldots, c_{l(i)}, \chi_{l(1)}, \ldots\ldots\chi_{l(i)}, \delta) = \frac{f\left(c_{l(i)} \middle| \chi_{l(i)}, \omega_k, \delta\right) p(\omega_k) \middle\| c_{l(1)}, \ldots, c_{l(i-1)}, \chi_{l(1)}, \ldots\ldots\chi_{l(i-1)}, \delta}{\sum_{m=1}^{n_\omega} f\left(c_{l(i)} \middle| \chi_{l(i)}, \delta, \omega_m\right) p\left(\omega_m \middle| c_{l(1)}, \ldots, c_{l(i-1)}, \chi_{l(1)}, \ldots\ldots\chi_{l(i-1)}, \delta\right)}$$

where $f(\omega_k | c_{l(i)}, \chi_{l(i)})$, $i = 1, 2, \ldots\ldots n_L$ is defined in the following paragraph. If there are any loci with no information they are ignored in the calculation as having no information.

The probability density of the peak height in the crime profile $c_{l(i)}$ at locus $l(i)$ for a given mixing proportion $\omega_k$ is given by:

$$f(c_{l(i)} \middle| \chi_{l(i)}, \omega_k) = \sum_{j=1}^{n_g} f\left(c_{l(i)} \middle| g_{U1,j,l(i)}, g_{U2,j,l(i)}, \chi_{l(i)}, \omega_k\right) p\left(g_{U1,j,l(i)}, g_{U2,j,l(i)}\right)$$

where $p(g_{U1,j,l(i)}, g_{U2,j,l(i)})$ is the probability of the two genotypes prior to observing the crime profile. This is based on the

$$p\left(g_{U1,j,l(i)}, g_{U2,j,l(i)}\right) = \frac{1}{n_g}$$

assumption of an equal probability of all genotype pairs:

This $\frac{1}{n_g}$ will cancel out in the following equations and it is thus ignored. Then the probability density in the above equation then simplifies to:

$$f(c_{l(i)} \middle| \chi_{l(i)}, \omega_k) = \sum_{j=1}^{n_g} f\left(c_{l(i)} \middle| g_{U1,j,l(i)}, g_{U2,j,l(i)}, \chi_{l(i)}, \omega_k\right)$$

The pdf in the summation of the above is described in section 6.5.5.

The calculations in this method can be readily represented using Bayesian Networks. The starting point is the Bayesian Network in Figure 25a (with $\Omega$ as the mixing proportion). The effect of the above equation is represented in Figure 25b by erasing the nodes corresponding to the putative genotypes. The effect of using equation

$$p(\omega_k|c_{l(1)},....,c_{l(i)},\chi_{l(1)},.........\chi_{l(i)},\delta) = \frac{f\left(c_{l(i)}\middle|\chi_{l(i)},\omega_k,\delta\right)p(\omega_k)\middle\|c_{l(1)},....,c_{l(i-1)},\chi_{l(1)},.........\chi_{l(i-1)},\delta}{\sum_{m=1}^{n_\omega}f\left(c_{l(i)}\middle|\chi_{l(i)},\delta,\omega_m\right)p\left(\omega_m\middle|c_{l(1)},....,c_{l(i-1)},\chi_{l(1)},.........\chi_{l(i-1)},\delta\right)}$$

for locus $l(i)$ takes us from Figure 25b to Figure 25c. The effect of using the same equation for locus $l(j)$ takes the position from Figure 25c to Figure 25d.

### 6.5.5 - Probability density function $f(c_{l(i)}|g_{1,l(i)},g_{2,l(i)},\omega,\chi_{l(i)},\delta)$

*6.5.5.1 - Overview*

As identified above, in evidential and intelligence uses involving mixed source samples, the pdf $f(c_{l(i)}|g_{1,l(i)},g_{2,l(i)},\omega,\chi_{l(i)},\delta)$ is of great importance.

In this section we describe the construction and use of the pdf for a crime profile given two putative donors. We use a running example to illustrate the method. The example is for locus D2. The crime profile and putative donors used in the example are given in Table 1.

Table 1

| allele | height | donor 1 | donor 2 | base pair count |
|--------|--------|---------|---------|-----------------|
| 16 | 124 | | | 293 |
| 17 | 2243 | • | | 297 |
| 21 | 0 | | | 313 |
| 22 | 271 | | • | 317 |
| 23 | 169 | | | 321 |
| 24 | 2044 | • | • | 325 |

*6.5.5.2 - Construction*

This pdf is closely related in principles and approach to that detailed for singl source samples and again is constructed in seven steps:

**Step 1** The associated peak-height sum for donor 1 is $\omega \times \chi_{l(i)}$. Let's denote the corresponding means for the peak height of the alleles and the stutters of this donor by $\mu_{a,1,l(i)}$ and $\mu_{s,1,l(i)}$, respectively. They are obtained as a function of $\chi_{D2}$ using the method described in section 6.2.7.3. For each allele $a$ of donor 1, we assign the allele mean $\mu_{a,1,l(i)}$ to the position of allele $a$, and the stutter mean $\mu_{s,1,l(i)}$ to position $a - 1$. If the donor is homozygote, we do the assignment twice.

In the example, the sum of peak height above the limit of detection of 30 rfu is $\chi_{D2} = 4851$ and the mixing proportion is $\omega = 0.9$. The associated sum of peak heights for donor 1 is $\omega \times \chi_{D2} = 4365.9$. The sum of peak heights are in fact across all loci and therefore to obtain the means for alleles and stutters we multiply by 10. The expected mean height for stutters and alleles are given in table 2.

Table 2

| Mean | Value |
|---|---|
| $\mu_{a,1,D2}$ | 1501.87 |
| $\mu_{s,1,D2}$ | 100.42 |

**Step 2** The associated peak-height sum for donor 2 is $(1 - \omega) \times \chi_{l(i)}$ with associated mean for allele and stutters: $\mu_{a,2,l(i)}$ and $\mu_{s,2,l(i)}$. The assignment of means is done as in step 1.

The associated peak height sum for donor 2 is $(1 - \omega) \times \chi_{D2} = 485.1$. The means for alleles and stutter are given in Table 3.

Table 3

| Mean | Value |
|---|---|
| $\mu_{a,2,D2}$ | 166.87 |
| $\mu_{s,2,D2}$ | 11.16 |

**Step 3** If a donor is a heterozygote, the means are modified to take into account factors, such as PCR efficiency and degradation, that affect the resulting peak heights. For example, if donor 1 is a heterozygote in locus $l(i)$, the mean for his/her alleles and stutters are: $\delta_1 \times \mu_{a,1,l(i)}$ and $\delta_1 \times \mu_{s,1,l(i)}$ for the low-molecular-weight

allele and $\delta_2$ x $\mu_{a,1,i(t)}$ and $\delta_2$ $\mu_{s,1,i(t)}$ for the high-molecular-weight allele. We give a detailed description of the method for calculating $\delta_1$ and $\delta_2$ elsewhere.

In the example, both donors are heterozygotes. The EAQ factors for both donors are given in the Table 4. Notice that the EAQ factors for donor 1 are more separated than the EAQ factors of donor 2. This is due to the greater separation in base pairs between the alleles of donor 1 and the alleles of donor 2. The adjusted means for alleles and stutters are also given in Table 4.

| Allele | | Donor 1 | | | Donor 2 | | | Base pair count |
|---|---|---|---|---|---|---|---|---|
| | Detected? | EAQ factor | means | Detected? | EAQ factor | means | count | |
| 16 | | $\delta1=1.027$ | 103.13 | | | | | 293 |
| 17 | X | $\delta1=1.027$ | 1542.42 | | | | | 297 |
| 21 | | | | | $\delta1=1.082$ | 12.07 | 313 | |
| 22 | | | | X | $\delta1=1.082$ | 180.56 | 317 | |
| 23 | | $\delta2=0.972$ | 97.60 | | $\delta2=0.918$ | 10.24 | 321 | |
| 24 | X | $\delta2=0.972$ | 1459.2 | X | $\delta2=0.918$ | 153.19 | 325 | |

**Step 4**  The variances for each allele and stutter are obtained as a function of means using the method described above. In later step we need to add random Gamma variables. A condition for a close form calculation is that the $\beta$-parameters are the same. Also in a later step, we divide each Gamma by the overall sum of peak height to account for using the sum of peak heights in this locus. A closed form calculation can be done if all $\beta$ parameters are the same. The conditioned on the $\beta$-parameters can be obtained by estimating a line between the points formed by the means, in the x-axis, and the variances, in the y-axis. A regression line with zero intercept is fitted to obtain:

$$\overset{\wedge}{\sigma}^2 = \kappa_2 x \mu$$

So, if peak i has mean and variance $\left(\mu_i, \sigma_i^2\right)$,

$$\beta = \mu_i / \sigma_i^2 = \mu_i / \left(\kappa_2 x \mu_i\right) = 1/\kappa_2$$

regardless of the value of $\mu_i$. In this example $k_2 = 118.2$. Table 5 shows the standard deviations computed from the data and with the linear relationship between the means and variances.

**Step 5**  The shape $(\alpha)$ and rate $(\beta)$ parameters are obtained from the mean $(\mu)$ and the variances $(\overset{\wedge}{\sigma}^2)$ using the known formulae $\alpha = (\mu / \overset{\wedge}{\sigma}^2)^2$ and $\beta = \mu / \overset{\wedge}{\sigma}^2$.

**Step 6**  The alpha parameters for alleles and stutters in the same allele position are added to obtain an overall $\alpha$ for that position. Now we have the parameters of a Gamma distribution for each position.

| Allele | | Donor 1 | | | | Donor 2 | | |
|---|---|---|---|---|---|---|---|---|
| | | $(\mu)$ | $(\sigma)$ | $(\sigma^2)$ | | $(\mu)$ | $(\sigma)$ | $(\sigma^2)$ |
| 16 | | 103.13 | 45.17 | 110.41 | | | | |
| 17 | X | 1542.42 | 435.38 | 426.98 | | | | |

| 21 | | | | | | 12.07 | 3.56 | 37.77 |
|---|---|---|---|---|---|---|---|---|
| 22 | | | | | X | 180.56 | 66.44 | 146.10 |
| 23 | | 97.60 | 43.57 | 107.41 | | 10.24 | 12.06 | 35.29 |
| 24 | X | 1459.2 | 412.06 | 415.39 | X | 153.19 | 59.10 | 134.56 |

Table 5: Estimated means (μ) and standard deviations (σ) and the standard deviations $\hat{\mu}$ obtained from the linear relationship between the means (μ) and the variances (σ²)

**Step 7** To account for using the sum of peak height in the locus, the collection of Gamma pdf's whose peak heights are above the peak-height reporting limit are converted to a Dirichlet pdf. This is achieved in closed form because all β's are the same. The resulting Dirichlet pdf inherit the α parameters of the Gammas.

### 6.5.6 - Use of the Approach

Given a pair of putative genotypes, the peaks in the crime profile are either bigger or smaller than the reporting threshold $T_r$, or not present at all. We treat missing peaks and peaks smaller than $T_r$ as peak that has dropped out. We write the crime profile for a given pair of genotype as:

$$c_{l(i)} = \left\{h : h \in c_{l(i)}, h < T_r\right\} \cup \left\{h : h \in c_{l(i)}, h \geq T_r\right\}$$

The resulting pdf is given by:

$$f\left(c_{l(i)} \mid g_{1,l(i)}, g_{2,l(i)}, \chi_{l(i)}, \delta, \omega\right) = f\left(\pi \mid \alpha\right) x \prod_{\{h:h \in c_{l(i)}, h < T_r\}} F\left(T \mid \alpha_h, \beta_h\right)$$

The terms are explained below:
$f(\pi|\alpha)$ is a Dirichlet pdf with parameters

$$\alpha = \cup \left\{\alpha_h : h \in c_{l(i)}, h \geq T_r\right\}$$

and

$$\pi = \cup \left\{h / \chi'_{l(i)} : h \in c_{l(i)}, h \geq T_r\right\}$$

where $a_h$ is the alpha parameter of the associated Gamma pdf in the corresponding position of height $h$.

$$\chi'_{l(i)} = \sum_{h \in c_{l(i)}, h \geq T_r} J$$

$h$ is the sum of peak heights bigger than reporting threshold $T_r$. $F(T_r|\alpha_h,\beta_h)$ is the CDF of a Gamma distribution with parameters $\alpha_h$ and $\beta_h$ for the peak in the position of $h$
calculated as described above.

**6.7 - Peak Imbalance Parameter Model - Mixed Source Samples**

**6.7.1 - Overview**

The peak imbalance parameter or effective amplified quantity (EAQ) is manifested in a crime profile through a reduction of peak heights for high molecular weight alleles. In this section a model for quantifying EAQ is described. Methods for estimating EAQ parameters and deploying them for a profile in a locus are also given.

We model EAQ prior to any knowledge of the suspect's genotype. For each locus we associate molecular weight of the peaks in the profiles with the sum of heights. As the molecular weight of the locus increases we estimate the reduction in the sum of peak heights.

**6.7.2 - Details**

Assume that in locus $l(i)$ we have the set of peak heights $h_{l(i)} = \{h_{j,l(i)} : j = 1,...,n_{l(i)}\}$. Each height has an associated base pair count $b_{l(i)} = \{b_{j,l(i)} : j = 1,...,n_{l(i)}\}$. An average base pair count, weighted by peak heights, is used as a measure of molecular weight for the locus. More specifically, this is defined as:

$$\overline{b}_{l(i)} = \frac{\sum_{j=1}^{n} b_{j,l(i)} h_{j,l(i)}}{\chi_{l(i)}}$$

where

$$\chi_{l(i)} = \sum_{j=1}^{n_{l(i)}} h_{j,l(i)}$$

We define the EAQ model as:

$$\chi_{l(i)} = d_1 + d_2 \overline{b}_{l(i)}$$

where $d_1$ and $d_2$ are the same for all loci.

The sum of peak heights $\chi_{l(i)}$ is assumed to be a linear function of the weighted base-pair average,

$$\overline{b}_{l(i)} = \frac{\sum_{j=1}^{n} b_{j,l(i)} h_{j,l(i)}}{\chi_{l(i)}}.$$

**6.7.3 - Estimation of Peak Imbalance Parameters $d_1$ and $d_2$**

The parameters $d_1$ and $d_2$ are calculated using least squared estimation. However some loci may behave differently, and therefore the sum of peak heights of these loci can be treated as outlier. We use a Jackknife method to deal with this problem. There are $n_L$ loci with peak height and base pair information.

1. We fit the regression model $n_L$ times, removing the $i^{th}$ value of the sets
$\{\chi_{l(i)} : i = 1,...n_L\}$ and $\{\overline{b}_{l(i)} : i = 1,...n_L\}$.

2. We use least-squares estimation to produce a prediction interval, $\hat{\chi}_{l(i)} \pm 2\sigma$, where $\sigma$ is the standard deviation of the residuals in the fitted regression line.

3. If the sum of peak height $\chi_i$ which was not used for the estimation of the regression line, does not lie within the prediction interval then we consider it an outlier.

4. After the $n_L$ models have been produced we remove any outliers from the dataset and re-fit the model.

The values of $d_1$ and $d_2$ are extracted from the model estimated without outliers.

If the degradation in the profile is negligible, peak height variability may cause the estimated value of $d_2$ to be greater than zero. In this case we set $d_2 = 0$ and $d_1 = 1$.

**6.7.4 - Deploying the peak Imbalance Parameter Model**

The peak imbalance parameter or EAQ model is used for taking into account EAQ within a locus. EAQ between loci is taken account by conditioning on the sum of peak height per locus. The EAQ model is used when the pdf of the peak heights for single and two-person profiles is deployed. More specifically, it is deployed for each heterozygote donor. In this section we describe the calculation of the EAQ factors $\delta_1$ and $\delta_2$ to be used for deploying pdf for peak heights.

Assume that at locus $l(i)$ we have a putative heterozygote donor with alleles $a_1, l(i)$ and $a_2, l(i)$ with corresponding molecular weights in base pairs $b_1, l(i)$ and $a_2, l(i)$, respectively. If we were not considering EAQ, given the sum of peak heights $\chi_{l(i)}$ for this locus we can obtain a mean $\mu_{l(i)}$ and variance $\sigma^2_{l(i)}$ of a Gamma distribution that models the behaviour of a peak height. In other words, if $H_{j,l(i)}$ denotes the random variable for the height corresponding the allele $s_{j,l(i)}$, then:

$$H_{j,l(i)} \Box \, \Gamma\left(\mu_{l(i)}, \sigma^2_{l(i)} \middle| \chi_{l(i)}\right), j = 1, 2.$$

The same Gamma pdf is used for any allele in the locus. The EAQ model issued to adapt the Gamma pdf by taking into account the molecular weight of the allele. The EAQ model is used to calculate a pair of factors $\delta_1$ and $\delta_2$ so that the mean values of the Gamma distribution are adjusted accordingly. The new mean is given by:

$$\mu_{j,l(i)} = \delta \, x \mu_{l(i)}, j = 1, 2.$$

In the rest of the section we describe a method for calculating $\delta_1$ and $\delta_2$ using the slope $d_2$ of the EAQ regression line. The first condition that the $\delta$'s must fulfill is that the slope of a line going through the coordinates $(b_{1,l(i)}, \mu_{1,l(i)})$ and $(b_{2,l(i)}, \mu_{2,l(i)})$ is the same as the slope $d_2$ of the EAQ regression line, i.e.:

$$\frac{\mu_{1,l(i)} - \mu_{2,l(i)}}{b_{1,l(i)} - b_{2,l(i)}} = d_2$$

The second condition that the $\delta$'s must fulfilled is the preservation of the mean $\mu_{l(i)}$:

$$\frac{\mu_{1,l(i)} - \mu_{2,l(i)}}{2} = \mu_{l(i)}$$

Substituting $\mu_{j,l(i)} = \delta x \mu_{l(i)}$, $j = 1, 2$. in $\dfrac{\mu_{1,l(i)} - \mu_{2,l(i)}}{b_{1,l(i)} - b_{2,l(i)}} = d_2$ and $\dfrac{\mu_{1,l(i)} - \mu_{2,l(i)}}{2} = \mu_{l(i)}$ we obtain two equations with two unknowns $\delta_1$ and $\delta_2$. The solution of the equations is

$$\delta_1 = \frac{d_2\left(b_{1,l(i)} - b_{2,l(i)} + 2\mu_{l(i)}\right)}{2\mu_{l(i)}}$$

$$\delta_2 = 2 - \delta_1$$

The stutter associated with the allelic peak will have the same degradation factor because it is the starting DNA molecules of the allele that is affected by degradation.

**6.7.5 - Other Information**

1. In the present version of the model we are not using the peak heights of Amelo for calculating the EAQ line, however, it is deployed for this locus.
2. The maximum value that $\delta_1$ can take in 1.9 because if $\delta_1 \geq 2$, $\delta_2 = 2 - \delta_1$ is not positive.

**Claims**

1. A computer-implemented method comprising:

comparing, using a computer, a first sample result set with a second sample result set to automatically identify a match therebetween, the first sample result set comprising the identities of alleles present at one or more loci in the DNA of a first sample, the second sample result set comprising the identities of alleles present at the same respective loci in the DNA of a second sample, the first and second samples each obtained from one or more donors; and

outputting, by the computer, an automatically identified match between the first and second sample result sets to provide an indication of a potential match between at least one donor of the first sample and at least one donor of the second sample,

wherein comparing the first and second sample result sets involves consideration of a likelihood ratio which provides an indication of the probability of the alleles being present at their respective loci in the DNA, the likelihood ratio determined using one of the following probability density functions:

$f(C_{l(i)}|g_{l(i)}, \chi_{l(i)}, \delta)$ for single donor samples where $g_{l(i)}$ is the genotype of the donor of sample $C_{l(i)}$, $\delta$ is an effect parameter accounting for variations in the allele identities caused by a method of analysing the sample to establish the allele identities and/or degradation of the sample, and $\chi_{l(i)}$ denotes the sum of peak magnitudes for the locus $i$; or

$f(c_{l(i)}|g_{U,1,l(i)}, g_{U,2,l(i)}, \chi_{l(i)}, \delta)$ for multiple donor samples where $g_{U,1,l(i)}$ is one of the genotypes of the donor of sample $c_{l(i)}$, $g_{U,2,l(i)}$ is another of the genotypes of the donor of the sample $c_{l(i)}$, $\delta$ is an effect parameter accounting for variations in the allele identities caused by a method of analysing the sample to establish the allele identities and/or degradation of the sample, and $\chi_{l(i)}$ denotes the sum of peak magnitudes for the locus i; or

$f(c_{l(i)}|g_{1,l(i)}, g_{2,l(i)}, \omega, \chi_{l(i)}, \delta)$ for mixed donor samples where $g_{1,l(i)}$ is one of the genotypes of the donor of sample $c_{l(i)}$, $g_{2,l(i)}$ is another of the genotypes of the donor of the sample $C_{l(i)}$, $\delta$ is an effect parameter accounting for variations in the allele identities caused by a method of analysing the sample to establish the allele identities and/or degradation of the sample, $\chi_{l(i)}$ denotes the sum of peak magnitudes for the locus $i$, and $\omega$ is the proportion of the DNA associated with the donor.

2. A method according to claim 1 wherein the effect parameter accounts for variations in the allele identities associated with one or more of stutter, allele dropout and one of more effects which impact upon the amount of an allele.

3. A method according to claim 2 wherein the one or more effects comprise at least one of degradation effects, variations in amplification efficiency and variations in the amount of allele present in a sub-sample of a sample.

4. A method according to any preceding claim wherein the method comprises analysing, using a computer, one or both of the first and second samples to establish the allele identities present at the one or more loci in the DNA of the respective sample.

5. A method according to claim 4 wherein the analysis comprises one or more of: a sampling step; a dilution step; a purification step; a pooling of samples step; a division of samples step; an amplification step; a detection step; an electrophoresis step; an interpretation step; a peak identification step; and a peak magnitude determination step.

6. A method according to any preceding claim wherein the peak magnitudes comprise at least one of peak heights, peak areas and peak weights present for one or more peaks indicative of the allele identities.

**Patentansprüche**

1. Computerimplementiertes Verfahren, umfassend:

   Vergleichen eines ersten Probenergebnissatzes mit einem zweiten Probenergebnissatz unter Verwendung eines Computers, um automatisch eine Übereinstimmung zwischen diesen zu identifizieren, wobei der erste Probenergebnissatz die Identitäten von Allelen umfasst, die an einem oder mehreren Loci in der DNA einer ersten Probe vorhanden sind, wobei der zweite Probenergebnissatz die Identitäten von Allelen umfasst, die an denselben jeweiligen Loci in der DNA einer zweiten Probe vorhanden sind, wobei die erste und die zweite Probe jeweils von einem oder mehreren Spendern erhalten wurden; und
   Ausgeben einer automatisch identifizierten Übereinstimmung zwischen dem ersten und dem zweiten Probenergebnissatz durch den Computer, um einen Hinweis auf eine mögliche Übereinstimmung zwischen mindestens einem Spender der ersten Probe und mindestens einem Spender der zweiten Probe zu liefern,
   wobei das Vergleichen des ersten und des zweiten Probenergebnissatzes die Berücksichtigung eines Wahrscheinlichkeitsverhältnisses beinhaltet, das einen Hinweis auf die Wahrscheinlichkeit liefert, dass die Allele an ihren jeweiligen Loci in der DNA vorhanden sind, wobei das Wahrscheinlichkeitsverhältnis unter Verwendung einer der folgenden Wahrscheinlichkeitsdichtefunktionen bestimmt wird:

   $f(C_{l(i)}|g_{l(i)}, \chi_{l(i)}, \delta)$ für Einzelspenderproben, wobei $g_{l(i)}$ der Genotyp des Spenders der Probe $C_{l(i)}$ ist, $\delta$ ein Effektparameter ist, der Variationen in den Allelidentitäten berücksichtigt, verursacht durch ein Verfahren zum Analysieren der Probe, um die Allelidentitäten und/oder den Abbau der Probe festzustellen, und $\chi_{l(i)}$ die Summe der Peakgrößen für den Locus $i$ bezeichnet; oder
   $f(c_{l(i)}|g_{U,1,l(i)}, g_{U,2,l(i)}, \chi_{l(i)}, \delta)$ für Proben von mehreren Spendern, wobei $g_{U,1,l(i)}$ einer der Genotypen des Spenders der Probe $c_{l(i)}$ ist, $g_{U,2,l(i)}$ ein anderer der Genotypen des Spenders der Probe $c_{l(i)}$ ist, $\delta$ ein Effektparameter ist, der Variationen in den Allelidentitäten berücksichtigt, verursacht durch ein Verfahren zum Analysieren der Probe, um die Allelidentitäten und/oder den Abbau der Probe festzustellen, und $\chi_{l(i)}$ die Summe der Peakgrößen für den Locus i bezeichnet; oder
   $f(c_{l(i)}|g_{1,l(i)}, g_{2,l(i)}, \omega, x_{l(i)}, \delta)$ für Proben von gemischten Spendern, wobei $g_{1,l(i)}$ einer der Genotypen des Spenders der Probe $c_{l(i)}$ ist, $g_{2,l(i)}$ ein anderer der Genotypen des Spenders der Probe $C_{l(i)}$ ist, $\delta$ ein Effektparameter ist, der Variationen in den Allelidentitäten berücksichtigt, verursacht durch ein Verfahren zum Analysieren der Probe, um die Allelidentitäten und/oder den Abbau der Probe festzustellen, $\chi_{l(i)}$ die Summe der Peakgrößen für den Locus $i$ bezeichnet; und $\omega$ der Anteil der mit dem Spender assoziierten DNA ist.

2. Verfahren nach Anspruch 1, wobei der Effektparameter Variationen in den Allelidentitäten berücksichtigt, die mit Stutter, Allelausfall und/oder einem oder mehreren Effekten verbunden sind, die sich auf die Menge eines Allels auswirken.

3. Verfahren nach Anspruch 2, wobei der eine oder die mehreren Effekte mindestens eines von Abbaueffekten, Variationen in der Amplifikationseffizienz und Variationen in der Menge des in einer Unterprobe einer Probe vorhandenen Allels umfassen.

4. Verfahren nach einem vorhergehenden Anspruch, wobei das Verfahren das Analysieren einer oder beider der ersten und der zweiten Probe unter Verwendung eines Computers umfasst, um die an einem oder mehreren Loci in der DNA der jeweiligen Probe vorhandenen Allelidentitäten festzustellen.

5. Verfahren nach Anspruch 4, wobei die Analyse eines oder mehrere umfasst aus: einem Abtastschritt; einem Verdünnungsschritt; einem Reinigungsschritt; einem Probenpoolschritt; einem Probenaufteilungsschritt; einem Amplifikationsschritt; einem Nachweisschritt; einem Elektrophoreseschritt; einem Interpretationsschritt; einem Peakidentifikationsschritt; und einem Peakgrößenbestimmungsschritt.

6. Verfahren nach einem vorhergehenden Anspruch, wobei die Peakgrößen mindestens eines von Peakhöhen, Peakflächen und Peakgewichtungen umfassen, die für einen oder mehrere Peaks vorliegen, die die Allelidentitäten anzeigen.

**Revendications**

1. Procédé mis en œuvre par ordinateur, consistant à :

comparer, à l'aide d'un ordinateur, un premier ensemble de résultats d'échantillon avec un second ensemble de résultats d'échantillon afin d'identifier automatiquement une correspondance entre eux, le premier ensemble de résultats d'échantillon comprenant les identités des allèles présents à un ou plusieurs loci dans l'ADN d'un premier échantillon, le second ensemble de résultats d'échantillons comprenant les identités des allèles présents aux mêmes loci respectifs dans l'ADN d'un second échantillon, les premier et second échantillons étant chacun obtenus à partir d'un ou plusieurs donneurs ; et à

délivrer, par l'ordinateur, une correspondance automatiquement identifiée entre les premier et second ensembles de résultats d'échantillon afin de fournir une indication d'une correspondance potentielle entre au moins un donneur du premier échantillon et au moins un donneur du second échantillon,

la comparaison des premier et second ensembles de résultats d'échantillon impliquant la considération d'un rapport de vraisemblance qui fournit une indication de la probabilité que les allèles sont présents à leurs locus respectifs dans l'ADN, le rapport de vraisemblance étant déterminé en utilisant l'une des fonctions de densité de probabilité suivantes :

$f(C_{l(i)}|g_{l(i)}, x_{l(i)}, \delta)$ pour les échantillons à donneur unique où $g_{l(i)}$ est le génotype du donneur de l'échantillon $C_{l(i)}$, $\delta$ est un effet paramètre tenant compte des variations des identités d'allèles provoquées par une méthode d'analyse de l'échantillon pour établir les identités d'allèles et/ou la dégradation de l'échantillon, et $x_{l(i)}$ désigne la somme des amplitudes de pic maximales pour le locus $i$ ; ou

$f(c_{l(i)}|g_{U,1,l(i)}, g_{U,2,l(i)}, x_{l(i)}, \delta)$ pour les échantillons à donneurs multiples où $g_{U,1,l(i)}$ est l'un des génotypes du donneur de l'échantillon $c_{l(i)}$, $g_{U,2,l(i)}$ est un autre des génotypes du donneur de l'échantillon $c_{l(i)}$, $\delta$ est un paramètre d'effet tenant compte des variations des identités d'allèles provoquées par une méthode d'analyse de l'échantillon pour établir les identités d'allèles et/ou la dégradation de l'échantillon, et $x_{l(i)}$ désigne la somme des amplitudes de pic maximales pour le locus $i$ ; ou

$f(c_{l(i)}|g_{1,l(i)}, g_{2,l(i)}, \omega, x_{l(i)}, \delta)$ pour les échantillons de donneurs mixtes où $g_{1,l(i)}$ est l'un des génotypes du donneur de l'échantillon $c_{l(i)}$, $g_{2,l(i)}$ est un autre des génotypes du donneur de l'échantillon $C_{l(i)}$, $\delta$ est un paramètre d'effet tenant compte des variations des identités alléliques causées par une méthode d'analyse de l'échantillon pour établir les identités alléliques et/ou la dégradation de l'échantillon, $x_{l(i)}$ désigne la somme des amplitudes de pic maximales pour le locus $i$, et $\omega$ est la proportion de l'ADN associée au donneur.

2. Procédé selon la revendication 1, dans lequel le paramètre d'effet tient compte des variations des identités d'allèles associées à au moins l'un parmi les bégaiements, l'abandon d'allèles et à au moins un effet qui a un impact sur la quantité d'allèles.

3. Procédé selon la revendication 2, dans lequel le ou les effets comprennent au moins l'un des effets de dégradation, des variations d'efficacité d'amplification et des variations de la quantité d'allèle présent dans un sous-échantillon d'échantillon.

4. Procédé selon l'une quelconque des revendications précédentes, dans lequel le procédé comprend l'analyse, à l'aide d'un ordinateur, d'un ou des deux des premier et second échantillons afin d'établir les identités d'allèles présentes au niveau d'un ou plusieurs loci dans l'ADN de l'échantillon respectif.

5. Procédé selon la revendication 4, dans lequel l'analyse comprend une ou plusieurs parmi : une étape d'échantillonnage ; une étape de dilution ; une étape de purification ; une étape de regroupement d'échantillons ; une étape de division des échantillons ; une étape d'amplification ; une étape de détection ; une étape d'électrophorèse ; une étape d'interprétation ; une étape d'identification de pic ; et une étape de détermination de l'amplitude de pic.

6. Procédé selon l'une quelconque des revendications précédentes, dans lequel les amplitudes de pic comprennent au moins l'un parmi des hauteurs de pic, des zones de pic et des poids de pic présents pour au moins un pic indicatif des identités d'allèles.

FIG. 1

FIG. 2a

FIG. 2b

First Case                    Second Case

FIG.    2c

Homozygote heights as a function of DNA quantity

FIG.    2d

Alpha parameters of a Beta PDF

Beta parameters of a Beta PDF

(a)

(b)

FIG.    2e

EP 2 545 480 B1

FIG.    3a

FIG.    3b

FIG.    3c

Conditional PDF's of M given X

Sigma as a function of mean height

FIG.    3e

FIG.    3f

EP 2 545 480 B1

FIG.   4a

FIG.   4b

FIG. 4c

FIG. 5

FIG.   6

FIG. 7

X = DNA quantity; $M$ = Mean height

$$M \mid X = \chi \sim Gamma\left(\alpha(\chi), \beta(\chi)\right)$$

Profile mean vs profile standard deviation

$$\sigma_M \approx 0.36\chi$$

FIG.  8a

X = DNA quantity; $M$ = Mean height

$$M \mid X = \chi \sim Gamma\left(\alpha(\chi), \beta(\chi)\right)$$

Mean height given DNA quantity

$$\mu_M \approx 0.8\chi$$

FIG.  8b

Log(M) v. Log(R)

FIG. 9

FIG.    10

FIG.    11a

FIG. 11c

$\mu = k_2 X \sigma^2$

FIG. 11b

(a)

mean height of allele as a function
of the sum of peak height per profile

(b)

mean height of stutter as a function
of the sum of peak height per profile

(c)

variance of allele heights
as a function of allele height means

(d)

coefficient of variation
as a function of mean

FIG.    12

(a)

mean height of allele as a function
of the sum of peak height per profile

(b)

mean height of stutter as a function
of the sum of peak height per profile

(c)

variance of allele heights
as a function of allele height means

(d)

coefficient of variation
as a function of mean

FIG.    13

d16

**(a)**

mean height of allele as a function
of the sum of peak height per profile

Locusd16

**(b)**

mean height of stutter as a function
of the sum of peak height per profile

**(c)**

variance of allele heights
as a function of allele height means

**(d)**

coefficient of variation
as a function of mean

FIG. 14

79

(a)

mean height of allele as a function
of the sum of peak height per profile

(b)

mean height of stutter as a function
of the sum of peak height per profile

(c)

variance of allele heights
as a function of allele height means

(d)

coefficient of variation
as a function of mean

FIG.    15

amelo

(a)

mean height of allele as a function
of the sum of peak height per profile

(b)

mean height of stutter as a function
of the sum of peak height per profile

(c)

coefficient of variation
as a function of mean

FIG. 16

81

d8

Locusd8

(a)

(b)

mean height of allele as a function
of the sum of peak height per profile

mean height of stutter as a function
of the sum of peak height per profile

(c)

(d)

variance of allele heights
as a function of allele height means

coefficient of variation
as a function of mean

FIG.    17

(a)

mean height of allele as a function
of the sum of peak height per profile

(b)

mean height of stutter as a function
of the sum of peak height per profile

(c)

variance of allele heights
as a function of allele height means

(d)

coefficient of variation
as a function of mean

FIG. 18

d18

(a)

mean height of allele as a function
of the sum of peak height per profile

Locusd18

(b)

mean height of stutter as a function
of the sum of peak height per profile

(c)

variance of allele heights
as a function of allele height means

(d)

coefficient of variation
as a function of mean

FIG. 19

(a)

mean height of allele as a function
of the sum of peak height per profile

(b)

mean height of stutter as a function
of the sum of peak height per profile

(c)

variance of allele heights
as a function of allele height means

(d)

coefficient of variation
as a function of mean

FIG. 20

(a)

mean height of allele as a function
of the sum of peak height per profile

(b)

mean height of stutter as a function
of the sum of peak height per profile

(c)

variance of allele heights
as a function of allele height means

(d)

coefficient of variation
as a function of mean

FIG.    21

fg

(a)

mean height of allele as a function
of the sum of peak height per profile

Locusfg

(b)

mean height of stutter as a function
of the sum of peak height per profile

(c)

variance of allele heights
as a function of allele height means

(d)

coefficient of variation
as a function of mean

FIG. 22

| Locus | Allele | Base pair | Height | $X_i$ | $\overline{b}_i$ |
|---|---|---|---|---|---|
| D3 | 16 | 130 | 370 | 742 | 133.8 |
| | 17 | 134 | 31 | | |
| | 18 | 138 | 341 | | |
| $\nu$Wa | 16 | 177 | 151 | 2716 | 180.8 |
| | 17 | 185 | 2565 | | |
| D16 | 13 | 266 | 229 | 417 | 267.8 |
| | 14 | 270 | 188 | | |
| D2 | 17 | 297 | 159 | 302 | 300.8 |
| | 19 | 305 | 143 | | |
| D8 | 11 | 140 | 94 | 3270 | 149.2 |
| | 12 | 144 | 1699 | | |
| | 14 | 152 | 75 | | |
| | 15 | 156 | 1402 | | |
| D21 | 28 | 303 | 388 | 626 | 354.3 |
| | 31.2 | 217 | 238 | | |
| D18 | - | - | - | - | - |
| D19 | 11 | 114 | 68 | 2213 | 124.1 |
| | 12 | 118 | 1144 | | |
| | 14.2 | 128 | 86 | | |
| | 15.2 | 132 | 915 | | |
| THO | 9.3 | 188 | 613 | 613 | 188 |
| FGA | 20 | 227 | 61 | 102 | 227 |
| | 24 | 243 | 41 | | |

Degraded profile information

## FIG. 23

FIG.    24

| Locus | Genotype | Allele | Height | Mean (ad) | Degradation Factor | Adjusted Mean (ad) |
|-------|----------|--------|--------|-----------|--------------------|--------------------|
| D3 | (16,17) | 15 | 0 | 28.6 (21.8) | 1.02 | 29.3 (21.8) |
| | | 16 | 370 | 378.4 (107.1) | 1.02 | 387.7 (109.2) |
| | | 17 | 31 | 28.6 (21.8) | 0.98 | 27.9 (21.4) |
| | | 18 | 341 | 378.4 (107.1) | 0.98 | 369.2 (105.1) |
| D21 | (28,31.2) | 27 | 0 | 18.7 (17.4) | 1.08 | 20.1 (17.9) |
| | | 28 | 388 | 319.3 (94.4) | 1.08 | 344.7 (100.0) |
| | | 30.2 | 0 | 18.7 (17.4) | 0.92 | 17.2 (16.4) |
| | | 31.2 | 238 | 319.3 (94.4) | 0.92 | 293.8 (89.0) |

FIG.    25

FIG. 26

EP 2 545 480 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009066067 A
- GB 2008003882 W

**Non-patent literature cited in the description**

- **J.D. BALDING ; R. NICHOLS.** DNA profile match probability calculation: How to allow for population stratification, relatedness, database selection and single bands. *Forensic Science International,* 1994, vol. 64, 125-140